(19) Europäisches Patentamt
European Patent Office
Office européen des brevets

(11) EP 2 776 586 B1

(12) # EUROPEAN PATENT SPECIFICATION

(45) Date of publication and mention of the grant of the patent:
**07.03.2018 Bulletin 2018/10**

(21) Application number: **12846898.0**

(22) Date of filing: **09.11.2012**

(51) Int Cl.:
***C12Q 1/68*** *(2018.01)*

(86) International application number:
**PCT/US2012/064520**

(87) International publication number:
**WO 2013/071163 (16.05.2013 Gazette 2013/20)**

(54) **BIOMARKERS OF RESPONSE TO PROTEASOME INHIBITORS**

BIOMARKER DER REAKTION AUF PROTEASOMENHEMMER

BIOMARQUEURS DE LA SENSIBILITÉ VIS-À-VIS D'INHIBITEURS DU PROTÉASOME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.11.2011 US 201161558470 P**

(43) Date of publication of application:
**17.09.2014 Bulletin 2014/38**

(73) Proprietor: **Millennium Pharmaceuticals, Inc.**
**Cambridge, MA 02139 (US)**

(72) Inventors:
- **BERGER, Allison**
  **Arlington, MA 02474 (US)**
- **BERNARD, Hughes**
  **Woburn, MA 01801 (US)**
- **CHATTOPADHYAY, Nibedita**
  **Wellesley, MA 02482 (US)**
- **KOENIG, Erik, M.**
  **North Reading, MA 01864-1517 (US)**
- **MULLIGAN, George, J.**
  **Lexington, MA 02421 (US)**
- **SCHU, Matthew, C.**
  **Brookline, MA 02446 (US)**

(74) Representative: **Lau, Sarah Jane et al**
**Kilburn & Strode LLP**
**Lacon London**
**84 Theobalds Road**
**London WC1X 8NL (GB)**

(56) References cited:

| | |
|---|---|
| **WO-A2-2004/053066** | **US-A1- 2006 281 122** |
| **US-A1- 2008 227 096** | **US-A1- 2009 023 149** |
| **US-A1- 2009 181 393** | **US-A1- 2010 215 678** |
| **US-A1- 2010 310 503** | **US-B1- 6 361 941** |
| **US-B1- 6 361 941** | |

- **MULLIGAN GEORGE ET AL: "Gene expression profiling and correlation with outcome in clinical trials of the proteasome inhibitor bortezomib", BLOOD, AMERICAN SOCIETY OF HEMATOLOGY, US, vol. 109, no. 8, 1 April 2007 (2007-04-01) , pages 3177-3188, XP002548156, ISSN: 0006-4971, DOI: 10.1182/BLOOD-2006-09-044974 [retrieved on 2006-12-21]**
- **BISPING ET AL.: 'Bortezomib, Dexamethasone, and Fibroblast Growth Factor Receptor 3-SpecificTyrosine Kinase Inhibitor in t(4;14)Myeloma' CLIN CANCER RES vol. 15, 15 January 2009, pages 520 - 531, XP002624033**
- **FONSECA ET AL.: 'International Myeloma Working Group molecular classification of multiple myeloma: spotlight review' LEUKEMIA DECEMBER 2009. vol. 23, December 2009, pages 2210 - 2221, XP055152874**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Background

Cells become cancerous when their genotype or phenotype alters in a way that there is uncontrolled growth that is not subject to the confines of the normal tissue environment. One or more genes is mutated, amplified, deleted, overexpressed or underexpressed. Chromosome portions can be lost or moved from one location to another. Some cancers have characteristic patterns by which genotypes or phenotypes are altered.

Many genes have mutations which are associated with cancer. Some genes have multiple sites where mutations can occur. Many cancers have mutations in and/or mis-expression of more than one gene. Gene mutations can facilitate tumor progression, tumor growth rate or whether a tumor will metastasize. Some mutations can affect whether a tumor cell will respond to therapy.

A variety of agents treat cancers. Cancers of the blood and bone marrow often are treated with steroids/glucocorticoids, imids, proteasome inhibitors and alkylating agents. Cancers of other tissues often are treated with alkylating agents, topoisomerase inhibitors, kinase inhibitors, microtubule inhibitors, angiogenesis inhibitors or other agents. Some patients respond to one therapy better than another, presenting the potential for a patient to follow multiple therapeutic routes to effective therapy. Valuable time early in a patient's treatment program can be lost pursuing a therapy which eventually is proven ineffective for that patient. Many patients cannot afford the time for trial-and-error choices of therapeutic regimens. Expedient and accurate treatment decisions lead to effective management of the disease.

WO2004/053066 relates to the identification of markers that can be used to determine whether patients with cancer are clinically responsive or non-responsive to a therapeutic regimen prior to treatment. In particular, this document is directed to the use of certain combinations of markers, wherein the expression of the markers correlates with responsiveness or non-responsiveness to or time-to-progression after a therapeutic regimen comprising proteasome inhibition. Thus, by examining the expression levels of individual markers and those comprising a marker set, it is possible to determine whether a therapeutic agent, or combination of agents, will be most likely to reduce the growth rate of tumors in a clinical setting.

### Summary

The present disclosure relates to prognosis and planning for treatment of hematological tumors by measurement of at least one characteristic of a marker provided herein. Markers were identified in pre-treatment tumor samples by associating their characteristics, e.g., size, sequence, composition or amount, with outcome of subsequent treatment in patients undergoing proteasome inhibition therapy. The markers are predictive of whether there will be a favorable outcome (*e.g.*, good response and/or long time-to-progression) after treatment with a proteasome inhibitor, such as a peptidyl boronic acid or peptidyl epoxy ketone. Testing samples comprising tumor cells to determine the presence, amounts or changes of genetic markers identifies particular patients who are expected to have a favorable outcome with treatment, *e.g.*, with a proteasome inhibitor, e.g., a peptidyl boronic acid, and whose disease may be managed by standard or less aggressive treatment, as well as those patients who are expected have an unfavorable outcome with the treatment and may require an alternative treatment to, a combination of treatments and/or more aggressive treatment with a proteasome inhibitor to ensure a favorable outcome and/or successful management of the disease.

In one aspect, the invention provides a method for identifying a cancer patient as a candidate for treatment with bortezomib, wherein the patient has a hematological tumor, comprising: a) identifying the sequence of at least one marker associated with at least one marker gene in a patient sample comprising hematological tumor cells, wherein one marker gene is neuroblastoma RAS viral (v-ras) oncogene homolog (NRAS); and b) identifying the patient as a candidate for treatment with bortezomib if the sequence indicates that the tumor cells comprise wild type NRAS. In another aspect, the invention provides the use of a kit comprising a stabilizer to add to a sample comprising tumor cells and a reagent to identify the sequence of at least one marker in a sample, wherein the result indicates whether there is a mutation in at least one marker gene, wherein the at least one marker gene is NRAS, in a method of the first aspect of the invention. In another aspect, the invention provides a proteasome inhibitor for use in treating a patient having a hematological tumor comprising wild type NRAS, comprising the step of administering to the patient a therapeutically effective amount of a proteasome inhibitor, if a sample of hematological tumor cells from the patient has been determined to have wild type NRAS, wherein the proteasome inhibitor is bortezomib. In another aspect, the invention provides a method of identifying a hematological cancer patient who will be nonresponsive to treatment with bortezomib, comprising determining the presence or absence of at least one NRAS mutation by sequencing a marker or a portion of a marker suspected of comprising the mutation in a sample comprising tumor cells from the patient, wherein the at least one NRAS mutation is an activating mutation, whereby the presence of at least one NRAS mutation indicates that the hematological cancer patient will not respond to bortezomib. In the invention, the tumor is a hematological tumor, e.g., myeloma, *e.g.*, multiple myeloma, lymphoma, *e.g.*, mantle cell lymphoma or follicular lymphoma, or leukemia, e.g., acute myeloid leukemia or

acute lymphoid leukemia.

In the invention, the sequence of marker DNA, the sequence of marker RNA and/or the sequence of marker protein corresponding to a marker gene with one or more mutation, e.g., somatic mutation, described herein is measured. Useful information leading to the prognosis or treatment or disease management strategies is obtained when assays reveal information about a marker gene, e.g., whether the gene is mutated, or not, the identity of the mutation, and/or whether the RNA or protein amount of a mutated gene or genes indicates overexpression or underexpression. In the invention, the strategy is determined for therapy with the proteasome inhibitor bortezomib (VELCADE®).

The marker gene used in the present invention to test for determination of hematological tumor prognosis or treatment or disease management strategy is neuroblastoma RAS viral (v-ras) oncogene homolog (NRAS). The marker gene includes mutations or alterations whose presence in marker DNA or whose effects, *e.g.*, on marker RNA and/or protein characteristics, e.g., amounts, size, sequence, activity or composition, can provide information for determination of prognosis or treatment or disease management. In some embodiments, a gene or a mutant or modified form thereof useful as a marker gene, is associated with one or more markers, e.g., a DNA, an RNA and/or protein characteristic, e.g., size, sequence, composition, activity or amount, *e.g.*, in a sample comprising tumor cells, which is different than a normal DNA, RNA and/or protein. Described herein are examples of modifications of this gene, referred to as a "marker gene" whose mutation can provide such information.

The mutation of a marker gene of the present invention provides information about outcome after treatment, *e.g.*, with a proteasome inhibitor, e.g., a peptidyl boronic acid or peptidyl epoxy ketone. By examining a characteristic, e.g., size, sequence, composition, activity or amount of one or more of identified markers in a tumor, it is possible to determine which therapeutic agent, combination of agents, dosing and/or administration regimen is expected to provide a favorable outcome upon treatment. By examining the characteristic, e.g., size, sequence, composition, activity or amount of one or more of the identified markers or marker sets in a cancer, it is also possible to determine which therapeutic agent, combination of agents, dosing and/or administration regimen is less likely to provide a favorable outcome upon treatment. By examining the characteristic, e.g., size, sequence, composition, activity or amount of one or more of the identified markers, it is therefore possible to eliminate ineffective or inappropriate therapeutic agents or regimens. Importantly, these determinations can be made on a patient-by-patient basis. Thus, one can determine whether or not a particular therapeutic regimen is likely to benefit a particular patient or type of patient, and/or whether a particular regimen should be started or avoided, continued, discontinued or altered.

The present invention is directed to methods of identifying and/or selecting a cancer patient who is expected to demonstrate a favorable outcome upon administration of bortezomib. Additionally provided are methods of identifying a patient who is expected to have an unfavorable outcome upon administration of bortezomib. These methods include identifying the sequence of one or more markers or mutation of marker gene in a patient's tumor (*e.g.*, in a patient's hematological cancer cells), optionally comparing that to the sequence of a reference marker, and in a further embodiment, identifying or advising whether the result from the sample corresponds to a favorable outcome of a treatment regimen, i.e. a bortezomib treatment regimen.

Also described herein are therapeutic methods which further include the step of beginning, continuing, or commencing a therapy accordingly where the presence of a mutation in a marker gene or the characteristic, e.g., size, sequence, composition, activity or amount of a patient's marker or markers indicates that the patient is expected to demonstrate a favorable outcome with the therapy, *e.g.*, the proteasome inhibitor, such as a peptidyl boronic acid or peptidyl epoxy ketone therapeutic regimen. Also described herein are therapeutic methods which further include the step of stopping, discontinuing, altering or halting a therapy accordingly where the presence of a mutation in a marker gene or the characteristic, e.g., size, sequence, composition, activity or amount of a patient's marker indicates that the patient is expected to demonstrate an unfavorable outcome with the treatment, *e.g.*, with the proteasome inhibitor, such as a peptidyl boronic acid or peptidyl epoxy ketone, regimen, *e.g.*, as compared to a patient identified as having a favorable outcome receiving the same therapeutic regimen. In another aspect, methods are provided for analysis of a patient not yet being treated with a therapy, *e.g.*, an proteasome inhibitor, e.g., a peptidyl boronic acid or peptidyl epoxy ketone and identification and prediction of treatment outcome based upon the presence of a mutation in a marker gene or characteristic, e.g., size, sequence, composition, activity or amount of one or more of a patient's marker described herein. Such methods can include not being treated with the therapy, *e.g.*, proteasome inhibitor, e.g., a peptidyl boronic acid or peptidyl epoxy ketone therapy, being treated with therapy, *e.g.*, proteasome inhibitor, or being treated with a peptidyl boronic acid or peptidyl epoxy ketone therapy in combination with one more additional therapies, being treated with an alternative therapy to a proteasome inhibitor, such as a peptidyl boronic acid or peptidyl epoxy ketone therapy, or being treated with a more aggressive dosing and/or administration regimen of a therapy, *e.g.*, proteasome inhibitor, e.g., a peptidyl boronic acid or peptidyl epoxy ketone inhibitor, *e.g.*, as compared to the dosing and/or administration regimen of a patient identified as having a favorable outcome to standard proteasome inhibitor, e.g., a peptidyl boronic acid or peptidyl epoxy ketone therapy. Thus, the provided methods of the invention can eliminate ineffective or inappropriate use of therapy, *e.g.*, proteasome inhibitor, e.g., peptidyl boronic acid or peptidyl epoxy ketone therapy regimens.

Also described herein are methods to determine the activity of an agent, the efficacy of an agent, or identify

new therapeutic agents or combinations. Such methods include methods to identify an agent as useful, *e.g.*, as a proteasome inhibitor, e.g., a peptidyl boronic acid, for treating a cancer, *e.g.*, a hematological cancer (*e.g.,* multiple myeloma, lymphoma, *e.g.*, mantle cell lymphoma or follicular lymphoma, or leukemia, e.g., acute myeloid leukemia or acute lymphoid leukemia), based on its ability to affect the presence of a mutation in a marker gene or characteristic, e.g., size, sequence, composition, activity or amount of a marker or markers of the invention. In some embodiments, an inhibitor which decreases or increases the presence of a mutation in a marker gene or characteristic, e.g., size, sequence, composition, activity or amount of a marker or markers provided (i.e., in a cell population, the inhibitor selects against cells comprising the mutation characteristic or selects for cells comprising the mutation or characteristic, respectively) in a manner that indicates favorable outcome of a patient having cancer would be a candidate agent for the cancer. In another embodiment, an agent which is able to decrease the viability of a tumor cell comprising a marker indicative of an unfavorable outcome would be a candidate agent for the cancer.

Also described herein are methods of treating a cancer patient, with a therapeutic regimen, *e.g.*, a proteasome inhibitor, e.g., a peptidyl boronic acid or peptidyl epoxy ketone therapy regimen (*e.g.*, alone, or in combination with an additional agent such as a chemotherapeutic agent, *e.g.*, a glucocorticoid agent, a microtubule inhibitor, an alkylating agent, a kinase inhibitor or a topoisomerase inhibitor), which includes the step of selecting for treatment a patient whose marker characteristic, e.g., size, sequence, composition, activity or amount indicates that the patient is expected to have a favorable outcome with the therapeutic regimen, and treating the patient with the therapy, *e.g.*, proteasome inhibitor, e.g., a peptidyl boronic acid therapy. In some embodiments, the method can include the step of selecting a patient whose marker characteristic, e.g., size, sequence, composition, activity or amount or amounts indicates that the patient is expected to have a favorable outcome and administering a therapy other than a proteasome inhibitor therapy that demonstrates similar expected progression-free survival times as the proteasome inhibitor, e.g., a peptidyl boronic acid therapy.

Additional methods of treating a cancer patient described herein include selecting patients that are unlikely to experience a favorable outcome upon treatment with a cancer therapy (*e.g.*, proteasome inhibitor, e.g., a peptidyl boronic acid or peptidyl epoxy ketone therapy). Such methods can further include one or more of: administering a higher dose or increased dosing schedule of a therapy, *e.g.*, proteasome inhibitor, e.g., a peptidyl boronic acid or peptidyl epoxy ketone as compared to the dose or dosing schedule of a patient identified as having a favorable outcome with standard therapy; administering a cancer therapy other than a proteasome inhibitor, e.g., a peptidyl boronic acid or peptidyl epoxy ketone therapy; administering a proteasome inhibitor, e.g., a peptidyl boronic acid or peptidyl epoxy ketone agent in combination with an additional agent. Further described are methods for selection of a patient having aggressive disease which is expected to demonstrate more rapid time to progression.

Additional methods described herein include a method to evaluate whether to treat or pay for the treatment of cancer, *e.g.*, hematological cancer (*e.g.,* myeloma, *e.g.*, multiple myeloma, lymphoma, *e.g.*, mantle cell lymphoma or follicular lymphoma, or leukemia, e.g., acute myeloid leukemia or acute lymphoid leukemia) by reviewing the amount of a patient’s marker or markers for indication of outcome to a cancer therapy, *e.g.*, a proteasome inhibitor, e.g., a peptidyl boronic acid therapy regimen, and making a decision or advising on whether payment should be made.

Other features and advantages of the invention will be apparent from the following detailed description, drawings and from the claims.

## Drawings

Figure 1. Kaplan-Meier plot of the dependence of time-to-progression of disease in multiple myeloma patients after treatment with bortezomib on mutation status of RAS. The p-value of association of an NRAS mutation with time-to-progression is $3.5 \times 10^{-4}$.

## Detailed Description

One of the continued problems with therapy in cancer patients is individual differences in response to therapies. While advances in development of successful cancer therapies progress, only a subset of patients respond to any particular therapy. With the narrow therapeutic index and the toxic potential of many available cancer therapies, such differential responses potentially contribute to patients undergoing unnecessary, ineffective and even potentially harmful therapy regimens. If a designed therapy could be optimized to treat individual patients, such situations could be reduced or even eliminated. Furthermore, targeted designed therapy may provide more focused, successful patient therapy overall. Accordingly, there is a need to identify particular cancer patients who are expected to have a favorable outcome when administered particular cancer therapies as well as particular cancer patients who may have a favorable outcome using more aggressive and/or alternative cancer therapies, *e.g.*, alternative to previous cancer therapies administered to the patient. It would therefore be beneficial to provide for the diagnosis, staging, prognosis, and monitoring of cancer patients, including, *e.g.*, hematological cancer patients (*e.g.*, myeloma, *e.g.*, multiple myeloma, lymphoma, *e.g.*, mantle

cell lymphoma or follicular lymphoma, or leukemia, e.g., acute myeloid leukemia or acute lymphoid leukemia) who would benefit from particular cancer inhibition therapies as well as those who would benefit from a more aggressive and/or alternative cancer inhibition therapy, *e.g.*, alternative to a cancer therapy or therapies the patient has received, thus resulting in appropriate preventative measures.

The present invention is based, in part, on the recognition that mutation of a marker gene can be associated with sensitivity of a cell comprising the mutated gene to a proteasome inhibitor, e.g., a peptidyl boronic acid. In the present invention, the marker gene is involved in the Rat Sarcoma (RAS) signaling pathway, i.e., a gene whose mutation enables activation of the pathway. RAS is an oncogenic GTPase whose active GTP-bound state activates pathways (e.g., the mitogen-activated protein (MAP) kinase cascade) involved in tumorigenesis. Proteins, including tumor suppressors, facilitate hydrolysis of RAS-bound GTP to GDP to inactivate RAS and thus limit the signaling from a RAS oncogene. A mutation in a gene involved in this checkpoint, either in an oncogene upstream of RAS (e.g., p210BCR-ABL or erbB), in a RAS oncogene (e.g., HRAS, KRAS or NRAS), in a RAS-associated tumor suppressor (neurofibromatosis 1(NF1)), and/or in a GTPase-activating protein (e.g., RASGAP), can enable activation of RAS signaling pathways. A protein encoded by a marker gene for sensitivity to a proteasome inhibitor can be a RAS protein. NRAS is an example of a marker gene. In the GTP-bound state of RAS proteins, e.g., NRAS, HRAS and KRAS, the RAS signaling occurs, and in the GDP-bound state, the signaling is abrogated. A mutated RAS protein can prolong its time in the GTP-bound state and the resulting signaling pathway activation can lead to proliferation of cells harboring the mutated gene. A marker gene can exhibit one or more mutations, e.g., somatic mutations, whose presence can affect expression or activity of the encoded gene product. In some embodiments, there can be more than one mutation in a marker gene in a tumor cell or tumor. In additional embodiments, there can be marker gene mutations in cells which have mutations in one or more additional genes, including mutations that can lead to tumorigenesis, but the additional mutated gene(s) may not be a marker gene as considered herein. In some embodiments, the mutation is an activating mutation. In other embodiments, the mutation affects the expression of the marker gene. In other embodiments, a mutation can result in an altered interaction of the encoded gene product with a cellular binding partner.

In one aspect, the invention provides a method for identifying a cancer patient as a candidate for treatment with bortezomib, wherein the patient has a hematological tumor, comprising: a) identifying the sequence of at least one marker associated with at least one marker gene in a patient sample comprising hematological tumor cells, wherein one marker gene is neuroblastoma RAS viral (v-ras) oncogene homolog (NRAS); and b) identifying the patient as a candidate for treatment with bortezomib if the sequence indicates that the tumor cells comprise wild type NRAS. In some embodiments, the method comprises identifying whether the hematological tumor is a t(4;14) translocation subtype. The method to determine whether to treat with bortezomib is performed *in vitro.*

In one aspect, the method of the invention further comprises determining whether to continue bortezomib treatment of the hematological tumor in the patient by a method comprising: a) identifying on a second biological sample comprising tumor cells from the patient identified for treatment with bortezomib the sequence of the at least one marker associated with the at least one marker gene in the sample, wherein at least one marker gene is NRAS, wherein the patient is treated with bortezomib prior to the second sample being obtained; b) comparing the results in a) to the results in the first sample; and c) determining to continue treatment with bortezomib if the comparison indicates that the tumor cells in the second sample comprise wild type NRAS. The method to determine whether to continue to treat with bortezomib is performed *in vitro.* In some embodiments, the method additionally comprises determining the hematological tumor subtype. In some embodiments, the hematological tumor subtype is a t(4;14).

In one aspect, the invention provides use of a kit comprising a stabilizer to add to a sample comprising tumor cells and a reagent to identify the sequence of at least one marker in a sample, wherein the result indicates whether there is a mutation in at least one marker gene, wherein the at least one marker gene is NRAS, in a method of the invention. In some embodiments, the kit further provides a reagent to identify the tumor subtype. In some embodiments, the tumor subtype reagent identifies whether there is a t(4;14) translocation.

Also described herein is a kit comprising at least two reagents to measure at least one characteristic of at least two markers in a sample, wherein the result of the measurement indicates whether there is a mutation in at least one marker gene, wherein at least one marker gene is NRAS and wherein the sample comprises hematological tumor cells. In some embodiments at least two reagents comprises at least one reagent to identify the mutational status of the NRAS marker gene and at least one reagent to identify whether the tumor is a t(4;14) subtype.

Also described herein is a method for predicting sensitivity of a hematological tumor cell to a proteasome inhibitor, comprising: a) assessing whether the hematological tumor cell expresses mutated NRAS; and b) predicting sensitivity of the cell to a proteasome inhibitor, wherein expression of mutated NRAS is predictive of poor sensitivity to the proteasome inhibitor.

In one aspect, the invention provides a proteasome inhibitor for use in treating a patient having a hematological tumor comprising wild type NRAS, comprising the step of administering to the patient a therapeutically effective amount of a proteasome inhibitor, if a sample of hematological tumor cells from the patient has been determined to have wild type NRAS, wherein the proteasome inhibitor is bortezomib. In some embodiments, the patient has a hematological

tumor with a t(4;14) subtype.

Also described herein is the use of a proteasome inhibitor in the manufacture of a medicament to treat a hematological tumor, wherein the hematological tumor has a wild type NRAS. In some embodiments, the hematological tumor has a t(4;14) subtype.

Also described herein is the use of a proteasome inhibitor for treating a hematological tumor in a patient whose hematological tumor has wild type NRAS. In one embodiment, the hematological tumor has a t(4;14) subtype.

Also described herein is a method for identifying a proteasome inhibitor as suitable for use in treating a patient with a hematological cancer, comprising: a) contacting a hematological tumor cell comprising at least one mutation in at least one marker gene with a test proteasome inhibitor, wherein at least one marker gene is NRAS; b) assessing the effect of the test proteasome inhibitor on the viability of the cell; and c) determining that the test proteasome inhibitor is suitable for use in treating a patient with a hematological cancer if it decreases the viability of the hematological tumor cell. In some embodiments, the hematological tumor cell does not have the t(4;14) subtype.

Also described herein is a method for paying for the treatment of cancer with a proteasome inhibitor comprising: a) recording the whether NRAS is mutated in a sample comprising hematological tumor cells from a patient, and b) authorizing payment of the proteasome inhibitor treatment if NRAS is wild type. In some embodiments, the hematological tumor cells comprise a t(4;14) subtype.

In one aspect, the invention provides a method of identifying a hematological cancer patient who will be non-responsive to treatment with bortezomib, comprising determining the presence or absence of at least one NRAS mutation by sequencing a marker or a portion of a marker suspected of comprising the mutation in a sample comprising tumor cells from the patient, wherein at least one NRAS mutation is an activating mutation, whereby the presence of at least one NRAS mutation indicates that the hematological cancer patient will not respond to bortezomib. In some embodiments, the tumor cells are not of the t(4;14) translocation subtype.

Also described herein is a method of identifying a hematological cancer patient who will have a favorable to treatment with a proteasome inhibitor, comprising determining the presence or absence of at least one NRAS mutation in a sample comprising tumor cells from the patient, whereby the presence of wild type NRAS mutation indicates that the hematological cancer patient will respond to the proteasome inhibitor.

The identification and/or measurement of the mutation in the marker gene can be used to determine whether a favorable outcome can be expected by treatment of a tumor, *e.g.,* with a proteasome inhibitor, e.g., a peptidyl boronic acid therapy or peptidyl epoxy ketone or whether an alternative therapy to and/or a more aggressive therapy with, *e.g.*, a proteasome inhibitor, e.g., a peptidyl boronic acid or peptidyl epoxy ketone inhibitor may enhance the response. For example, the compositions and methods provided herein can be used to determine whether a patient is expected to have a favorable outcome to a proteasome inhibitor, e.g., a peptidyl boronic acid or peptidyl epoxy ketone therapeutic agent dosing or administration regimen. Based on these identifications, the present invention provides, and described herein are, without limitation: 1) methods and compositions for determining whether a proteasome inhibitor, e.g., a peptidyl boronic acid or peptidyl epoxy ketone therapy regimen will or will not be effective to achieve a favorable outcome and/or manage the cancer; 2) methods and compositions for monitoring the effectiveness of a proteasome inhibitor, e.g., a peptidyl boronic acid or peptidyl epoxy ketone therapy (alone or in a combination of agents) and dosing and administrations used for the treatment of tumors; 3) methods and compositions for treatments of tumors comprising, *e.g.*, proteasome inhibitor, e.g., a peptidyl boronic acid or peptidyl epoxy ketone inhibition therapy regimen; 4) methods and compositions for identifying specific therapeutic agents and combinations of therapeutic agents as well as dosing and administration regimens that are effective for the treatment of tumors in specific patients; and 5) methods and compositions for identifying disease management strategies.

Proteasome inhibition represents an important strategy in cancer treatment. The proteasome is a multi-enzyme complex present in all cells which play a role in degradation of proteins involved in regulation of the cell cycle. For example, King et al. (Science 274:1652-1659 (1996)) demonstrated that the ubiquitin-proteasome pathway plays an essential role in regulating cell cycle, neoplastic growth and metastasis. A number of key regulatory proteins, including p53, cyclins, and the cyclin-dependent kinases p21 and $p27^{KIP1}$, are temporally degraded during the cell cycle by the ubiquitin-proteasome pathway. The ordered degradation of these proteins is required for the cell to progress through the cell cycle and to undergo mitosis. Furthermore, the ubiquitin-proteasome pathway is required for transcriptional regulation. Palombella *et al.* (International Patent Application Publication No. WO 95/25533) teach that the activation of the transcription factor NF-κB is regulated by proteasome-mediated degradation of the inhibitor protein IκB. In turn, NF-κB plays a central role in the regulation of genes involved in the immune and inflammatory responses. For example, Read et al. (Immunity 2:493-506 (1995)) demonstrated that the ubiquitin-proteasome pathway is required for expression of cell adhesion molecules, such as E-selectin, ICAM-1, and VCAM-1. Additional findings further support the role for proteasome inhibition in cancer therapy, as Zetter (Seminars in Cancer Biology 4:219-229 (1993)) found that cell adhesion molecules are involved in tumor metastasis and angiogenesis *in vivo,* by directing the adhesion and extravasation of tumor cells to and from the vasculature to distant tissue sites within the body. Moreover, Beg and Baltimore (Science 274:782 (1996)) found that NF-κB is an anti-apoptotic factor, and inhibition of NF-κB activation makes cells more sensitive

to environmental stress and cytotoxic agents. Bortezomib (Velcade®) is a first-in-class peptidyl boronic acid proteasome inhibitor.

As used herein, the term "proteasome" refers to a subcellular complex which participates in protein homeostasis by degrading proteins no longer needed by a cell or defective proteins and which are targeted for degradation by being tagged with ubiquitin or a ubiquitin-like protein. The proteasome comprises a core complex with proteases which mediate the protein degradation.

As used herein, the term "proteasome inhibitor" refers to any substance which directly inhibits enzymatic activity of the 20S or 26S proteasome *in vitro* or *in vivo.* Proteasome inhibitors, their pharmacological properties and use in treating disease, including oncological diseases and inflammatory diseases are reviewed in Ruggeri et al. (2009) Adv. Pharmacol. 57:91-135. In the present invention, the proteasome inhibitor is a peptidyl boronic acid. Examples of peptidyl boronic acid proteasome inhibitors suitable for use in the methods of the invention are disclosed in Adams et al., U.S. Patent Nos. 5,780,454 (1998), 6,066,730 (2000), 6,083,903 (2000); 6,297,217 (2001), 6,465,433 (2002), 6,548,668 (2003), 6,617,317 (2003), and 6,747,150 (2004). In some embodiments, the peptidyl boronic acid proteasome inhibitor is selected from the group consisting of: N (4 morpholine)carbonyl-β-(1-naphthyl)-L-alanine-L-leucine boronic acid; N (8 quinoline)sulfonyl-β-(1-naphthyl)-L-alanine-L-alanine-L-leucine boronic acid; N (pyrazine)carbonyl-L-phenylalanine-L-leucine boronic acid, and N (4 morpholine)¬carbonyl-[O-(2-pyridylmethyl)]-L-tyrosine-L-leucine boronic acid. In the present invention, the proteasome inhibitor is N (pyrazine)carbonyl-L-phenylalanine-L-leucine boronic acid (bortezomib; VELCADE®; formerly known as MLN341 or PS-341). In another embodiment, the proteasome inhibitor is disclosed in U.S. Patent No. 7,442,830, for example, [(1R)-1({[(2,4-dichlorobenzoyl)amino]acetyl{-amino)-3-methylbutyl]boronic acid (MLN2238) or a boronate ester thereof, e.g., a citrate ester thereof, e.g., as disclosed in PCT Publication No. WO2009154737 (ixazomib citrate, MLN9708). Ixazomib citrate has anti-tumor activity in a range of hematological and solid tumor xenograft models (Kupperman et al. (2010) Cancer Res. 70:1970-1980). In another embodiment, the peptide boronic acid is disclosed in U.S. Patent No. 7,915,236, for example [(1R)-1-[[(2S,3R)-3-hydroxy-2-[(6-phenyl-pyridine-2-carbonyl)amino]-1-oxo-butyl]amino]-3-methylbutyl] boronic acid (delanzomib).

Further examples of peptidyl boronic acid proteasome inhibitors are disclosed in Fleming and Li, International Patent Publications WO 2010/036357 and WO 2011/123502.

In some embodiments, proteasome inhibitor is characterized by a compound of formula (***I***):

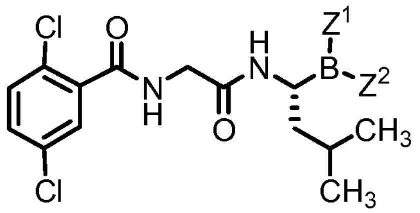

(***I***);

or a pharmaceutically acceptable salt or a pharmaceutical composition or a boronic acid anhydride thereof, wherein:

> $Z^1$ and $Z^2$ are each independently hydroxy, alkoxy, aryloxy, or aralkoxy; or $Z^1$ and $Z^2$ together form a moiety derived from a boronic acid complexing agent.

As used herein, the term "boronic acid" refers to a chemical compound containing a $-B(OH)_2$ moiety. In some embodiments, boronic acid compounds can form oligomeric anhydrides by dehydration of the boronic acid moiety. For example, Snyder et al., J. Am. Chem. Soc. 80:3611 (1958), reports oligomeric arylboronic acids.

As used herein, the term "boronic acid anhydride" refers to a chemical compound formed by combination of two or more molecules of a boronic acid compound, with loss of one or more water molecules. When mixed with water, the boronic acid anhydride compound is hydrated to release the free boronic acid compound. In various embodiments, the boronic acid anhydride can comprise two, three, four, or more boronic acid units, and can have a cyclic or linear configuration. Non-limiting examples of oligomeric boronic acid anhydrides of peptide boronic acids compound of the invention are illustrated below:

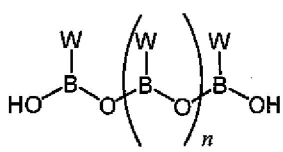

(1)

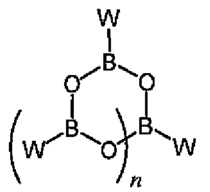

(2)

In formulae (1) and (2) directly above, the variable *n* is an integer from 0 to about 10, preferably 0, 1, 2, 3, or 4. In some embodiments, the boronic acid anhydride compound comprises a cyclic trimer ("boroxine") of formula (2), wherein *n* is 1. The variable W has the formula (3):

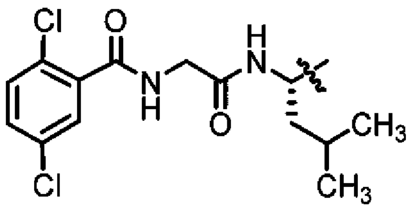

(3).

In some embodiments, at least 80% of the boronic acid present in the boronic acid anhydride compound exists in a single oligomeric anhydride form. In some embodiments, at least 85%, 90%, 95%, or 99% of the boronic acid present in the boronic acid anhydride compound exists in a single oligomeric anhydride form. In certain preferred embodiments, the boronic acid anhydride compound consists of, or consists essentially of, a boroxine having formula (3).

The boronic acid anhydride compound preferably can be prepared from the corresponding boronic acid by exposure to dehydrating conditions, including, but not limited to, recrystallization, lyophilization, exposure to heat, and/or exposure to a drying agent. Nonlimiting examples of suitable recrystallization solvents include ethyl acetate, dichloromethane, hexanes, ether, acetonitrile, ethanol, and mixtures thereof.

In some embodiments, $Z^1$ and $Z^2$ together form a moiety derived from a boronic acid complexing agent as disclosed in Olhava and Danca, U.S. Patent Nos. 7,442,830, 7,867,662, and 8,003,819. For purposes of the invention, the term "boronic acid complexing agent" refers to any compound having at least two functional groups, each of which can form a covalent bond with boron. Nonlimiting examples of suitable functional groups include amino, hydroxyl, and carboxyl. In some embodiments, at least one of the functional groups is a hydroxyl group. The term "moiety derived from a boronic acid complexing agent" refers to a moiety formed by removing the hydrogen atoms from two functional groups of a boronic acid complexing agent.

As used herein, the terms "boronate ester" and "boronic ester" are used interchangeably and refer to a chemical compound containing a $-B(Z^1)(Z^2)$ moiety, wherein at least one of $Z^1$ or $Z^2$ is alkoxy, aralkoxy, or aryloxy; or $Z^1$ and $Z^2$ together form a moiety derived from a boronic acid complexing agent having at least one hydroxyl group.

In some embodiments, $Z^1$ and $Z^2$ are each hydroxy and the compound of formula (***I***) is characterized by formula (***II***):

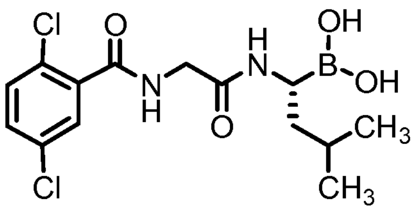

(***II***);

or a pharmaceutically acceptable salt or a pharmaceutical composition or a boronic acid anhydride thereof.

The compound of formula (***II***), [(1R)-1({[(2,4-dichlorobenzoyl)amino]acetyl{-amino)-3-methylbutyl]boronic acid (MLN2238) is disclosed in Olhava and Danca, U.S. Patent No. 7,442,830.

In some other embodiments, $Z^1$ and $Z^2$ together form a moiety derived from a compound having at least two hydroxyl groups separated by at least two connecting atoms in a chain or ring, said chain or ring comprising carbon atoms and, optionally, a heteroatom or heteroatoms which can be N, S, or O, wherein the atom attached to boron in each case is an oxygen atom.

As employed herein, the term "compound having at least two hydroxyl groups" refers to any compound having two or more hydroxyl groups. For purposes of the invention, the two hydroxyl groups preferably are separated by at least two connecting atoms, preferably from about 2 to about 5 connecting atoms, more preferably 2 or 3 connecting atoms. For convenience, the term "dihydroxy compound" may be used to refer to a compound having at least two hydroxyl groups, as defined above. Thus, as employed herein, the term "dihydroxy compound" is not intended to be limited to compounds having only two hydroxyl groups. The moiety derived from a compound having at least two hydroxyl groups may be attached to boron by the oxygen atoms of any two of its hydroxyl groups. Preferably, the boron atom, the oxygen atoms attached to boron, and the atoms connecting the two oxygen atoms together form a 5- or 6-membered ring.

For purposes of the present invention, the boronic acid complexing agent preferably is pharmaceutically acceptable, i.e., suitable for administration to humans. In some preferred embodiments, the boronic acid complexing agent is a sugar, as described, e.g., in Plamondon et al., WO 02/059131 and Gupta et al., WO 02/059130. The term "sugar" includes any polyhydroxy carbohydrate moiety, including monosaccharides, disaccharides, polysaccharides, sugar alcohols and amino sugars. In some embodiments, the sugar is a monosaccharide, disaccharide, sugar alcohol, or amino sugar. Non-limiting examples of suitable sugars include glucose, sucrose, fructose, trehalose, mannitol, sorbitol, glucosamine, and *N*-methylglucosamine. In certain embodiments, the sugar is mannitol or sorbitol. Thus, in the embodiments wherein the sugar is mannitol or sorbitol, $Z^1$ and $Z^2$ together form a moiety of formula $C_6H_{12}O_6$, wherein the oxygen atoms of the two deprotonated hydroxyl groups form covalent attachments with boron to form a boronate ester compound. In certain embodiments, $Z^1$ and $Z^2$ together form a moiety derived from D-mannitol as disclosed in U.S. Patent No. 7,442,830.

In some embodiments, the boronic acid complexing agent is an alpha-hydroxycarboxylic acid or a beta-hydroxycarboxylic acid, as described, e.g., in Elliott et al., WO 09/154737. In some embodiments, the boronic acid complexing agent is selected from the group consisting of glycolic acid, malic acid, hexahydromandelic acid, citric acid, 2-hydroxyisobutyric acid, 3-hydroxybutyric acid, mandelic acid, lactic acid, 2-hydroxy-3,3-dimethylbutyric acid, 2-hydroxy-3-methylbutyric acid, 2-hydroxyisocaproic acid, beta-hydroxyisovaleric acid, salicylic acid, tartaric acid, benzilic acid, glucoheptonic acid, maltonic acid, lactobionic acid, galactaric acid, embonic acid, 1-hydroxy-2-naphthoic acid, and 3-hydroxy-2-naphthoic acid. In certain embodiments, the boronic acid complexing agent is citric acid.

In certain embodiments, wherein the alpha-hydroxy carboxylic acid or beta-hydroxy carboxylic acid is citric acid, the compound of formula (***I***) is characterized by formula (***III-A***) or (***III-B***):

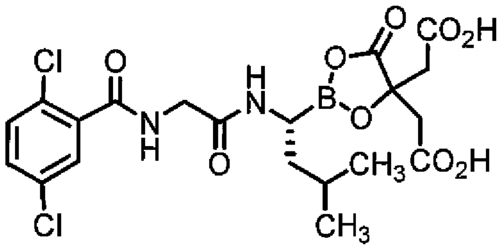

(***III-A***);

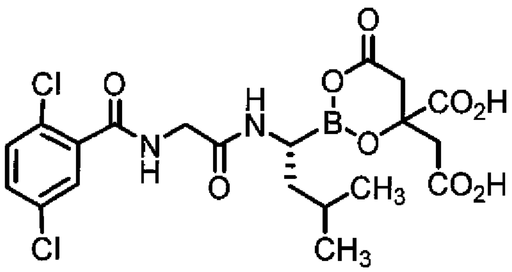

(***III-B***);

or a mixture thereof or a pharmaceutical composition thereof.

In certain embodiments, wherein the alpha-hydroxy carboxylic acid or beta-hydroxy carboxylic acid is citric acid, the compound of formula (***I***) is characterized by formula (***III-A***):

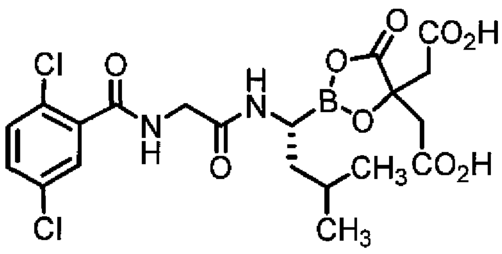

(*III-A*);

or a pharmaceutical composition thereof.

The compound of formula (***III-A***), 2,2'-{2-[(1*R*)-1-({[(2,5dichlorobenzoyl)amino]acetyl}amino)-3-methylbutyl]-5-oxo-1,3,2-dioxaborolane-4,4-diyl}diacetic acid (MLN9708, ixazomib citrate) is disclosed in Elliott et al., WO 09/154737.

Additionally, proteasome inhibitors include peptide aldehyde proteasome inhibitors (Stein et al., U.S. Patent No. 5,693,617 (1997); Siman *et al.,* international patent publication WO 91/13904; Iqbal et al., J. Med. Chem. 38:2276-2277 (1995); and Iinuma *et al.,* international patent publication WO 05/105826), peptidyl epoxy ketone proteasome inhibitors (Crews et al., U.S. Patent No. 6,831,099; Smyth et al., international patent publication WO 05/111008; Bennett et al., international patent publication WO 06/045066 or U.S. Patent Application publication No. US20050245435, e.g., (*S*)-4-Methyl-*N*-((*S*)-1-(((*S*)-4-methyl-1-((*R*)-2-methyloxiran-2-yl)-1-oxopentan-2-yl)amino)-1-oxo-3-phenylpropan-2-yl)-2-((*S*)-2-(2-morpholinoacetamido)-4-phenylbutanamido)pentanamide (carfilzomib); Spaltenstein et al. Tetrahedron Lett. 37:1343 (1996); Meng, Proc. Natl. Acad. Sci. 96: 10403 (1999); and Meng, Cancer Res. 59: 2798 (1999)), alpha-ketoamide proteasome inhibitors (Chatterjee and Mallamo, U.S. Patent Nos. 6,310,057 (2001) and 6,096,778 (2000); and Wang et al., U.S. Patent Nos. 6,075,150 (2000) and 6,781,000 (2004)), peptidyl vinyl ester proteasome inhibitors (Marastoni et al., J. Med. Chem. 48:5038 (2005), and peptidyl vinyl sulfone and 2-keto-1,3,4-oxadiazole proteasome inhibitors, such as those disclosed in Rydzewski et al., J. Med. Chem. 49:2953 (2006); and Bogyo et al., Proc. Natl. Acad. Sci. 94:6629 (1997)), azapeptoids and (Bouget et al., Bioorg. Med. Chem. 11:4881 (2003); Baudy-Floc'h *et al.,* international patent publication WO 05/030707; and Bonnemains *et al.,* international patent publication WO 03/018557), efrapeptin oligopeptides (Papathanassiu, international patent publication WO 05/115431), lactacystin and salinosporamide and analogs thereof (Fenteany et al., U.S. Patent Nos. 5,756,764 (1998), 6,147,223 (2000), 6,335,358 (2002), and 6,645,999 (2003); Fenteany et al., Proc. Natl. Acad. Sci. USA (1994) 91:3358; Fenical *et al.,* international patent publication WO 05/003137; Palladino *et al.,* international patent publication WO 05/002572; Stadler et al., international patent publication WO 04/071382; Xiao and Patel, U.S. patent publication 2005/023162; and Corey, international patent publication WO 05/099687).

Genes such as NRAS and KRAS are mutated in many cancer types. There has been interest in public cataloging of mutations associated with cancers. Examples of public databases which include information about mutations associated with cancers are the Database of Genotypes and Phenotypes (dbGaP) maintained by the National Center for Biotechnology Information (Bethesda, MD) and Catalogue of Somatic Mutations in Cancer (COSMIC) database maintained by the Wellcome Trust Sanger Institute (Cambridge, UK).

In an evaluation of 514 known mutations in 43 distinct oncogenes and tumor suppressor genes in tumor samples from multiple myeloma patients described herein, the most common mutations observed were in KRAS (n=32 [24.1%], 95% CI:17.0-31.3) and NRAS (n=26 [19.5%], 95% CI:12.8-26.3). Mutations in BRAF were detected in three patient samples (2.3%, 95% CI: 0-4.8). For all three genes, the mutation rate in this patient population was similar to that reported by Chapman et al. (2011) Nature 471:467-472 (26.3%, 23.7% and 4% for KRAS, NRAS, and BRAF, respectively). At least 50%, at least 70%, or 75-85% or about 80% of patients with myeloma tumors have wild type NRAS. Accordingly at least 50%, at least 70%, about 75-85% or about 80% of patients with myeloma tumors are candidates for treatment with a proteasome inhibitor. Patients with a tumor that has wild type NRAS, and wild type KRAS (at least 40%, at least 50%, or about 53-57% of patients) also can be candidates for treatment with a proteasome inhibitor. In some embodiments, a patient with a hematological tumor whose tumor cells comprise wild type NRAS can be a candidate for treatment with a proteasome inhibitor. In some embodiments, the hematological tumor is selected from the group consisting of myeloma, multiple myeloma, lymphoma, mantle cell lymphoma, follicular lymphoma, leukemia, acute myeloid leukemia and acute lymphoid leukemia. In some embodiments, the hematological tumor is selected from the group consisting of multiple myeloma, mantle cell lymphoma and follicular lymphoma. In some embodiments, the hematological tumor is multiple myeloma. In some embodiments, the hematological tumor is mantle cell lymphoma.

Compositions and methods are provided to determine the mutational status, e.g., to identify mutations in marker genes in hematological tumor, e.g., myeloma, *e.g*., multiple myeloma, lymphoma, *e.g*., mantle cell lymphoma or follicular lymphoma, or leukemia, e.g., acute myeloid leukemia or acute lymphoid leukemia, to predict outcome, e.g., response to treatment or time-to-progression, upon treatment with a proteasome inhibitor, e.g., a peptidyl boronic acid or a peptidyl epoxy ketone. In some embodiments, compositions and methods are provided to determine the mutational status of a

hematological tumor selected from the group consisting of myeloma, multiple myeloma, lymphoma, mantle cell lymphoma, follicular lymphoma, leukemia, acute myeloid leukemia and acute lymphoid leukemia to predict outcome of treatment with a peptidyl boronic acid.

Markers were identified based on genetic profiles of pretreatment tumor samples from patients and correlation with outcome of treatment with bortezomib. Statistical analysis of the mutation data aided in correlation of mutation status to outcome.

Unless otherwise defined, all technical and scientific terms used herein have the meanings which are commonly understood by one of ordinary skill in the art to which this invention belongs. Generally, nomenclature utilized in connection with, and techniques of cell and tissue culture, molecular biology and protein and oligo- or polynucleotide chemistry and hybridization described herein are those known in the art. GenBank or GenPept accession numbers and useful nucleic acid and peptide sequences can be found at the website maintained by the National Center for Biotechnology Information, Bethesda, MD. Standard techniques are used for recombinant DNA, oligonucleotide synthesis, protein purification, tissue culture and transformation and transfection (e.g., electroporation, lipofection, etc). Enzymatic reactions, such as GTPase assay for RAS activity or assays, e.g., reporter assays for RAS-activated signaling activity, are performed according to manufacturer's specifications or as commonly accomplished in the art or as described herein. Some methods for determining RAS localization and signaling are reviewed in Prior and Hancock (2011) Semin.Clin. Dev. Biol. Sep 8 epub; or found in Cuiffo and Ren (2010) Blood 114:3598-3605 or reviewed in Lim et al. (1996) Eur. J. Biochem. 242:171-185. The foregoing techniques and procedures generally are performed according to methods known in the art, e.g., as described in various general and more specific references that are cited and discussed throughout the present specification. See e.g., Sambrook et al. (2000) Molecular Cloning: A Laboratory Manual (3rd ed., Cold Spring Harbor Laboratory Press, Cold Spring Harbor, NY) or Harlow, E. and Lane, D. (1988) Antibodies: A Laboratory Manual (Cold Spring Harbor Laboratory Press, Cold Spring Harbor, NY). The nomenclatures utilized in connection with, and the laboratory procedures and techniques of, analytical chemistry, synthetic organic chemistry, and medicinal and pharmaceutical chemistry described herein are known in the art. Standard techniques are used for chemical syntheses, chemical analyses, pharmaceutical preparation, formulation and delivery, and treatment of patients. Furthermore, unless otherwise required by context, singular terms shall include pluralities and plural terms shall include the singular. In the case of conflict, the present specification, including definitions, will control.

The articles "a," "an" and "at least one" are used herein to refer to one or to more than one of the grammatical object of the article. By way of example, "an element" means one or more than one element, at least one element. In the case of conflict, the present specification, including definitions, will control.

As used herein, a "marker gene" refers to a gene which can have a mutation such that its DNA, RNA and/or protein has a characteristic, e.g., size, sequence, composition, activity or amount(s) which provide information about prognosis or outcome (*i.e.*, are "informative") upon treatment. Marker genes described herein as linked to outcome after proteasome inhibitor, e.g., peptidyl boronic acid (*e.g.*, bortezomib or ixazomib citrate) treatment are examples of genes within the chromosome locus markers described herein and are provided in Table 1. Sequences of mRNA, open reading frames and proteins corresponding to marker genes also are listed in Table 1. A marker gene listed in Table 1 can have isoforms which are either ubiquitous or have restricted expression. The DNA SEQ ID NOs in Table 1 refer only to the mRNA encoding the major or longest isoform and the protein SEQ ID NOs represent at least a precursor of such isoform and not necessarily the mature protein. These sequences are not intended to limit the marker gene identity to that isoform or precursor. The additional isoforms and mature proteins are readily retrievable and understandable to one of skill in the art by reviewing the information provided under the Entrez Gene (database maintained by the National Center for Biotechnology Information, Bethesda, MD) identified by the ID number listed in Table 1.

Table 1 Marker Gene Description for Proteasome Inhibitor Treatment

| Marker Gene ID | Marker Gene Name | Entrez Gene ID | Chromosome location | Start base pair | End base pair | SEQ ID NOs: |
|---|---|---|---|---|---|---|
| NRAS | neuroblastoma RAS viral (v-ras) oncogene homolog | 4893 | 1p | 115247085 | 115259515 | 1, 2, 3 |
| KRAS | v-Ki-ras2 Kirsten rat sarcoma viral oncogene homolog | 3845 | 12p | 25358180 | 25403854 | 4, 5, 6 |

As used herein, "NRAS" refers to neuroblastoma RAS viral (v-ras) oncogene homolog, the gene associated with GenBank Accession No. NM_002524, SEQ ID NO:1 (open reading frame is SEQ ID NO:2, nucleotides 255 to 824 of SEQ ID NO:1), encoding GenPept Accession No. NP_002515, SEQ ID NO:3). Other names for NRAS include Autoimmune Lymphoproliferative Syndrome type IV (ALPS4), NRAS1, and Noonan Syndrome 6 (NS6). NRAS functions

as an oncogene with GTPase activity and can be found on chromosome 1p. NRAS interacts with the cell membrane and various effector proteins, such as Raf and RhoA, which carry out its signaling function through the cytoskeleton and effects on cell adhesion (Fotiadou et al. (2007) Mol. Cel. Biol. 27:6742-6755).

As used herein, "KRAS" refers to v-Ki-ras2 Kirsten rat sarcoma viral oncogene homolog, the gene associated with GenBank Accession No. NM_004985, SEQ ID NO:4 (open reading frame is SEQ ID NO:5, nucleotides 182 to 748 of SEQ ID NO:4), encoding GenPept Accession No. NP_004976, SEQ ID NO:6, the predominant transcript variant of KRAS gene on chromosome 12. Other names for KRAS include KRAS2, and Noonan Syndrome 3 (NS3). KRAS functions as an oncogene with GTPase activity and can be found on chromosome 12. KRAS interacts with the cell membrane and various effector proteins, such as Akt and Cdc42, which carry out its signaling function through the cytoskeleton and effects on cell motility (Fotiadou et al. *supra*).

A "marker" as used herein, includes a material associated with a marker gene which has been identified as having a mutation in tumor cells of a patient and furthermore that mutation is characteristic of a patient whose outcome is favorable or unfavorable with treatment *e.g.*, by a proteasome inhibitor, e.g., a peptidyl boronic acid or peptidyl epoxy ketone. Examples of a marker include a material, e.g., a chromosome locus, DNA for a gene, RNA for a gene or protein for a gene. For example, a marker includes a marker gene material, e.g., a chromosome locus, DNA, RNA or protein whose mutation or characteristic, e.g., size, sequence, composition, activity or amount is indicative of a patient with a poor response to treatment; alternatively a marker includes a marker gene material, e.g., a chromosome locus, DNA, RNA or protein whose mutation or characteristic, e.g., size, sequence, composition, activity or amount is indicative of a patient with a good response. In another example, a marker includes a marker gene material, e.g., a chromosome locus, DNA, RNA or protein whose mutation or characteristic, e.g., size, sequence, composition, activity or amount is indicative of a patient whose disease has a short time-to-progression (TTP) upon treatment; alternatively a marker includes a marker gene material, e.g., a chromosome locus, DNA, RNA or protein whose mutation or characteristic, e.g., size, sequence, composition, activity or amount is indicative of a patient whose disease has a long TTP. Thus, as used herein, a marker is intended to include each and every one of these possibilities, and further can include each single marker individually or independently as a marker; or alternatively can include one or more, or all of the characteristics collectively when reference is made to "markers" or "marker sets."

In some embodiments, the marker is selected from the group consisting of nucleic acid and protein. In some embodiments, the marker is nucleic acid. In some embodiments, the nucleic acid marker is chromosomal or genomic DNA. In some embodiments, the nucleic acid is mRNA. In some embodiments, the nucleic acid is cDNA prepared from mRNA. In some embodiments, the marker is protein.

In the present invention, the characteristic is sequence.

In some embodiments, the marker is chromosome DNA, or genomic DNA and the characteristic is sequence. In some embodiments, the marker is mRNA and the characteristic is sequence. In some embodiments, the marker is cDNA and the characteristic is sequence.

In some embodiments, the marker is protein and the characteristic is sequence.

A chromosome locus marker useful to measure for determination of prognosis or treatment or disease management strategy is selected from the group consisting of chromosome 1p13.2 (NRAS), e.g., from base pair 115247085 to 115259515 and chromosome 12p12.1 (KRAS), e.g., from base pair 25358180 to 25403854. Chromosome locus and base pair numbers are based on the reference human genome Build 37.3 (current as of October 5, 2011) in the NCBI Gene database. A marker DNA, marker RNA or marker protein can correspond to base pairs on a chromosome locus marker. For example, a marker DNA can include genomic DNA from a chromosome locus marker, marker RNA can include a polynucleotide transcribed from a locus marker, and a marker protein can include a polypeptide resulting from expression at a chromosome locus marker in a sample, *e.g.*, comprising tumor cells.

A "marker nucleic acid" is a nucleic acid (*e.g.*, genomic DNA, mRNA, cDNA) encoded by, associated with or corresponding to a marker gene of the invention. Such marker nucleic acids include DNA, e.g., sense and anti-sense strands of genomic DNA (*e.g.*, including any introns occurring therein), comprising the entire or a partial sequence, e.g., one or more of the exons of the genomic DNA, up to and including the open reading frame of any of the marker genes or the complement of such a sequence. The marker nucleic acids also include RNA comprising the entire or a partial sequence of any marker or the complement of such a sequence, wherein all thymidine residues are replaced with uridine residues, RNA generated by transcription of genomic DNA (*i.e.* prior to splicing), RNA generated by splicing of RNA transcribed from genomic DNA, and proteins generated by translation of spliced RNA (*i.e.* including proteins both before and after cleavage of normally cleaved regions such as transmembrane signal sequences). As used herein, a "marker nucleic acid" may also include a cDNA made by reverse transcription of an RNA generated by transcription of genomic DNA (including spliced RNA). A marker nucleic acid also includes sequences which differ, due to degeneracy of the genetic code, from the nucleotide sequence of nucleic acids encoding a protein which corresponds to a marker of the invention, and thus encode the same protein or highly similar protein, e.g., wild type protein or protein with polymorphism but wild type function. As used herein, the phrase "allelic variant" refers to a nucleotide sequence which occurs at a given locus or to a polypeptide encoded by the nucleotide sequence. Such naturally occurring allelic variations can

typically result in 1-5% variance in the nucleotide sequence of a given gene. Alternative alleles can be identified by sequencing the gene of interest in a number of different individuals, e.g., in cells, e.g., germline cells, of individuals without cancer. This can be readily carried out by using hybridization probes to identify the same genetic locus in a variety of individuals. Detection of any and all such nucleotide variations and resulting amino acid polymorphisms or variations that are the result of naturally occurring allelic variation and that do not alter the functional activity of a wild type marker gene is intended to be within the scope of the wild type version of a marker described herein. A "marker protein" is a protein encoded by or corresponding to a marker, e.g., a nucleic acid, of the invention. The terms "protein" and "polypeptide' are used interchangeably. A protein of a marker specifically can be referred to by its name or amino acid sequence, but it is understood by those skilled in the art, that mutations, such as non-sense, missense or deletions can affect protein structure, appearance, cellular location and/or behavior in a variety of ways. Unless indicated otherwise, such differences are not distinguished herein, and a mutant marker described herein is intended to include any or all such varieties.

A typical tumor with a mutated RAS gene has a mutation in one RAS gene or another, not more than one. Mutations in codon 61 were most common for both NRAS and KRAS (85% and 47%, respectively), with mutations in codon13 in most of the other samples. While the majority of RAS mutations which indicate sensitivity or response to a proteasome inhibitor in hematological tumors, e.g., myeloma, *e.g.*, multiple myeloma, lymphoma, *e.g.*, mantle cell lymphoma or follicular lymphoma, or leukemia, e.g., acute myeloid leukemia or acute lymphoid leukemia, are in the NRAS gene, a small proportion can be in the KRAS gene. In general embodiments, a wild type RAS gene is predictive of favorable outcome, e.g., responsiveness and/or long time to progression, in hematological tumors in response to proteasome inhibitor treatment. In some embodiments, a wild type NRAS gene is predictive of a favorable outcome of treatment of a hematological tumor with a proteasome inhibitor. In some embodiments, a wild type NRAS gene is predictive of a favorable outcome of treatment of a hematological tumor with a peptidyl boronic acid. In some embodiments, a wild type NRAS gene is predictive of a favorable outcome of treatment of a myeloma tumor with a proteasome inhibitor. While myeloma tumor cell samples can have a mutated KRAS gene, data show that KRAS mutation alone is not predictive of response to bortezomib in multiple myeloma. Accordingly, a wild type RAS gene, e.g., NRAS, is predictive of favorable outcome, e.g., responsiveness and/or long time to progression, in myeloma tumors in response to proteasome inhibitor treatment, while a mutated NRAS gene is predictive of unfavorable outcome. Hematological malignancies with a higher proportion of tumors with a KRAS mutation include acute lymphoblastic leukemia, acute myeloid leukemia and lymphomas such as diffuse large B-cell lymphoma (DLBCL).

In the case of NRAS, an example of wild type NRAS nucleic acid, such as mRNA, is SEQ ID NO:1. In some embodiments, a mutation can be found in at least one mutation site of a NRAS marker. Examples of mutant NRAS, whose occurrence in a hematological cancer patient is indicative of nonresponse or unfavorable outcome, include marker nucleic acid with at least one change in at least one base of codon 12 (bases 34-36), codon 13 (bases 37-39) or codon 61 (bases 181-183) of SEQ ID NO:2, or the analogous codons in SEQ ID NO:1 (bases 288-290, bases 291-293 or bases 435-437, respectively of SEQ ID NO:1). In some embodiments, an allelic variant of NRAS has a change in a codon that is not codon 12, codon 13 or codon 61 of SEQ ID NO:2, or the analogous codons in SEQ ID NO:1, wherein the resulting encoded allelic variant protein has wild type RAS, e.g., GTPase, activity. Alternatively, an allelic variant of NRAS has a change in a base of codon 12, codon 13, or codon 61 wherein the change, e.g., is due to the degeneracy of the genetic code and does not result in a change in the translated residue. In the foregoing embodiments, an allelic variant nucleic acid encodes glycine at position 12 of SEQ ID NO:3, glycine at position 13 of SEQ ID NO:3 and glutamine at position 61 of SEQ ID NO:3 and the encoded polypeptide has wild type RAS activity, e.g., GTPase activity, and thus is not associated with unfavorable outcome.

A nucleic acid marker comprising a fragment of at least 10 consecutive nucleotides of SEQ ID NO:1, or a complement thereof, wherein the fragment comprises bases 288 to 290 can have a characteristic informative of a mutation of codon 12 if at least one base selected from the group consisting of bases 288, 289 and 290 is mutated in a manner that can result in a change of the amino acid residue 12 of SEQ ID NO:3. In some embodiments, the nucleic acid marker comprises bases 34 to 36 of SEQ ID NO:2. In some embodiments, a mutation in NRAS nucleic acid can result in a NRAS protein which has an amino acid residue selected from the group consisting of alanine, cysteine, aspartate, arginine, serine and valine instead of glycine at residue 12 of SEQ ID NO:3. A nucleic acid marker comprising a fragment of at least 10 consecutive nucleotides of SEQ ID NO:1, or a complement thereof, wherein the fragment comprises bases 291 to 293, can have a characteristic informative of a mutation of codon 13 if at least one base selected from the group consisting of bases 291, 292 and 293 is mutated in a manner that can result in a change of the amino acid residue 13 of SEQ ID NO:3. In some embodiments, the nucleic acid marker comprises bases 37 to 39 of SEQ ID NO:2. In some embodiments, a mutation in NRAS nucleic acid can result in a NRAS protein which has an amino acid residue selected from the group consisting of aspartate, valine, cysteine, serine, alanine and arginine instead of glycine at residue 13 of SEQ ID NO:3. A nucleic acid marker comprising a fragment of at least 10 consecutive nucleotides of SEQ ID NO:1, or a complement thereof, wherein the fragment comprises bases selected from the group consisting of bases 435 to 437, can have a characteristic informative of a mutation of codon 61 if at least one base selected from the

group consisting of bases 435, 436 and 437 is mutated in a manner that can result in a change of the amino acid residue 61 of SEQ ID NO:3. In some embodiments, the nucleic acid marker comprises bases 181 to 183 of SEQ ID NO:2. In some embodiments, a mutation in NRAS nucleic acid can result in a NRAS protein which has an amino acid residue selected from the group consisting of glutamate, histidine, lysine, leucine, proline and arginine instead of glutamine at residue 61 of SEQ ID NO:3.

As used herein, an "informative" characteristic, e.g., size, sequence, composition, activity or amount of a marker refers to a characteristic, e.g., size, sequence, composition, activity or amount whose difference is correlated to prognosis or outcome. The informative characteristic, e.g., size, sequence, composition, activity or amount of a marker can be obtained by analyzing either nucleic acid, *e.g.,* DNA or RNA, or protein corresponding to the marker gene. The characteristic, e.g., size (*e.g.*, length or molecular weight), sequence (e.g., nucleic acid sequence or protein sequence), composition (*e.g.*, base or amino acid composition or peptide digest or gene fragment pattern), activity (e.g., enzymatic activity or signaling activity) or amount (*e.g.,* copy number and/or expression level) of a marker, *e.g.,* a chromosome locus marker or a marker in a sample from a patient is "informative" if it is different than the wild type or allelic variant of the substance being analyzed. In some embodiments, a characteristic of a marker is informative if it indicates that the marker gene is wild type. In some embodiments where the amount of a marker is being measured, an amount is "informative" if it is greater than or less than a reference amount by a degree greater than the standard error of the assay employed to assess expression. The informative expression level of a marker can be determined upon statistical correlation of the measured expression level and the outcome, *e.g.*, good response, poor response, long time-to-progression, short time-to-progression, short term survival or long term survival. The result of the statistical analysis can establish a threshold for selecting markers to use in the methods described herein. Alternatively, a marker, *e.g.*, a chromosome locus marker, or a marker gene that has differential characteristic, e.g., size, sequence, composition, activity or amounts will have typical ranges of amounts that are predictive of outcome. An informative characteristic, e.g., size, sequence, composition, activity or amount is a characteristic, e.g., size, sequence, composition, activity or amount that falls within the range of characteristic, e.g., size, sequence, composition, activity or amounts determined for the outcome. Still further, a set of markers may together be "informative" if the combination of their characteristics, e.g., sizes, sequences, compositions, activities or amounts either meets or is above or below a pre-determined score for a marker, *e.g.*, a chromosome locus marker, or a marker gene set as determined by methods provided herein. Measurement of only one characteristic, e.g., marker, of a marker gene (i.e., DNA, RNA or protein) can provide a prognosis, i.e., predict or indicate outcome. Gene translocation, transcript splice variation, deletion and truncation are examples of events which can change marker size, sequence or composition, in addition to point mutations which can change marker sequence or composition. Measurement of more than one characteristic, e.g., marker, of a marker gene can provide a prognosis when the informative results of the two characteristics are consistent with each other, *i.e.,* the biologies of the results are not contradictory. Examples of consistent results from measurement of multiple characteristics of a marker gene can be identification of a nonsense mutation, point mutation, insertion or deletion in a DNA or RNA and a low amount or altered molecular weight of encoded protein, or a mutation in a region which encodes a binding pocket or active site of a protein and altered activity of the encoded protein. A different example can occur when a protein is in a pathway with a feedback loop controlling its synthesis based on its activity level. In this example, a low amount or activity of protein can be associated with a high amount of its mutated mRNA as a tissue, due to the marker gene mutation, thus is starved for the protein activity and repeatedly signals the production of the protein.

By way of non-limiting illustration, in the present case, an example of an informative result upon measuring a characteristic, e.g, a sequence, of a NRAS marker, e.g., DNA, mRNA, or protein, would be a result identifying the mutational status of a NRAS sequence, e.g., SEQ ID NO:1, 2 or 3. In the present case, identifying a mutation in the sequence would be informative of an unfavorable outcome, while identifying wild type sequence would be informative of a favorable outcome of treatment of the tumor with a proteasome inhibitor. In another example, measuring a characteristic, e.g, activity of a NRAS marker, e.g, protein, would provide an informative result of a favorable outcome if there is a high GTPase activity or a low signaling activity.

In some embodiments, a NRAS mutant in a hematological sample is predictive of unfavorable outcome in a patient whose tumor does not have a t(4;14) translocation. In some embodiments, the methods comprise identifying the mutational status of NRAS and the subtype of the tumor. In some embodiments, the subtype of the tumor is t(4;14) translocation. In some embodiments, a favorable outcome to treatment with a proteasome inhibitor is predicted if a hematological tumor has wild type NRAS and a t(4; 14) translocation.

A "normal" characteristic, e.g., size, sequence, composition, activity or amount of a marker may refer to the characteristic, e.g., size, sequence, composition, activity or amount in a "reference sample." A reference sample can be a matched normal, e.g., germline, sample from the same patient from whom the tumor, e.g., with a somatic mutation, is derived. A reference sample can be a sample from a healthy subject not having the marker-associated disease or a reference characteristic e.g., the average characteristic, e.g., size, sequence, composition, activity or amount of the wild type marker in several healthy subjects. A reference sample characteristic, e.g., size, sequence, composition or amount may be comprised of a characteristic, e.g., size, sequence, composition, activity or amount of one or more markers from

a reference database. Alternatively, a "normal" characteristic, e.g., size, sequence, composition, activity or level of expression of a marker is the characteristic, e.g., size, sequence, composition, activity or amount of the marker, *e.g.,* marker gene in non-tumor cells in a similar environment or response situation from the same patient from whom the tumor is derived. The normal amount of DNA copy number is 2 or diploid, with the exception of X-linked genes in males, where the normal DNA copy number is 1.

"Over-expression" and "under-expression" of a marker gene, refer to expression of the marker gene of a patient at a greater or lesser level (*e.g.* more than three-halves-fold, at least twofold, at least three-fold, greater or lesser level etc.), respectively, than normal level of expression of the marker gene, e.g., as measured by mRNA or protein, in a test sample that is greater than the standard error of the assay employed to assess expression. A "significant" expression level may refer to a level which either meets or is above or below a pre-determined score for a marker gene set as determined by methods provided herein.

As used herein, "gene deletion" refers to an amount of DNA copy number less than 2 and "amplification" refers to an amount of DNA copy number greater than 2. A "diploid" amount refers to a copy number equal to 2. The term "diploid or amplification" can be interpreted as "not deletion" of a gene copy. In a marker whose alternative informative amount is gene deletion, amplification generally would not be seen. Conversely, the term "diploid or deletion" can be interpreted as "not amplification" of copy number. In a marker whose alternative informative amount is amplification, gene deletion generally would not be seen. For the sake of clarity, sequence deletion can occur within a gene as a result of marker gene mutation and can result in absence of transcribed protein or a shortened mRNA or protein. Such a deletion may not affect copy number.

As used herein, a "favorable" outcome or prognosis refers to long time-to-progression (TTP, or progression-free survival), and/or good response. Conversely, an "unfavorable" outcome or prognosis refers to short term survival, short time-to-progression (TTP, or progression-free survival) and/or poor response.

The terms "long term survival" and "short term survival" refer to the length of time after receiving a first dose of treatment that a cancer patient is predicted to live. A "long term survivor" refers to a patient expected have a slower rate of progression or later death from the tumor than those patients identified as short term survivors. "Enhanced survival" or "a slower rate of death" are estimated life span determinations based upon characteristic, e.g., size, sequence, composition, activity or amount of one or more of markers described herein, e.g., as compared to a reference standard such that 70%, 80%, 90% or more of the population will be alive a sufficient time period after receiving a first dose of treatment. A "faster rate of death" or "shorter survival time" refer to estimated life span determinations based upon characteristic, e.g., size, sequence, composition, activity or amount of one or more of markers described herein, e.g., as compared to a reference standard such that 50%, 40%, 30%, 20%, 10% or less of the population will not live a sufficient time period after receiving a first dose of treatment. In some embodiments, the sufficient time period is at least 6, 12, 18,24 or 30 months measured from the first day of receiving a cancer therapy.

A cancer is "responsive" to a therapeutic agent or there is a "good response" to a treatment if its rate of growth is inhibited as a result of contact with the therapeutic agent, compared to its growth in the absence of contact with the therapeutic agent. Growth of a cancer can be measured in a variety of ways, for instance, the characteristic, e.g., size of a tumor or the expression of tumor markers appropriate for that tumor type may be measured. For example, the response definitions used to support the identification of markers associated with myeloma and its response to an proteasome inhibitor, e.g., peptidyl boronic acid therapy, the Southwestern Oncology Group (SWOG) criteria as described in Blade et al. (1998) Br J Haematol. 102:1115-23 can be used. These criteria define the type of response measured in myeloma and also the characterization of time to disease progression which is another important measure of a tumor’s sensitivity to a therapeutic agent. For solid tumors, the Response Evaluation Criteria in Solid Tumors (RECIST) guidelines (Eisenhauer et al. (2009) E. J. Canc. 45:228-247) can be used to support the identification of markers associated with solid tumors and response of solid tumors to a proteasome inhibitor. International Working Groups convene periodically to set, update and publish response criteria for various types of cancers. Such published reports can be followed to support the identification of markers of the subject tumors and their response to proteasome inhibitors. Examples are criteria for Acute Myelogenous Leukemia (AML, Cheson et al. (2003) J. Clin. Oncol. 21:4642-4649), lymphomas, e.g., non-Hodgkin’s and Hodgkin’s lymphoma (Cheson et al. (2007) J. Clin. Oncol. 25:579-596). Criteria take into account analysis methods such as Positron Emission Tomography (PET), e.g., for identifying sites with measurable altered metabolic activity (e.g., at tumor sites) or to trace specific markers into tumors *in vivo,* immunohistochemistry, e.g., to identify tumor cells by detecting binding of antibodies to specific tumor markers, and flow cytometry, e.g., to characterize cell types by differential markers and fluorescent stains, in addition to traditional methods such as histology to identify cell composition (e.g., blast counts in a blood smear or a bone marrow biopsy, presence and number of mitotic figures) or tissue structure (e.g., disordered tissue architecture or cell infiltration of basement membrane). The quality of being responsive to a proteasome inhibitor, e.g., a peptidyl boronic acid therapy can be a variable one, with different cancers exhibiting different levels of "responsiveness" to a given therapeutic agent, under different conditions. Still further, measures of responsiveness can be assessed using additional criteria beyond growth size of a tumor, including patient quality of life, degree of metastases, etc. In addition, clinical prognostic markers and variables can be assessed (e.g., M protein

in myeloma, PSA levels in prostate cancer) in applicable situations.

A cancer is "non-responsive" or has a "poor response" to a therapeutic agent or there is a poor response to a treatment if its rate of growth is not inhibited, or inhibited to a very low degree, as a result of contact with the therapeutic agent when compared to its growth in the absence of contact with the therapeutic agent. As stated above, growth of a cancer can be measured in a variety of ways, for instance, the size of a tumor or the expression of tumor markers appropriate for that tumor type may be measured. For example, the response definitions used to support the identification of markers associated with non- response of tumors to therapeutic agents, guidelines such as those described above can be used. The quality of being non-responsive to a therapeutic agent can be a highly variable one, with different cancers exhibiting different levels of "non-responsiveness" to a given therapeutic agent, under different conditions. Still further, measures of non-responsiveness can be assessed using additional criteria beyond growth size of a tumor, including patient quality of life, degree of metastases, etc. In addition, clinical prognostic markers and variables can be assessed (e.g., M protein in myeloma, PSA levels in prostate cancer) in applicable situations.

As used herein, "long time-to-progression, "long TTP" and "short time-to-progression," "short TTP" refer to the amount of time until when the stable disease brought by treatment converts into an active disease. On occasion, a treatment results in stable disease which is neither a good nor a poor response, *e.g.,* MR, the disease merely does not get worse, *e.g.,* become a progressive disease, for a period of time. This period of time can be at least 4-8 weeks, at least 3-6 months or more than 6 months.

"Treatment" shall mean the use of a therapy to prevent or inhibit further tumor growth, as well as to cause shrinkage of a tumor, and to provide longer survival times. Treatment is also intended to include prevention of metastasis of tumor. A tumor is "inhibited" or "treated" if at least one symptom (as determined by responsiveness/non-responsiveness, time to progression, or indicators known in the art and described herein) of the cancer or tumor is alleviated, terminated, slowed, minimized, or prevented. Any amelioration of any symptom, physical or otherwise, of a tumor pursuant to treatment using a therapeutic regimen (*e.g.*, proteasome inhibitor, e.g., a peptidyl boronic acid regimen) as further described herein, is within the scope of the present disclosure.

As used herein, the term "agent" is defined broadly as anything that cancer cells, including tumor cells, may be exposed to in a therapeutic protocol. In the context of the present invention, such agents include, but are not limited to, proteasome inhibitor, e.g., a peptidyl boronic acid agents, as well as chemotherapeutic agents as known in the art and described in further detail herein.

The term "probe" refers to any molecule which is capable of selectively binding to a specifically intended target molecule, for example a marker of the invention. Probes can be either synthesized by one skilled in the art, or derived from appropriate biological preparations. For purposes of detection of the target molecule, probes may be specifically designed to be labeled, as described herein. Examples of molecules that can be utilized as probes include, but are not limited to, RNA, DNA, proteins, antibodies, and organic monomers.

"Complementary" refers to the broad concept of sequence complementarity between regions of two nucleic acid strands or between two regions of the same nucleic acid strand. It is known that an adenine residue of a first nucleic acid region is capable of forming specific hydrogen bonds ("base pairing") with a residue of a second nucleic acid region which is antiparallel to the first region if the residue is thymine or uracil. Similarly, it is known that a cytosine residue of a first nucleic acid strand is capable of base pairing with a residue of a second nucleic acid strand which is antiparallel to the first strand if the residue is guanine. A first region of a nucleic acid is complementary to a second region of the same or a different nucleic acid if, when the two regions are arranged in an antiparallel fashion, at least one nucleotide residue of the first region is capable of base pairing with a residue of the second region. In some embodiments, the first region comprises a first portion and the second region comprises a second portion, whereby, when the first and second portions are arranged in an antiparallel fashion, at least about 50%, at least about 75%, at least about 90%, or at least about 95% or all of the nucleotide residues of the first portion are capable of base pairing with nucleotide residues in the second portion.

"Homologous" as used herein, refers to nucleotide sequence similarity between two regions of the same nucleic acid strand or between regions of two different nucleic acid strands. When a nucleotide residue position in both regions is occupied by the same nucleotide residue, then the regions are homologous at that position. A first region is homologous to a second region if at least one nucleotide residue position of each region is occupied by the same residue. Homology between two regions is expressed in terms of the proportion of nucleotide residue positions of the two regions that are occupied by the same nucleotide residue (i.e., by percent identity). By way of example, a region having the nucleotide sequence 5'-ATTGCC-3' and a region having the nucleotide sequence 5'-TATGGC-3' share homology with 50% identity. In one embodiment, the first region comprises a first portion and the second region comprises a second portion, whereby, at least about 50%, at least about 75%, at least about 90%, or at least about 95% of the nucleotide residue positions of each of the portions are occupied by the same nucleotide residue. In some embodiments of 100% identity, all nucleotide residue positions of each of the portions are occupied by the same nucleotide residue.

Unless otherwise specified herewithin, the terms "antibody" and "antibodies" broadly encompass naturally-occurring forms of antibodies, e.g., polyclonal antibodies (e.g., IgG, IgA, IgM, IgE) and monoclonal and recombinant

antibodies such as single-chain antibodies, two-chain and multi-chain proteins, chimeric, CDR-grafted, human and humanized antibodies and multi-specific antibodies, as well as fragments and derivatives of all of the foregoing, which fragments (e.g., dAbs, scFv, Fab, $F(ab)'_2$, Fab') and derivatives have at least an antigenic binding site. Antibody derivatives may comprise a protein or chemical moiety conjugated to an antibody. The term "antibody" also includes synthetic and genetically engineered variants.

A "kit" is any article of manufacture (*e.g.*, a package or container) comprising at least one reagent, *e.g.* a probe, for specifically detecting a marker or marker set of the invention. The article of manufacture may be promoted, distributed, sold or offered for sale as a unit for performing, e.g., *in vitro,* the methods of the present invention, e.g., on a sample having been obtained from a patient. The reagents included in such a kit can comprise probes/primers and/or antibodies for use in analyzing one or more markers described herein. In addition, a kit of the present invention can contain instructions which describe a suitable detection assay. Such a kit can be conveniently used, *e.g.*, in a clinical or a contract testing setting, to generate information, e.g., on expression levels, characteristic, e.g., size, sequence, activity or composition of one or more marker, to be recorded, stored, transmitted or received to allow for diagnosis, evaluation or treatment of patients exhibiting symptoms of cancer, in particular patients exhibiting the possible presence of a cancer capable of treatment with proteasome inhibition therapy, including, *e.g.*, hematological cancers *e.g.,* myelomas (*e.g.,* multiple myeloma), lymphomas (*e.g.,* mantle cell lymphoma or follicular lymphoma), or leukemia (e.g., acute myeloid leukemia or acute lymphoid leukemia).

The present methods and compositions are designed for use in diagnostics and therapeutics for a patient suffering from cancer. A cancer or tumor is treated or diagnosed according to the present methods. "Cancer" or "tumor" is intended to include any neoplastic growth in a patient, including an initial tumor and any metastases. The cancer can be of the hematological or solid tumor type. Hematological tumors include tumors of hematological origin, including, *e.g.,* myelomas (*e.g.,* multiple myeloma), leukemias (*e.g.,* Waldenstrom's syndrome, chronic lymphocytic leukemia, acute myelogenous leukemia, chronic myelogenous leukemia, other leukemias), lymphomas (*e.g.*, B-cell lymphomas, non-Hodgkin's lymphoma) and myelodysplastic syndrome. Solid tumors can originate in organs, and include cancers such as in skin, lung, brain, breast, prostate, ovary, colon, kidney, pancreas, liver, esophagus, stomach, intestine, bladder, uterus, cervix, testis, adrenal gland, etc. The cancer can comprise a cell in which a marker gene has a mutation. As used herein, cancer cells, including tumor cells, refer to cells that divide at an abnormal (increased) rate or whose control of growth or survival is different than for cells in the same tissue where the cancer cell arises or lives. Cancer cells include, but are not limited to, cells in carcinomas, such as squamous cell carcinoma, basal cell carcinoma, sweat gland carcinoma, sebaceous gland carcinoma, adenocarcinoma, papillary carcinoma, papillary adenocarcinoma, cystadenocarcinoma, medullary carcinoma, undifferentiated carcinoma, bronchogenic carcinoma, melanoma, renal cell carcinoma, hepatoma-liver cell carcinoma, bile duct carcinoma, cholangiocarcinoma, papillary carcinoma, transitional cell carcinoma, choriocarcinoma, semonoma, embryonal carcinoma, mammary carcinomas, gastrointestinal carcinoma, colonic carcinomas, bladder carcinoma, prostate carcinoma, and squamous cell carcinoma of the neck and head region; sarcomas, such as fibrosarcoma, myxosarcoma, liposarcoma, chondrosarcoma, osteogenic sarcoma, chordosarcoma, angiosarcoma, endotheliosarcoma, lymphangiosarcoma, synoviosarcoma and mesotheliosarcoma; hematologic cancers, such as myelomas, leukemias (*e.g.*, acute myelogenous leukemia, chronic lymphocytic leukemia, granulocytic leukemia, monocytic leukemia, lymphocytic leukemia), and lymphomas (*e.g.*, follicular lymphoma, mantle cell lymphoma, diffuse large Bcell lymphoma, malignant lymphoma, plasmocytoma, reticulum cell sarcoma, or Hodgkins disease); and tumors of the nervous system including glioma, meningoma, medulloblastoma, schwannoma or epidymoma.

As used herein, the term "noninvasive" refers to a procedure which inflicts minimal harm to a subject. In the case of clinical applications, a noninvasive sampling procedure can be performed quickly, e.g., in a walk-in setting, typically without anaesthesia and/or without surgical implements or suturing. Examples of noninvasive samples include blood, serum, saliva, urine, buccal swabs, throat cultures, stool samples and cervical smears. Noninvasive diagnostic analyses include x-rays, magnetic resonance imaging, positron emission tomography, etc.

Described herein is the assessment of outcome for treatment of a tumor through measurement of the amount of pharmacogenomic markers, e.g, the mutation status of RAS. Methods of the invention can be characterized as comprising detecting, in a sample of cells or nucleic acid from the patient, the presence or absence of wild type or mutant NRAS gene. The mutations can be: (i) a difference in the identity of at least one nucleotide or (ii) a difference in the number of nucleotides, which difference can be a single nucleotide or several nucleotides. The invention also provides methods for detecting differences in NRAS genes such as chromosomal rearrangements, *e.g.,* chromosomal dislocation. Also described are assessing the outcome by noninvasive, convenient or low-cost means, for example, from blood samples. Typical methods to determine extent of cancer or outcome of a hematological tumor, *e.g.,* myeloma, *e.g.,* multiple myeloma, lymphoma, *e.g.,* mantle cell lymphoma or follicular lymphoma, or leukemia, e.g., acute myeloid leukemia or acute lymphoid leukemia can employ bone marrow biopsy to collect tissue for genotype or phenotype, *e.g.*, histological analysis. The invention provides methods for determining, assessing, advising or providing an appropriate therapy regimen for treating a tumor or managing disease in a patient. Monitoring a treatment using the kits and methods disclosed herein can identify the potential for unfavorable outcome and allow their prevention, and thus a savings in

morbidity, mortality and treatment costs through adjustment in the therapeutic regimen, cessation of therapy or use of alternative therapy.

The term "sample" is intended to include a sample, e.g, tissue, cells, biological fluids and isolates thereof, isolated from a subject, as well as tissues, cells and fluids present within a subject and can be obtained from a patient or a normal subject. In hematological tumors of the bone marrow, *e.g.,* myeloma tumors, primary analysis of the tumor can be performed on bone marrow samples, e.g., samples which comprise myeloma tumor cells. However, some tumor cells, (*e.g.*, clonotypic tumor cells, circulating endothelial cells), are a percentage of the cell population in whole blood. These cells also can be mobilized into the blood during treatment of the patient with granulocyte-colony stimulating factor (G-CSF) in preparation for a bone marrow transplant, a standard treatment for hematological tumors, *e.g.,* leukemias, lymphomas and myelomas. Examples of circulating tumor cells in multiple myeloma have been studied e.g., by Pilarski et al. (2000) Blood 95:1056-65 and Rigolin et al. (2006) Blood 107:2531-5. Thus, noninvasive samples, *e.g.*, for *in vitro* measurement of markers to determine outcome of treatment, can include peripheral blood samples. Accordingly, cells within peripheral blood can be tested for marker amount. For patients with hematological tumors, a control, reference sample for normal characteristic, e.g., size, sequence, composition, activity or amount can be obtained from skin or a buccal swab of the patient. For solid tumors, a typical sample comprising tumor cells is a biopsy of the tumor. For solid tumors, a control, reference sample for normal characteristic, e.g., size, sequence, composition, activity or amount can be obtained from blood of the patient.

In some embodiments, hematological tumor cells are selected from the group consisting of myeloma cancer cells, lymphoma cancer cells and leukemia cancer cells. In some embodiments, a sample comprising hematological tumor cells are selected from the group consisting of multiple myeloma cancer cells, mantle cell lymphoma cancer cells, follicular lymphoma cancer cells, acute lymphoid leukemia cancer cells and acute myeloid leukemia cancer cells. In some embodiments, a sample comprising hematological tumor cells comprises multiple myeloma cancer cells. In some embodiments, a sample comprising hematological tumor cells comprises mantle cell lymphoma cancer cells.

Blood collection containers can comprise an anti-coagulant, *e.g.*, heparin or ethylenediaminetetraacetic acid (EDTA), sodium citrate or citrate solutions with additives to preserve blood integrity, such as dextrose or albumin or buffers, *e.g*., phosphate. If the amount of marker is being measured by measuring the level of its DNA in the sample, a DNA stabilizer, *e.g*., an agent that inhibits DNAse, can be added to the sample. If the amount of marker is being measured by measuring the level of its RNA in the sample, an RNA stabilizer, *e.g*., an agent that inhibits RNAse, can be added to the sample. If the amount of marker is being measured by measuring the level of its protein in the sample, a protein stabilizer, *e.g.,* an agent that inhibits proteases, can be added to the sample. An example of a blood collection container is PAXGENE® tubes (PREANALYTIX, Valencia, CA), useful for RNA stabilization upon blood collection. Peripheral blood samples can be modified, *e.g.,* fractionated, sorted or concentrated (*e.g.,* to result in samples enriched with tumor or depleted of tumor (e.g., for a reference sample)). Examples of modified samples include clonotypic myeloma cells, which can be collected by *e.g.,* negative selection, *e.g.,* separation of white blood cells from red blood cells (*e.g.,* differential centrifugation through a dense sugar or polymer solution (*e.g.,* FICOLL® solution (Amersham Biosciences division of GE healthcare, Piscataway, NJ) or HISTOPAQUE®-1077 solution, Sigma-Aldrich Biotechnology LP and Sigma-Aldrich Co., St. Louis, MO)) and/or positive selection by binding B cells to a selection agent (*e.g.,* a reagent which binds to a tumor cell or myeloid progenitor marker, such as CD34, CD38, CD138, or CD 133, for direct isolation (*e.g.*, the application of a magnetic field to solutions of cells comprising magnetic beads (*e.g.*, from Miltenyi Biotec, Auburn, CA) which bind to the B cell markers) or fluorescent-activated cell sorting).

Alternatively, a tumor cell line, *e.g.,* OCI-Ly3, OCI-Ly10 cell (Alizadeh et al. (2000) Nature 403:503-511), a RPMI 6666 cell, a SUP-B15 cell, a KG-1 cell, a CCRF-SB cell, an 8ES cell, a Kasumi-1 cell, a Kasumi-3 cell, a BDCM cell, an HL-60 cell, a Mo-B cell, a JM1 cell, a GA-10 cell or a B-cell lymphoma (*e.g*., BC-3) or a cell line or a collection of tumor cell lines (see e.g., McDermott et al. (2007) PNAS 104:19936-19941 or ONCOPANEL™ anti-cancer tumor cell profiling screen (Ricerca Biosciences, Bothell, WA)) can be assayed. A skilled artisan readily can select and obtain the appropriate cells *(e.g.,* from American Type Culture Collection (ATCC®), Manassas, VA) that are used in the present method. If the compositions or methods are being used to predict outcome of treatment in a patient or monitor the effectiveness of a therapeutic protocol, then a tissue or blood sample having been obtained from the patient being treated is a useful source of cells or marker gene or gene products for an assay.

The sample, *e.g.,* tumor, e.g., biopsy or bone marrow, blood or modified blood, (*e.g.,* comprising tumor cells) and/or the reference, e.g., matched control (e.g., germline), sample can be subjected to a variety of well-known post-collection preparative and storage techniques (*e.g*., nucleic acid and/or protein extraction, fixation, storage, freezing, ultrafiltration, concentration, evaporation, centrifugation, *etc*.) prior to assessing the amount of the marker in the sample.

In some embodiments, a mutation in a marker can be identified by sequencing a nucleic acid, e.g., a DNA, RNA, cDNA or a protein correlated with the marker gene, e.g., NRAS. There are several sequencing methods known in the art to sequence nucleic acids. A nucleic acid primer can be designed to bind to a region comprising a potential mutation site or can be designed to complement the mutated sequence rather than the wild type sequence. Primer pairs can be designed to bracket a region comprising a potential mutation in a marker gene. A primer or primer pair can be

used for sequencing one or both strands of DNA corresponding to the marker gene. A primer can be used in conjunction with a probe, e.g., a nucleic acid probe, e.g., a hybridization probe, to amplify a region of interest prior to sequencing to boost sequence amounts for detection of a mutation in a marker gene. Examples of regions which can be sequenced include an entire gene, transcripts of the gene and a fragment of the gene or the transcript, e.g., one or more of exons or untranslated regions or a portion of a marker comprising a mutation site. Examples of mutations to target for primer selection and sequence or composition analysis can be found in public databases which collect mutation information, such as COSMIC and dbGaP. Some mutations of a marker gene such as NRAS are listed in Table 11 in the Examples as examples of mutations that can be associated with resistance to proteasome inhibition, e.g., inhibition by a peptidyl boronic acid, e.g., bortezomib or ixazomib citrate.

Sequencing methods are known to one skilled in the art. Examples of methods include the Sanger method, the SEQUENOM™ method and Next Generation Sequencing (NGS) methods. The Sanger method, comprising using electrophoresis, e.g., capillary electrophoresis to separate primer-elongated labeled DNA fragments, can be automated for high-throughput applications. The primer extension sequencing can be performed after PCR amplification of regions of interest. Software can assist with sequence base calling and with mutation identification. SEQUENOM™ MASSARRAY® sequencing analysis (San Diego, CA) is a mass-spectrometry method which compares actual mass to expected mass of particular fragments of interest to identify mutations. NGS technology (also called "massively parallel sequencing" and "second generation sequencing") in general provides for much higher throughput than previous methods and uses a variety of approaches (reviewed in Zhang et al. (2011) J. Genet. Genomics 38:95-109 and Shendure and Hanlee (2008) Nature Biotech. 26:1135-1145). NGS methods can identify low frequency mutations in a marker in a sample. Some NGS methods (see, e.g., GS-FLX Genome Sequencer (Roche Applied Science, Branford, CT), Genome analyzer (Illumina, Inc. San Diego, CA) SOLID™ analyzer (Applied Biosystems, Carlsbad, CA), Polonator G.007 (Dover Systems, Salem, NH), HELISCOPE™ (Helicos Biosciences Corp., Cambridge, MA)) use cyclic array sequencing, with or without clonal amplification of PCR products spatially separated in a flow cell and various schemes to detect the labeled modified nucleotide that is incorporated by the sequencing enzyme (e.g., polymerase or ligase). In one NGS method, primer pairs can be used in PCR reactions to amplify regions of interest. Amplified regions can be ligated into a concatenated product. Clonal libraries are generated in the flow cell from the PCR or ligated products and further amplified ("bridge" or "cluster" PCR) for single-end sequencing as the polymerase adds a labeled, reversibly terminated base that is imaged in one of four channels, depending on the identity of the labeled base and then removed for the next cycle. Software can aid in the comparison to genomic sequences to identify mutations. Another NGS method is exome sequencing, which focuses on sequencing exons of all genes in the genome. As with other NGS methods, exons can be enriched by capture methods or amplification methods.

Composition of proteins and nucleic acids can be determined by many ways known in the art, such as by treating them in ways that cleave, degrade or digest them and then analyzing the components. Mass spectrometry, electrophoresis and chromatography can separate and define components for comparison. Mutations which cause deletions or insertions can be identified by size or charge differences in these methods. Protein digestion or restriction enzyme nucleic acid digestion can reveal different fragment patterns after some mutations. Antibodies that recognize particular mutant amino acids in their structural contexts can identify and detect these mutations in samples (see below).

In some embodiments, DNA, e.g., genomic DNA corresponding to the wild type or mutated marker can be analyzed both by *in situ* and by *in vitro* formats in a biological sample using methods known in the art. DNA can be directly isolated from the sample or isolated after isolating another cellular component, e.g., RNA or protein. Kits are available for DNA isolation, e.g., QIAAMP® DNA Micro Kit (Qiagen, Valencia, CA). DNA also can be amplified using such kits.

In another embodiment, mRNA corresponding to the marker can be analyzed both by *in situ* and by *in vitro* formats in a biological sample using methods known in the art. An example of a method for measuring expression level is included in the Examples. Many expression detection methods use isolated RNA. For *in vitro* methods, any RNA isolation technique that does not select against the isolation of mRNA can be utilized for the purification of RNA from tumor cells (see, *e.g.*, Ausubel et al., ed., Current Protocols in Molecular Biology, John Wiley & Sons, New York 1987-1999). Additionally, large numbers of tissue samples can readily be processed using techniques well known to those of skill in the art, such as, for example, the single-step RNA isolation process of Chomczynski (1989, U.S. Patent No. 4,843,155). RNA can be isolated using standard procedures (see *e.g.*, Chomczynski and Sacchi (1987) Anal. Biochern.162:156-159), solutions (*e.g.,* trizol, TRI REAGENT® (Molecular Research Center, Inc., Cincinnati, OH; see U.S. Patent No. 5,346,994) or kits (*e.g.*, a QIAGEN® Group RNEASY® isolation kit (Valencia, CA) or LEUKOLOCK™ Total RNA Isolation System, Ambion division of Applied Biosystems, Austin, TX).

Additional steps may be employed to remove DNA from RNA samples. Cell lysis can be accomplished with a nonionic detergent, followed by microcentrifugation to remove the nuclei and hence the bulk of the cellular DNA. DNA subsequently can be isolated from the nuclei for DNA analysis. In one embodiment, RNA is extracted from cells of the various types of interest using guanidinium thiocyanate lysis followed by CsCl centrifugation to separate the RNA from DNA (Chirgwin et al. (1979) Biochemistry 18:5294-99). Poly(A)+RNA is selected by selection with oligo-dT cellulose

(see Sambrook et al. (1989) Molecular Cloning--A Laboratory Manual (2nd ed.), Cold Spring Harbor Laboratory, Cold Spring Harbor, N.Y.). Alternatively, separation of RNA from DNA can be accomplished by organic extraction, for example, with hot phenol or phenol/chloroform/isoamyl alcohol. If desired, RNAse inhibitors may be added to the lysis buffer. Likewise, for certain cell types, it may be desirable to add a protein denaturation/digestion step to the protocol. For many applications, it is desirable to enrich mRNA with respect to other cellular RNAs, such as transfer RNA (tRNA) and ribosomal RNA (rRNA). Most mRNAs contain a poly(A) tail at their 3' end. This allows them to be enriched by affinity chromatography, for example, using oligo(dT) or poly(U) coupled to a solid support, such as cellulose or SEPHADEX.R™. medium (see Ausubel et al. (1994) Current Protocols In Molecular Biology, vol. 2, Current Protocols Publishing, New York). Once bound, poly(A)+mRNA is eluted from the affinity column using 2 mM EDTA/0.1% SDS.

Analyzing a characteristic of a marker of the invention in a biological sample involves obtaining a biological sample (*e.g.*, a bone marrow sample, a tumor biopsy or a reference sample) from a test subject. The characteristic can be assessed by any of a wide variety of well known methods for detecting or measuring the characteristic, e.g., of a marker or plurality of markers, e.g., of a nucleic acid (*e.g.*, RNA, mRNA, genomic DNA, or cDNA) and/or translated protein. Non-limiting examples of such methods include immunological methods for detection of secreted, cell-surface, cytoplasmic, or nuclear proteins, protein purification methods, protein function or activity assays, nucleic acid hybridization methods, optionally including "mismatch cleavage" steps (Myers, et al. (1985) Science 230:1242) to digest mismatched, i.e. mutant or variant, regions and separation and identification of the mutant or variant from the resulting digested fragments, nucleic acid reverse transcription methods, and nucleic acid amplification methods and analysis of amplified products. These methods include gene array/chip technology, RT-PCR, TAQMAN® gene expression assays (Applied Biosystems, Foster City, CA), e.g., under GLP approved laboratory conditions, *in situ* hybridization, immunohistochemistry, immunoblotting, FISH (flourescence *in situ* hybridization), FACS analyses, northern blot, southern blot, INFINIUM® DNA analysis Bead Chips (Illumina, Inc., San Diego, CA), quantitative PCR, bacterial artificial chromosome arrays, single nucleotide polymorphism (SNP) arrays (Affymetrix, Santa Clara, CA) or cytogenetic analyses.

Examples of techniques for detecting differences of at least one nucleotide between two nucleic acids include, but are not limited to, selective oligonucleotide hybridization, selective amplification, or selective primer extension. For example, oligonucleotide probes can be prepared in which the known polymorphic nucleotide is placed centrally (allele- or mutant-specific probes) and then hybridized to target DNA under conditions which permit hybridization only if a perfect match is found (Saiki et al. (1986) Nature 324:163); Saiki et al (1989) Proc. Natl Acad. Sci USA 86:6230; and Wallace et al. (1979) Nucl. Acids Res. 6:3543). Such allele specific oligonucleotide hybridization techniques can be used for the simultaneous detection of several nucleotide changes in different polymorphic or mutated regions of NRAS. For example, oligonucleotides having nucleotide sequences of specific allelic variants or mutants are attached to a solid support, e.g., a hybridizing membrane and this support, e.g., membrane, is then hybridized with labeled sample nucleic acid. Analysis of the hybridization signal thus can reveal the identity of the nucleotides of the sample nucleic acid.

The detection methods of the invention are used to detect RNA, mRNA, protein, cDNA, or genomic DNA, for example, in a biological sample *in vitro.* Also described herein are *in vivo* techniques for detection of a polypeptide or nucleic acid corresponding to a marker of the invention which include introducing into a subject a labeled probe to detect the biomarker, *e.g.*, a nucleic acid complementary to the transcript of a biomarker or a labeled antibody, Fc receptor or antigen directed against the polypeptide, *e.g.*, wild type or mutant marker. For example, the antibody can be labeled with a radioactive isotope whose presence and location in a subject can be detected by standard imaging techniques. These assays can be conducted in a variety of ways. A skilled artisan can select from these or other appropriate and available methods based on the nature of the marker(s), tissue sample and mutation in question. Some methods are described in more detail in later sections. Different methods or combinations of methods could be appropriate in different cases or, for instance in different types of tumors or patient populations.

*In vitro* techniques for detection of a polypeptide corresponding to a marker of the invention include enzyme linked immunosorbent assays (ELISAs), Western blots, protein array, immunoprecipitations, immunochemistry and immunofluorescence. In such examples, expression of a marker is assessed using an antibody (*e.g.*, an unlabeled, a radiolabeled, chromophore-labeled, fluorophore-labeled, or enzyme-labeled antibody), an antibody derivative (*e.g.*, an antibody conjugated with a substrate or with the protein or ligand of a protein-ligand pair (*e.g.,* biotin-streptavidin)), or an antibody fragment (*e.g.,* a single-chain antibody, an isolated antibody hypervariable domain, *etc*.) which binds specifically with a marker protein or fragment thereof, e.g., a protein or fragment comprising a region which can be mutated or a portion comprising a mutated sequence, or a mutated residue in its structural context, including a marker protein which has undergone all or a portion of its normal post-translational modification. An antibody can detect a protein with an amino acid sequence selected from the group consisting of SEQ ID NO:3 and 6. Alternatively, an antibody can detect a mutated protein with a variant amino acid sequence selected from the group consisting of a mutant of SEQ ID NO:3 and 6. Residues listed as mutated in public databases such as COSMIC of dbGaP can be prepared in immunogenic compositions for generation of antibodies that will specifically recognize and bind to the mutant residues. Another method can employ pairs of antibodies, wherein one of the pair would bind a marker protein upstream, i.e. N-terminal to the region of expected mutation, e.g., nonsense mutation, point mutation, insertion or deletion and the other of the pair would

bind the protein downstream. Wild type protein would bind both antibodies of the pair, but a protein with a nonsense mutation, point mutation, insertion or deletion mutation would bind only the N-terminal antibody of the pair. An assay such as a sandwich ELISA assay could detect a loss of quantity of the wild type protein in the tumor sample, e.g., in comparison to the reference sample, or a standard ELISA would comparison of the levels of binding of the antibodies to infer that a mutation is present in a tumor sample.

In some embodiments, the method includes measuring the amount of marker protein. In some embodiments, an assay to measure marker protein expression uses an antibody which binds to SEQ ID NO:3 or 6. In some embodiments, quantification of protein expression measures, in a sample comprising tumor cells, the amount of binding an antibody which binds to SEQ ID NO:3 or 6. In some embodiments, the amount of marker protein is quantified by immunohistochemistry of a tumor biopsy. In some embodiments, the amount of marker protein is quantified by immunohistochemistry of tumor cell enriched from blood. In some embodiments, the amount of marker protein is determined by a score of antibody binding or staining intensity. In some embodiments, the amount of marker protein is determined by comparison of the antibody binding or staining in a tumor cell with a non-tumor cell in the sample comprising tumor cells.

Indirect methods for determining the amount or functionality of a protein marker also include measurement of the activity of the protein. For example, a sample, or a protein isolated from the sample or expressed from nucleic acid isolated, cloned or amplified from the sample can be assessed for marker protein activity. For a RAS oncogene, an activating mutation can be measured as reduced GTPase activity or altered binding to RasGAP or a cell membrane.

In one embodiment, expression of a marker is assessed by preparing mRNA/cDNA (*i.e.,* a transcribed polynucleotide) from cells in a patient sample, and by hybridizing the mRNA/cDNA with a reference polynucleotide which is a complement of a marker nucleic acid, or a fragment thereof. cDNA can, optionally, be amplified using any of a variety of polymerase chain reaction methods prior to hybridization with the reference polynucleotide. Expression of one or more markers likewise can be detected using quantitative PCR to assess the level of expression of the marker(s). An example of the use of measuring mRNA levels is that an inactivating mutation in a marker gene can result in an altered level of mRNA in a cell. The level can be upregulated due to feedback signaling protein production in view of nonfunctional or absent protein or downregulated due to instability of an altered mRNA sequence. Alternatively, any of the many known methods of detecting mutations or variants (e.g. single nucleotide polymorphisms, deletions, etc., discussed above) of a marker of the invention may be used to detect occurrence of a mutation in a marker gene in a patient.

An example of direct measurement is quantification of transcripts. As used herein, the level or amount of expression refers to the absolute amount of expression of an mRNA encoded by the marker or the absolute amount of expression of the protein encoded by the marker. As an alternative to making determinations based on the absolute expression amount of selected markers, determinations may be based on normalized expression amounts. Expression amount can be normalized by correcting the absolute expression level of a marker upon comparing its expression to the expression of a control marker that is not a marker, e.g., in a housekeeping role that is constitutively expressed. Suitable markers for normalization also include housekeeping genes, such as the actin gene or beta-2 microglobulin. Reference markers for data normalization purposes include markers which are ubiquitously expressed and/or whose expression is not regulated by oncogenes. Constitutively expressed genes are known in the art and can be identified and selected according to the relevant tissue and/or situation of the patient and the analysis methods. Such normalization allows one to compare the expression level in one sample, to another sample, e.g., between samples from different times or different subjects. Further, the expression level can be provided as a relative expression level. The baseline of a genomic DNA sample, *e.g.*, diploid copy number, can be determined by measuring amounts in cells from subjects without a tumor or in non-tumor cells from the patient. To determine a relative amount of a marker or marker set, the amount of the marker or marker set is determined for at least 1, or 2, 3, 4, 5, or more samples, *e.g.*, 7, 10, 15, 20 or 50 or more samples in order to establish a baseline, prior to the determination of the expression level for the sample in question. To establish a baseline measurement, the mean amount or level of each of the markers or marker sets assayed in the larger number of samples is determined and this is used as a baseline expression level for the biomarkers or biomarker sets in question. The amount of the marker or marker set determined for the test sample (*e.g.*, absolute level of expression) is then divided by the baseline value obtained for that marker or marker set. This provides a relative amount and aids in identifying abnormal levels of marker protein activity.

Probes based on the sequence of a nucleic acid molecule of the invention can be used to detect transcripts or genomic sequences corresponding to one or more markers of the invention. The probe can comprise a label group attached thereto, *e.g.*, a radioisotope, a fluorescent compound, an enzyme, or an enzyme co-factor. Such probes can be used as part of a diagnostic test kit for identifying cells or tissues which express the protein, such as by measuring levels of a nucleic acid molecule encoding the protein in a sample of cells from a subject, e.g., detecting mRNA levels or determining whether a gene encoding the protein has been mutated or deleted.

In addition to the nucleotide sequences described in the database records described herein, it will be appreciated by those skilled in the art that DNA sequence polymorphisms that lead to changes in the amino acid sequence can exist within a population (*e.g.*, the human population). Such genetic polymorphisms can exist among individuals within a population due to naturally occuring allelic variation. An allele is one of a group of genes which occur alternatively at a

given genetic locus. In addition, it will be appreciated that DNA polymorphisms that affect RNA expression levels can also exist that may affect the overall expression level of that gene (*e.g.,* by affecting regulation or degradation).

Primers or nucleic acid probes comprise a nucleotide sequence complementary to a specific a marker or a mutated region thereof and are of sufficient length to selectively hybridize with a marker gene or nucleic acid associated with a marker gene. Primers and probes can be used to aid in the isolation and sequencing of marker nucleic acids. In one embodiment, the primer or nucleic acid probe, *e.g.,* a substantially purified oligonucleotide, comprises a region having a nucleotide sequence which hybridizes under stringent conditions to about 6, 8, 10, 12, or 15, 20, 25, 30, 40, 50, 60, 75, 100 or more consecutive nucleotides of a marker gene. In another embodiment, the primer or nucleic acid probe is capable of hybridizing to a marker nucleic acid comprising a nucleotide sequence of any sequence set forth in any of SEQ ID NOs:1, 2, 4, 5, or a sequence on chromosome 1p, e.g., from base pair 115247085 to 115259515 and chromosome 12p, e.g., from base pair 25358180 to 25403854, or a complement of any of the foregoing. For example, a primer or nucleic acid probe comprising a nucleotide sequence of at least about 15 consecutive nucleotides, at least about 25 nucleotides or having from about 15 to about 20 nucleotides set forth in any of SEQ ID NOs: 1, 2, 4, 5, or a sequence on chromosome 1p from base pair 115247085 to 115259515 or chromosome 12p, from base pair 25358180 to 25403854, or a complement of any of the foregoing are provided by the invention. Primers or nucleic acid probes having a sequence of more than about 25 nucleotides are also within the scope of the present disclosure. In another embodiment, a primer or nucleic acid probe can have a sequence at least 70%, at least 75%, 80% or 85%, or at least, 90%, 95% or 97% identical to the nucleotide sequence of any sequence set forth in any of SEQ ID NOs: 1, 2, 4, 5, or a sequence on chromosome 1p from base pair 115247085 to 115259515, chromosome 12p from base pair 25358180 to 25403854, or a complement of any of the foregoing. Nucleic acid analogs can be used as binding sites for hybridization. An example of a suitable nucleic acid analogue is peptide nucleic acid (see, *e.g.,* Egholm et al., Nature 363:566 568 (1993); U.S. Pat. No. 5,539,083).

Primers or nucleic acid probes can be selected using an algorithm that takes into account binding energies, base composition, sequence complexity, cross-hybridization binding energies, and secondary structure (see Friend *et al.,* International Patent Publication WO 01/05935, published Jan. 25, 2001; Hughes et al., Nat. Biotech. 19:342-7 (2001). Useful primers or nucleic acid probes of the invention bind sequences which are unique for each transcript, e.g., target mutated regions and can be used in PCR for amplifying, detecting and sequencing only that particular nucleic acid, e.g., transcript or mutated transcript. Examples of some mutations of a marker gene, e.g., NRAS are found in Table 11 in the Examples. Other mutations are described in reference articles cited herein and in public databases described herein. One of skill in the art can design primers and nucleic acid probes for the markers disclosed herein or related markers with similar characteristics, *e.g.,* markers on the chromosome loci, or mutations in different regions of the same marker gene described herein, using the skill in the art, *e.g.*, adjusting the potential for primer or nucleic acid probe binding to standard sequences, mutants or allelic variants by manipulating degeneracy or GC content in the primer or nucleic acid probe. Computer programs that are well known in the art are useful in the design of primers with the required specificity and optimal amplification properties, such as Oligo version 5.0 (National Biosciences, Plymouth, MN). While perfectly complementary nucleic acid probes and primers can be used for detecting the markers described herein and mutants, polymorphisms or alleles thereof, departures from complete complementarity are contemplated where such departures do not prevent the molecule from specifically hybridizing to the target region. For example, an oligonucleotide primer may have a non-complementary fragment at its 5' end, with the remainder of the primer being complementary to the target region. Alternatively, non-complementary nucleotides may be interspersed into the nucleic acid probe or primer as long as the resulting probe or primer is still capable of specifically hybridizing to the target region.

An indication of treatment outcome can be assessed by studying the amount of 1 marker, 2 markers, 3 markers or 4 markers, or more, *e.g.,* 5, 6, 7, 8, 9, 10, 15, 20, or 25 markers, or mutated portions thereof e.g., marker genes which participate in or interact with the RAS pathway e.g., genes which control the cell cycle, e.g., which can be inactivated by somatic mutation in cancer. Markers can be studied in combination with another measure of treatment outcome, *e.g.,* biochemical markers (*e.g.,* M protein, proteinuria) or histology markers (e.g., blast count, number of mitotic figures per unit area).

Statistical methods can assist in the determination of treatment outcome upon measurement of the amount of markers, *e.g.,* measurement of DNA, RNA or protein. The amount of one marker can be measured at multiple timepoints, *e.g.,* before treatment, during treatment, after treatment with an agent, *e.g.,* a proteasome inhibitor. To determine the progression of change in expression of a marker from a baseline, *e.g.,* over time, the expression results can be analyzed by a repeated measures linear regression model (Littell, Miliken, Stroup, Wolfinger, Schabenberger (2006) SAS for Mixed Models, 2nd edition. SAS Institute, Inc., Cary, NC)):

Equation 1

$$Y_{ijk} - Y_{ij0} = Y_{ij0} + treatment_i + day_k + (treatment * day)_{ik} + \varepsilon_{ijk}$$

where $Y_{ijk}$ is the $log_2$ transformed expression (normalized to the housekeeping genes) on the $k^{th}$ day of the $j^{th}$ animal in the $i^{th}$ treatment, $Y_{ij0}$ is the defined baseline $log_2$ transformed expression (normalized to the housekeeping genes) of the $j^{th}$ animal in the $i^{th}$ treatment, $day_k$ is treated as a categorical variable, and $\varepsilon_{ijk}$ is the residual error term. A covariance matrix (*e.g.*, first-order autoregressive, compound symmetry, spatial power law) can be specified to model the repeated measurements on each animal over time. Furthermore, each treatment time point can be compared back to the same time point in the vehicle group to test whether the treatment value was significantly different from vehicle.

A number of other methods can be used to analyze the data. For instance, the relative expression values could be analyzed instead of the cycle number. These values could be examined as either a fold change or as an absolute difference from baseline. Additionally, a repeated-measures analysis of variance (ANOVA) could be used if the variances are equal across all groups and time points. The observed change from baseline at the last (or other) time point could be analyzed using a paired t-test, a Fisher exact test (p-value = $\sum P(X=x)$ from x=1 to the number of situations, e.g., wild type or mutations, tested that show sensitivity, e.g., favorable outcome, or nonresponse to proteasome inhibition) for testing significance of data of small sample sizes, or a Wilcoxon signed rank test if the data is not normally distributed, to compare whether a tumor patient was significantly different from a normal subject.

A difference in amount from one timepoint to the next or from the tumor sample to the normal sample can indicate prognosis of treatment outcome. A baseline level can be determined by measuring expression at 1, 2, 3, 4, or more times prior to treatment, e.g., at time zero, one day, three days, one week and/or two weeks or more before treatment. Alternatively, a baseline level can be determined from a number of subjects, *e.g.,* normal subjects or patients with the same health status or disorder, who do not undergo or have not yet undergone the treatment, as discussed above. Alternatively, one can use expression values deposited with the Gene Expression Omnibus (GEO) program at the National Center for Biotechnology Information (NCBI, Bethesda, MD). For example, datasets of myeloma mRNA expression amounts sampled prior to proteasome inhibition therapy include GEO Accession number GSE9782, also analyzed in Mulligan, et al. (2006) Blood 109:3177-88 and GSE6477, also analyzed by Chng et al. (2007) Cancer Res. 67:292-9. To test the effect of the treatment on the tumor, the expression of the marker can be measured at any time or multiple times after some treatment, *e.g.,* after 1 day, 2 days, 3 days, 5 days, 1 week, 2 weeks, 3 weeks, one 3 week treatment cycle, 4 weeks, one 4 week treatment cycle, 1 month, one 5 week treatment cycle, 2 months, 3 months, 5 cycles and/or 6 or more months of treatment. For example, the amount of a marker can be measured once after some treatment, or at multiple intervals, *e.g.,* 1-week, 2-week, 4-week, one 3-week, 4-week or 5-week cycle, two cycles, 2-month, 3-month, five cycles or longer intervals during treatment. A treatment cycle for bortezomib or dexamethasone can be found in the Examples, in the publications of treatment with the agents, or in the product inserts. A treatment cycle for ixazomib citrate (MLN9708) can be found at the clinical trials website maintained by the U.S. National Institutes of Health, Bethesda, MD. Conversely, to determine onset of progressive disease after stopping the administration of a therapeutic regimen, the amount of the marker can be measured at any time or multiple times after, *e.g.,* 1 day, 2 days, 3 days, 5 days, 1 week, 2 weeks, 3 weeks, 4 weeks, 1 month, 2 months, 3 months and/or 6 or more months after the last treatment. One of skill in the art would determine the timepoint or timepoints to assess the amount of the marker depending on various factors, *e.g.,* the pharmacokinetics of the treatment, the treatment duration, pharmacodynamics of the treatment, age of the patient, the nature of the disorder or mechanism of action of the treatment. A trend in the negative direction or a decrease in the amount relative to baseline or a pre-determined standard of expression of a marker of sensitivity to proteasome inhibition therapy indicates a decrease in response of the tumor to the therapy, e.g., increase in resistance. A trend toward a favorable outcome relative to the baseline or a pre-determined standard of expression of a marker of treatment outcome indicates usefulness of the therapeutic regimen or continued benefit of the therapy.

Any marker, *e.g.,* marker gene or combination of marker, *e.g.,* marker genes of the invention, or mutations thereof as well as any known markers in combination with the markers, *e.g.,* marker genes of the invention, may be used in the compositions, kits, and methods of the present invention. In general, markers are selected for as great as possible difference between the characteristic, e.g., size, sequence, composition, activity or amount of the marker in samples comprising tumor cells and the characteristic, e.g., size, sequence, composition, activity or amount of the same marker in control cells. Although this difference can be as small as the limit of detection of the method for assessing the amount of the marker, in another embodiment, the difference can be at least greater than the standard error of the assessment method. In the case of RNA or protein amount, a difference can be at least 1.5-, 2-, 3-, 4-, 5-, 6-, 7-, 8-, 9-, 10-, 15-, 20-, 25-, 100-, 500-, 1000-fold or greater. "Low" RNA or protein amount can be that expression relative to the overall mean across tumor samples (*e.g.,* hematological tumor, *e.g.,* myeloma) is low. In the case of amount of DNA, e.g., copy number, the amount is 0, 1, 2, 3, 4, 5, 6, or more copies. A deletion causes the copy number to be 0 or 1; an amplification causes the copy number to be greater than 2. The difference can be qualified by a confidence level, e.g., $p < 0.05$, $p < 0.02$, $p < 0.01$ or lower p-value.

Measurement of more than one marker, *e.g.,* a set of 2, 3, 4, 5, 6, 7, 8, 9, 10, 12, 15, 20, or 25 or more markers, e.g., a set of markers comprising an NRAS marker, can provide an expression profile or a trend indicative of treatment outcome. In some embodiments, the marker set comprises no more than 2, 3, 4, 5, 6, 7, 8, 9, 10, 12, 15, 20, or 25

markers. In some embodiments, the marker set includes a plurality of chromosome loci, a plurality of marker genes, or a plurality of markers of one or more marker genes (e.g., nucleic acid and protein, genomic DNA and mRNA, or various combinations of markers described herein). Analysis of treatment outcome through assessing the amount of markers in a set can be accompanied by a statistical method, *e.g.,* a weighted voting analysis which accounts for variables which can affect the contribution of the amount of a marker in the set to the class or trend of treatment outcome, *e.g.,* the signal-to-noise ratio of the measurement or hybridization efficiency for each marker. A marker set, *e.g.,* a set of 2, 3, 4, 5, 6, 7, 8, 9, 10, 12, 15, 20, or 25 or more markers, can comprise a primer, probe or primers to analyze at least one marker DNA or RNA described herein, *e.g.,* a marker on chromosome 1p from base pair 115247085 to 115259515, chromosome 12p from base pair 25358180 to 25403854, NRAS, KRAS, or a complement of any of the foregoing. A marker set, e.g., a set of 2, 3, 4, 5, 6, 7, 8, 9, 10, 12, 15, 20, or 25 or more markers, can comprise a primer, probe or primers to detect at least one or at least two or more markers, or at least 2, 3, 4, 5, 6, 7, 8, 9, 10, 12, 15,20, or 25 or more mutations on the markers e.g., of NRAS and/or KRAS. In another embodiment, a marker set can comprise wild type NRAS nucleic acid or probes or primers comprising wild type versions of mutated regions, or the complement thereof or capable of aiding in the identification of the sequence of mutated regions of NRAS, e.g., codon 12, codon 13 or codon 61 of SEQ ID NO:2. Selected marker sets can be assembled from the markers provided herein or selected from among markers using methods provided herein and analogous methods known in the art. A way to qualify a new marker for use in an assay of the invention is to correlate DNA copy number in a sample comprising tumor cells with differences in expression (e.g., fold-change from baseline) of a marker, *e.g.,* a marker gene. A useful way to judge the relationship is to calculate the coefficient of determination r2, after solving for r, the Pearson product moment correlation coefficient and/or preparing a least squares plot, using standard statistical methods. A correlation can analyze DNA copy number versus the level of expression of marker, *e.g.,* a marker gene. A gene product can be selected as a marker if the result of the correlation (r2, e.g., the linear slope of the data in this analysis), is at least 0.1- 0.2, at least 0.3-0.5, or at least 0.6-0.8 or more. Markers can vary with a positive correlation to response, TTP or survival (i.e., change expression levels in the same manner as copy number, e.g., decrease when copy number is decreased). Markers which vary with a negative correlation to copy number (i.e., change expression levels in the opposite manner as copy number levels, e.g., increase when copy number is decreased) provide inconsistent determination of outcome.

Another way to qualify a new marker for use in the assay would be to assay the expression of large numbers of markers in a number of subjects before and after treatment with a test agent. The expression results allow identification of the markers which show large changes in a given direction after treatment relative to the pre-treatment samples. One can build a repeated-measures linear regression model to identify the genes that show statistically significant changes or differences. To then rank these significant genes, one can calculate the area under the change from *e.g.,* baseline *vs* time curve. This can result in a list of genes that would show the largest statistically significant changes. Then several markers can be combined together in a set by using such methods as principle component analysis, clustering methods (*e.g.*, k-means, hierarchical), multivariate analysis of variance (MANOVA), or linear regression techniques. To use such a gene (or group of genes) as a marker, genes which show 2-, 2.5-, 3-, 3.5-, 4-, 4.5-, 5-, 7-,10- fold, or more differences of expression from baseline would be included in the marker set. An expression profile, *e.g.,* a composite of the expression level differences from baseline or reference of the aggregate marker set would indicate at trend, *e.g.,* if a majority of markers show a particular result, *e.g.,* a significant difference from baseline or reference, e.g., 60%, 70%, 80%, 90%, 95% or more markers; or more markers, *e.g.,* 10% more, 20% more, 30% more, 40% more, show a significant result in one direction than the other direction.

In embodiments when the compositions, kits, and methods of the invention are used for characterizing treatment outcome in a patient, the marker or set of markers of the invention is selected such that a significant result is obtained in at least about 20%, at least about 40%, 60%, or 80%, or in substantially all patients treated with the test agent. The marker or set of markers of the invention can be selected such that a positive predictive value (PPV) of greater than about 10% is obtained for the general population and additional confidence in a marker can be inferred when the PPV is coupled with an assay specificity greater than 80%.

## Therapeutic Agents

The markers and marker sets of the present invention can be used to assess the likelihood of favorable outcome (e.g., sensitivity to a therapeutic agent) in patients, e.g., cancer patients, *e.g.,* patients having a hematological tumors (e.g., myeloma, *e.g.,* multiple myeloma, lymphoma, *e.g.,* mantle cell lymphoma or follicular lymphoma, or leukemia, e.g., acute myeloid leukemia or acute lymphoid leukemia), based on values or changes in at least one characteristic, e.g., composition or amount of a marker or markers of the invention. The markers and marker sets of the present invention assess the likelihood of favorable outcome in cancer patients, *e.g.,* patients having multiple myeloma. Using this prediction, cancer therapies can be evaluated to design a therapy regimen best suitable for a patient either predicted to have a favorable outcome or an unfavorable outcome.

Therapeutic agents for use in the methods of the invention include a class of therapeutic agents known as

proteosome inhibitors. Proteasome inhibitors are described in an earlier section. In particular, the methods can be used to predict patient sensitivity to proteasome inhibitors as described in earlier sections. The agents tested in the present methods can be a single agent or a combination of agents. The methods of the invention include combination of proteasome inhibition therapy with other or additional agents, a "combination agent", e.g., selected from the group consisting of chemotherapeutic agents. For example, the present methods can be used to determine whether a single chemotherapeutic agent, such as a proteasome inhibitor, such as a peptidyl boronic acid (*e.g.*, MLN9708) or a peptidyl epoxy ketone can be used to treat a cancer or whether a one or more agents should be used in combination with the proteasome inhibitor (*e.g.*, MLN9708). Useful combination agents can include agents that have different mechanisms of action, *e.g.,* the use of an anti-mitotic agent or an alkylating agent in combination with a proteasome inhibitor. Proteasome inhibitors are described in an earlier section.

In some embodiments, a proteasome inhibitor is administered in combination with at least one combination agent. In some embodiments, the combination agent is a glucocorticoid agent. The methods of the invention include combination of proteasome inhibition therapy with glucocorticoid inhibition therapy and/or other or additional agents, including chemotherapeutic agents. In some embodiments, a proteasome inhibitor is administered in combination with a chemotherapeutic agent. A "chemotherapeutic agent" is intended to include chemical reagents which inhibit the growth of proliferating cells or tissues wherein the growth of such cells or tissues is undesirable. Chemotherapeutic agents such as anti-metabolic agents, *e.g.,* Ara AC, 5-FU and methotrexate, antimitotic agents, *e.g.,* taxane, vinblastine and vincristine, alkylating agents, *e.g.,* melphanlan, Carmustine (BCNU) and nitrogen mustard, Topoisomerase II inhibitors, *e.g.,* VW-26, topotecan and Bleomycin, strand-breaking agents, *e.g.,* doxorubicin and Mitoxantrone (DHAD), Topoisomerase I inhibitors, *e.g.*, topotecan and irinotecan, tyrosine kinase inhibitors, *e.g.*, sorafenib or erlotinib, angiogenesis inhibitors/immunomodulatory agents, *e.g.,* thalidomide, lenalidomide and pomalidomide, cross-linking agents, *e.g.,* cisplatin, oxaliplatin and carboplatin (CBDCA), radiation and ultraviolet light and are well known in the art (see *e.g.*, Gilman A.G., et al., The Pharmacological Basis of Therapeutics, 8th Ed., Sec 12:1202-1263 (1990)), and are typically used to treat neoplastic diseases. Examples of chemotherapeutic agents generally employed in chemotherapy treatments are listed below in Table 2.

**TABLE 2: Chemotherapeutic Agents**

| CLASS | TYPE OF AGENT | NONPROPRIETARY NAMES (OTHER NAMES) |
|---|---|---|
| Alkylating | Nitrogen Mustards | Mechlorethamine ($HN_2$)<br>Cyclophosphamide<br>Ifosfamide<br>Melphalan (L-sarcolysin)<br>Chlorambucil |
| | Ethylenimines<br>And<br>Methylmelamines | Hexamethylmelamine<br>Thiotepa |
| | Alkyl Sulfonates | Busulfan |
| Alkylating | Nitrosoureas | Carmustine (BCNU)<br>Lomustine (CCNU)<br>Semustine (methyl-CCNU)<br>Streptozocin (streptozotocin) |
| Alkylating | Triazenes | Decarbazine (DTIC; dimethyltriazenoimi-dazolecarboxamide) |
| | Alkylator | cis-diamminedichloroplatinum II (CDDP) |
| Antimetabolites | Folic Acid Analogs | Methotrexate (amethopterin)<br>leucovorin<br>pemetrexed |
| | Pyrimidine<br>Analogs | Fluorouracil ('5-fluorouracil; 5-FU)<br>Floxuridine (fluorode-oxyuridine; FUdR)<br>Cytarabine (cytosine arabinoside)<br>gemcitabine |
| | Purine Analogs and<br>Related | Mercaptopuine (6-mercaptopurine; 6-MP)<br>Thioguanine (6-thioguanine; TG) |

(continued)

| CLASS | TYPE OF AGENT | NONPROPRIETARY NAMES (OTHER NAMES) |
| --- | --- | --- |
| | Inhibitors | Pentostatin (2' - deoxycoformycin) |
| | Microtubule-acting agents | Vinblastin (VLB)<br>Vincristine<br>vinorelbine<br>TAXOL (paclitaxel)<br>Taxotere (docetaxel) |
| Natural Products | Topoisomerase Inhibitors | Etoposide<br>Teniposide<br>Camptothecin<br>Topotecan<br>9-amino-campotothecin CPT-11 |
| | Antibiotics | Dactinomycin (actinomycin D)<br>Adriamycin<br>Daunorubicin (daunomycin; rubindomycin)<br>Doxorubicin<br>Bleomycin<br>Plicamycin (mithramycin)<br>Mitomycin (mitomycin C) |
| | Enzymes | L-Asparaginase |
| | Biological Response Modifiers | Interfon alfa<br>Interleukin 2 |
| Natural Products | Platinum Coordination Complexes | cis-diamminedichloroplatinum II (CDDP)<br>Carboplatin |
| | Anthracendione | Mitoxantrone |
| | Substituted Urea | Hydroxyurea |
| Miscellaneous Agents | Methyl Hydraxzine Derivative | Procarbazine<br>(N-methylhydrazine,(MIH) |
| | Adrenocortical Suppressant | Mitotane (o,p'-DDD)<br>Aminoglutethimide |
| Hormones and Antagonists | Progestins | Hydroxyprogesterone caproate<br>Medroxyprogesterone acetate<br>Megestrol acetate |
| | Estrogens | Diethylstilbestrol<br>Ethinyl estradiol |
| | Antiestrogen | Tamoxifen |
| | Androgens | Testosterone propionate Fluoxymesterone |
| | Antiandrogen | Flutamide |
| | Gonadotropin-releasing Hormone analog | Leuprolide |

The agents tested in the present methods can be a single agent or a combination of agents. For example, the present methods can be used to determine whether a single chemotherapeutic agent, such as a proteasome inhibitor, can be used to treat a cancer or whether a combination of two or more agents can be used in combination with a proteasome inhibitor (*e.g.*, bortezomib or ixazomib citrate). Useful combination agens can include agents that have

different mechanisms of action, *e.g.*, the use of an anti-mitotic agent in combination with an alkylating agent and a proteasome inhibitor.

In some embodiments, a proteasome inhibitor is administered in combination with at least one combination agent. Additional therapeutic agents for use in the methods of the invention comprise a known class of therapeutic agents comprising glucocorticoid steroids. Glucocorticoid therapy generally comprises at least one glucocorticoid agent (*e.g.*, dexamethasone, hydrocortisone, predisolone, prednisone, or triamcinolone). In certain applications of the invention, the second agent used in methods of the invention is a glucocorticoid agent. One example of a glucocorticoid utilized in the treatment of multiple myeloma patients as well as other cancer therapies is dexamethasone. In some embodiments, a combination agent is a glucocorticoid agent selected from the group consisting of dexamethasone, hydrocortisone, predisolone, prednisone, and triamcinolone.

In some embodiments, a proteasome inhibitor can be administered in combination with an inhibitor of the RAS/RAF/MEK pathway. In some embodiments, the combination agent is selected from the group consisting of sorafenib, tipifarnib and selumetinib.

The agents disclosed herein may be administered by any route, including intradermally, subcutaneously, orally, intraarterially or intravenously. In one embodiment, administration will be by the intravenous route. Parenteral administration can be provided in a bolus or by infusion.

The concentration of a disclosed compound in a pharmaceutically acceptable mixture will vary depending on several factors, including the dosage of the compound to be administered, the pharmacokinetic characteristics of the compound(s) employed, and the route of administration. The agent may be administered in a single dose or in repeat doses. Treatments may be administered daily or more frequently depending upon a number of factors, including the overall health of a patient, and the formulation and route of administration of the selected compound(s).

Detection Methods

A general principle of prognostic assays involves preparing a sample or reaction mixture that may contain a marker, and a probe, under appropriate conditions and for a time sufficient to allow the marker and probe to interact and bind, thus forming a complex that can be removed and/or detected in the reaction mixture. These assays can be conducted in a variety of ways.

For example, one method to conduct such an assay would involve anchoring the marker or probe onto a solid phase support, also referred to as a substrate, and detecting target marker/probe complexes anchored on the solid phase at the end of the reaction. In one embodiment of such a method, a sample from a subject, which is to be assayed for presence and/or concentration of marker, can be anchored onto a carrier or solid phase support. In another embodiment, the reverse situation is possible, in which the probe can be anchored to a solid phase and a sample from a subject can be allowed to react as an unanchored component of the assay. One example of such some embodiments includes use of an array or chip which contains a predictive marker or marker set anchored for expression analysis of the sample.

There are many established methods for anchoring assay components to a solid phase. These include, without limitation, marker or probe molecules which are immobilized through conjugation of biotin and streptavidin. Such biotinylated assay components can be prepared from biotin-NHS (*N*-hydroxy-succinimide) using techniques known in the art (*e.g.*, biotinylation kit, Pierce Chemicals, Rockford, IL), and immobilized in the wells of streptavidin-coated 96 well plates (Pierce Chemical). In certain embodiments, the surfaces with immobilized assay components can be prepared in advance and stored.

Other suitable carriers or solid phase supports for such assays include any material capable of binding the class of molecule to which the marker or probe belongs. Well-known supports or carriers include, but are not limited to, glass, polystyrene, nylon, polypropylene, nylon, polyethylene, dextran, amylases, natural and modified celluloses, polyacrylamides, gabbros, and magnetite. One skilled in the art will know many other suitable carriers for binding antibody or antigen, and will be able to adapt such support for use with the present invention. For example, protein isolated from cells can be run on a polyacrylamide gel electrophoresis and immobilized onto a solid phase support such as nitrocellulose. The support can then be washed with suitable buffers followed by treatment with the detectably labeled antibody. The solid phase support can then be washed with the buffer a second time to remove unbound antibody. The amount of bound label on the solid support can then be detected by conventional means.

In order to conduct assays with the above mentioned approaches, the non-immobilized component is added to the solid phase upon which the second component is anchored. After the reaction is complete, uncomplexed components may be removed (*e.g.*, by washing) under conditions such that any complexes formed will remain immobilized upon the solid phase. The detection of marker/probe complexes anchored to the solid phase can be accomplished in a number of methods outlined herein.

In some embodiments, the probe, when it is the unanchored assay component, can be labeled for the purpose of detection and readout of the assay, either directly or indirectly, with detectable labels discussed herein and which are well-known to one skilled in the art. The term "labeled", with regard to the probe (*e.g.*, nucleic acid or antibody), is

intended to encompass direct labeling of the probe by coupling (*i.e.*, physically linking) a detectable substance to the probe, as well as indirect labeling of the probe by reactivity with another reagent that is directly labeled. An example of indirect labeling includes detection of a primary antibody using a fluorescently labeled secondary antibody. It is also possible to directly detect marker/probe complex formation without further manipulation or labeling of either component (marker or probe), for example by utilizing the technique of fluorescence energy transfer (FET, see, for example, Lakowicz et al., U.S. Patent No. 5,631,169; Stavrianopoulos, et al., U.S. Patent No. 4,868,103). A fluorophore label on the first, 'donor' molecule is selected such that, upon excitation with incident light of appropriate wavelength, its emitted fluorescent energy will be absorbed by a fluorescent label on a second 'acceptor' molecule, which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, spatial relationships between the molecules can be assessed. In a situation in which binding occurs between the molecules, the fluorescent emission of the 'acceptor' molecule label in the assay should be maximal. An FET binding event can be conveniently measured through standard fluorometric detection means well known in the art (*e.g.*, using a fluorimeter).

In another embodiment, determination of the ability of a probe to recognize a marker can be accomplished without labeling either assay component (probe or marker) by utilizing a technology such as real-time Biomolecular Interaction Analysis (BIA) (see, *e.g.*, Sjolander, S. and Urbaniczky, C. (1991) Anal. Chem. 63:2338-2345 and Szabo et al. (1995) Curr. Opin. Struct. Biol. 5:699-705). As used herein, "BIA" or "surface plasmon resonance" is a technology for studying biospecific interactions in real time, without labeling any of the interactants (*e.g.*, BIACORE™). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

Alternatively, in another embodiment, analogous diagnostic and prognostic assays can be conducted with marker and probe as solutes in a liquid phase. In such an assay, the complexed marker and probe are separated from uncomplexed components by any of a number of standard techniques, including but not limited to: differential centrifugation, chromatography, electrophoresis and immunoprecipitation. In differential centrifugation, marker/probe complexes may be separated from uncomplexed assay components through a series of centrifugal steps, due to the different sedimentation equilibria of complexes based on their different sizes and densities (see, for example, Rivas, G., and Minton, A.P. (1993) Trends Biochem Sci. 18:284-7). Standard chromatographic techniques also can be utilized to separate complexed molecules from uncomplexed ones. For example, gel filtration chromatography separates molecules based on size, and through the utilization of an appropriate gel filtration resin in a column format, for example, the relatively larger complex may be separated from the relatively smaller uncomplexed components. Similarly, the relatively different charge properties of the marker/probe complex as compared to the uncomplexed components may be exploited to differentiate the complex from uncomplexed components, for example through the utilization of ion-exchange chromatography resins. Such resins and chromatographic techniques are well known to one skilled in the art (see, *e.g.,* Heegaard, N.H. (1998) J. Mol. Recognit. 11:141-8; Hage, D.S., and Tweed, S.A. (1997) J. Chromatogr. B. Biomed. Sci. Appl. 699:499-525). Gel electrophoresis may also be employed to separate complexed assay components from unbound components (see, *e.g.,* Ausubel et al., ed., Current Protocols in Molecular Biology, John Wiley & Sons, New York, 1987-1999). In this technique, protein or nucleic acid complexes are separated based on size or charge, for example. In some embodiments, non-denaturing gel matrix materials and conditions in the absence of reducing agent are used in order to maintain the binding interaction during the electrophoretic process. Appropriate conditions to the particular assay and components thereof will be well known to one skilled in the art.

The isolated mRNA can be used in hybridization or amplification assays that include, but are not limited to, Southern or Northern analyses, polymerase chain reaction and TAQMAN® gene expression assays (Applied Biosystems, Foster City, CA) and probe arrays. One diagnostic method for the detection of mRNA levels involves contacting the isolated mRNA with a nucleic acid molecule (probe) that can hybridize to the mRNA encoded by the gene being detected. Nucleic acids comprising mutations of marker genes can be used as probes or primers. The nucleic acid probes or primers of the invention can be single stranded DNA (*e.g.*, an oligonucleotide), double stranded DNA (*e.g.*, double stranded oligonucleotide) or RNA. Primers of the invention refer to nucleic acids which hybridize to a nucleic acid sequence which is adjacent to the region of interest and is extended or which covers the region of interest. A nucleic acid probe can be, for example, a full-length cDNA, or a portion thereof, such as an oligonucleotide of at least 7, 15, 20, 25, 30, 50, 75, 100, 125, 150, 175, 200, 250 or 500 or more consecutive nucleotides of the marker and sufficient to specifically hybridize under stringent conditions to a mRNA or genomic DNA encoding a marker of the present invention. The exact length of the nucleic acid probe will depend on many factors that are routinely considered and practiced by the skilled artisan. Nucleic acid probes of the invention may be prepared by chemical synthesis using any suitable methodology known in the art, may be produced by recombinant technology, or may be derived from a biological sample, for example, by restriction digestion. Other suitable probes for use in the diagnostic assays of the invention are described

herein. The probe can comprise a label group attached thereto, *e.g.*, a radioisotope, a fluorescent compound, an enzyme, an enzyme co-factor, a hapten, a sequence tag, a protein or an antibody. The nucleic acids can be modified at the base moiety, at the sugar moiety, or at the phosphate backbone. An example of a nucleic acid label is incorporated using SUPER™ Modified Base Technology (Nanogen, Bothell, WA, see U.S. Patent No. 7,045,610). The level of expression can be measured as general nucleic acid levels, *e.g.*, after measuring the amplified DNA levels (e.g. using a DNA intercalating dye, *e.g.,* the SYBR green dye (Qiagen Inc., Valencia, CA) or as specific nucleic acids, *e.g.*, using a probe based design, with the probes labeled. TAQMAN® assay formats can use the probe-based design to increase specificity and signal-to-noise ratio.

Such primers or probes can be used as part of a diagnostic test kit for identifying cells or tissues which express the protein, such as by measuring amounts of a nucleic acid molecule transcribed in a sample of cells from a subject, *e.g.*, detecting transcript, mRNA levels or determining whether a gene encoding the protein has been mutated or deleted. Hybridization of an RNA or a cDNA with the nucleic acid probe can indicate that the marker in question is being expressed. The invention further encompasses detecting nucleic acid molecules that differ, due to degeneracy of the genetic code, from the nucleotide sequence of nucleic acids encoding a marker protein (*e.g.*, protein having the sequence of the SEQ ID NO:3 or 6) and thus encode the same protein. It will be appreciated by those skilled in the art that DNA sequence polymorphisms that lead to changes in the amino acid sequence can exist within a population (*e.g.*, the human population). Such genetic polymorphisms can exist among individuals within a population due to natural allelic variation. An allele is one of a group of genes which occur alternatively at a given genetic locus. Such natural allelic variations can typically result in 1-5% variance in the nucleotide sequence of a given gene. Alternative alleles can be identified by sequencing the gene of interest in a number of different individuals, e.g., normal samples from individuals. This can be readily carried out by using hybridization probes to identify the same genetic locus in a variety of individuals. Detecting any and all such nucleotide variations and resulting amino acid polymorphisms or variations that are the result of natural allelic variation and that do not alter the functional activity are intended to be within the scope of the invention. In addition, it will be appreciated that DNA polymorphisms that affect RNA expression levels can also exist that may affect the overall expression level of that gene (*e.g.*, by affecting regulation or degradation).

As used herein, the term "hybridizes" is intended to describe conditions for hybridization and washing under which nucleotide sequences that are significantly identical or homologous to each other remain hybridized to each other. In some embodiments, the conditions are such that sequences at least about 70%, at least about 80%, at least about 85%, 90% or 95% identical to each other remain hybridized to each other for subsequent amplification and/or detection. Stringent conditions vary according to the length of the involved nucleotide sequence but are known to those skilled in the art and can be found or determined based on teachings in Current Protocols in Molecular Biology, Ausubel et al., eds., John Wiley & Sons, Inc. (1995), sections 2, 4 and 6. Additional stringent conditions and formulas for determining such conditions can be found in Molecular Cloning: A Laboratory Manual, Sambrook et al., Cold Spring Harbor Press, Cold Spring Harbor, NY (1989), chapters 7, 9 and 11. A non-limiting example of stringent hybridization conditions for hybrids that are at least 10 basepairs in length includes hybridization in 4X sodium chloride/sodium citrate (SSC), at about 65-70°C (or hybridization in 4X SSC plus 50% formamide at about 42-50°C) followed by one or more washes in 1X SSC, at about 65-70°C. A non-limiting example of highly stringent hybridization conditions for such hybrids includes hybridization in 1X SSC, at about 65-70°C (or hybridization in 1X SSC plus 50% formamide at about 42-50°C) followed by one or more washes in 0.3X SSC, at about 65-70°C. A non-limiting example of reduced stringency hybridization conditions for such hybrids includes hybridization in 4X SSC, at about 50-60°C (or alternatively hybridization in 6X SSC plus 50% formamide at about 40-45°C) followed by one or more washes in 2X SSC, at about 50-60°C. Ranges intermediate to the above-recited values, *e.g.*, at 65-70°C or at 42-50°C are also intended to be encompassed by the present invention. Another example of stringent hybridization conditions are hybridization in 6X sodium chloride/sodium citrate (SSC) at about 45°C, followed by one or more washes in 0.2X SSC, 0.1% SDS at 50-65°C. A further example of stringent hybridization buffer is hybridization in 1 M NaCl, 50 mM 2-(N-morpholino)ethanesulfonic acid (MES) buffer (pH 6.5), 0.5% sodium sarcosine and 30% formamide. SSPE (1xSSPE is 0.15M NaCl, 10mM $NaH_2PO_4$, and 1.25mM EDTA, pH 7.4) can be substituted for SSC (1xSSC is 0.15M NaCl and 15mM sodium citrate) in the hybridization and wash buffers; washes are performed for 15 minutes each after hybridization is complete The hybridization temperature for hybrids anticipated to be less than 50 base pairs in length should be 5-10°C less than the melting temperature ($T_m$) of the hybrid, where $T_m$ is determined according to the following equations. For hybrids less than 18 base pairs in length, $T_m$(°C) = 2(# of A + T bases) + 4(# of G + C bases). For hybrids between 18 and 49 base pairs in length, $T_m$(°C) = 81.5 + 16.6($\log_{10}$[$Na^+$]) + 0.41(%G+C) - (600/N), where N is the number of bases in the hybrid, and [$Na^+$] is the concentration of sodium ions in the hybridization buffer ([$Na^+$] for 1xSSC = 0.165 M). It will also be recognized by the skilled practitioner that additional reagents may be added to hybridization and/or wash buffers to decrease non-specific hybridization of nucleic acid molecules to membranes, for example, nitrocellulose or nylon membranes, including but not limited to blocking agents (*e.g.*, BSA or salmon or herring sperm carrier DNA), detergents (*e.g.*, SDS), chelating agents (*e.g.*, EDTA), Ficoll, polyvinylpyrrolidone (PVP) and the like. When using nylon membranes, in particular, an additional non-limiting example of stringent hybridization conditions is hybridization in 0.25-0.5M $NaH_2PO_4$, 7% SDS at about 65°C,

followed by one or more washes at 0.02M $NaH_2PO_4$, 1% SDS at 65°C, see *e.g.*, Church and Gilbert (1984) Proc. Natl. Acad. Sci. USA 81:1991-1995, (or alternatively 0.2X SSC, 1% SDS). A primer or nucleic acid probe can be used alone in a detection method, or a primer can be used together with at least one other primer or nucleic acid probe in a detection method. Primers can also be used to amplify at least a portion of a nucleic acid. In some embodiments, a portion of a nucleic acid marker comprising a mutation site is amplified. Nucleic acid probes of the invention refer to nucleic acids which hybridize to the region of interest and which are not further extended. For example, a nucleic acid probe is a nucleic acid which specifically hybridizes to a mutant region of a biomarker, and which by hybridization or absence of hybridization to the DNA of a patient or the type of hybrid formed can be indicative of the presence or identity of the mutation of the biomarker or the amount of marker activity.

In one format, the RNA is immobilized on a solid surface and contacted with a probe, for example by running the isolated RNA on an agarose gel and transferring the RNA from the gel to a membrane, such as nitrocellulose. In an alternative format, the nucleic acid probe(s) are immobilized on a solid surface and the RNA is contacted with the probe(s), for example, in an AFFYMETRIX® gene chip array or a SNP chip (Santa Clara, CA) or customized array using a marker set comprising at least one marker indicative of treatment outcome. A skilled artisan can readily adapt known RNA and DNA detection methods for use in detecting the amount of the markers of the present invention. For example, the high density microarray or branched DNA assay can benefit from a higher concentration of tumor cell in the sample, such as a sample which had been modified to isolate tumor cells as described in earlier sections. In a related embodiment, a mixture of transcribed polynucleotides obtained from the sample is contacted with a substrate having fixed thereto a polynucleotide complementary to or homologous with at least a portion (*e.g.*, at least 7, 10, 5, 20, 25, 30, 40, 50, 100, 500, or more nucleotide residues) of a marker nucleic acid. If polynucleotides complementary to or homologous with the marker are differentially detectable on the substrate (*e.g.*, detectable using different chromophores or fluorophores, or fixed to different selected positions), then the levels of expression of a plurality of markers can be assessed simultaneously using a single substrate (*e.g.*, a "gene chip" microarray of polynucleotides fixed at selected positions). In some embodiments when a method of assessing marker expression is used which involves hybridization of one nucleic acid with another, the hybridization can be performed under stringent hybridization conditions.

An alternative method for determining the amount of RNA corresponding to a marker of the present invention in a sample involves the process of nucleic acid amplification, *e.g.*, by RT-PCR (the experimental embodiment set forth in Mullis, 1987, U.S. Patent No. 4,683,202), ligase chain reaction (Barany, 1991, Proc. Natl. Acad. Sci. USA, 88:189-193), self sustained sequence replication (Guatelli et al., 1990, Proc. Natl. Acad. Sci. USA 87:1874-1878), transcriptional amplification system (Kwoh et al., 1989, Proc. Natl. Acad. Sci. USA 86:1173-1177), Q-Beta Replicase (Lizardi et al., 1988, Bio/Technology 6:1197), rolling circle replication (Lizardi et al., U.S. Patent No. 5,854,033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to about 30 nucleotides in length and flank a region from about 50 to about 200 nucleotides in length. Under appropriate conditions and with appropriate reagents, such primers permit the amplification of a nucleic acid molecule comprising the nucleotide sequence flanked by the primers.

For *in situ* methods, RNA does not need to be isolated from the cells prior to detection. In such methods, a cell or tissue sample is prepared/processed using known histological methods. The sample is then immobilized on a support, typically a glass slide, and then contacted with a probe that can hybridize to RNA that encodes the marker.

In another embodiment of the present invention, a polypeptide corresponding to a marker is detected. In some embodiments, an agent for detecting a polypeptide of the invention is an antibody capable of binding to a polypeptide corresponding to a marker of the invention. In related embodiments, the antibody has a detectable label. Antibodies can be polyclonal, or monoclonal. An intact antibody, or a fragment thereof (*e.g.*, Fab or $F(ab')_2$) can be used.

A variety of formats can be employed to determine whether a sample contains a protein that binds to a given antibody. Examples of such formats include, but are not limited to, immunohistochemistry (IHC), enzyme immunoassay (EIA), radioimmunoassay (RIA), Western blot analysis and enzyme linked immunoabsorbant assay (ELISA). A skilled artisan can readily adapt known protein/antibody detection methods for use in determining whether tumor cells express a marker of the present invention. A skilled artisan also can readily adapt such methods for quantifying the amount of the protein in tumor cells or determining whether tumor cells express more, a normal amount, or less of a protein than non-tumor cells in the sample.

Another method for determining the level of a polypeptide corresponding to a marker is mass spectrometry. For example, intact proteins or peptides, *e.g.*, tryptic peptides can be analyzed from a sample, *e.g.*, a blood sample, a lymph sample or other sample, containing one or more polypeptide markers. The method can further include treating the sample to lower the amounts of abundant proteins, *e.g.*, serum albumin, to increase the sensitivity of the method. For example, liquid chromatography can be used to fractionate the sample so portions of the sample can be analyzed

separately by mass spectrometry. The steps can be performed in separate systems or in a combined liquid chromatography/mass spectrometry system (LC/MS, see for example, Liao, et al. (2004) Arthritis Rheum. 50:3792-3803). The mass spectrometry system also can be in tandem (MS/MS) mode. The charge state distribution of the protein or peptide mixture can be acquired over one or multiple scans and analyzed by statistical methods, e.g. using the retention time and mass-to-charge ratio (m/z) in the LC/MS system, to identify proteins expressed at statistically significant levels differentially in samples from patients responsive or non-responsive to proteasome inhibition therapy. Examples of mass spectrometers which can be used are an ion trap system (ThermoFinnigan, San Jose, CA) or a quadrupole time-of-flight mass spectrometer (Applied Biosystems, Foster City, CA). The method can further include the step of peptide mass fingerprinting, e.g. in a matrix-assisted laser desorption ionization with time-of-flight (MALDI-TOF) mass spectrometry method. The method can further include the step of sequencing one or more of the tryptic peptides. Results of this method can be used to identify proteins from primary sequence databases, *e.g.*, maintained by the National Center for Biotechnology Information, Bethesda, MD, or the Swiss Institute for Bioinformatics, Geneva, Switzerland, and based on mass spectrometry tryptic peptide m/z base peaks.

Electronic Apparatus Readable Arrays

Electronic apparatus, including readable arrays comprising at least one predictive marker of the present invention is also contemplated for use in conjunction with the methods of the invention. As used herein, "electronic apparatus readable media" refers to any suitable medium for storing, holding or containing data or information that can be read and accessed directly by an electronic apparatus. As used herein, the term "electronic apparatus" is intended to include any suitable computing or processing apparatus or other device configured or adapted for storing data or information. Examples of electronic apparatus suitable for use with the present invention and monitoring of the recorded information include stand-alone computing apparatus; networks, including a local area network (LAN), a wide area network (WAN) Internet, Intranet, and Extranet; electronic appliances such as personal digital assistants (PDAs), cellular phone, pager and the like; and local and distributed processing systems. As used herein, "recorded" refers to a process for storing or encoding information on the electronic apparatus readable medium. Those skilled in the art can readily adopt any of the presently known methods for recording information on known media to generate manufactures comprising the markers of the present invention.

For example, microarray systems are well known and used in the art for assessment of samples, whether by assessment gene expression (*e.g.*, DNA detection, RNA detection, protein detection), or metabolite production, for example. Microarrays for use according to the invention include one or more probes of predictive marker(s) of the invention characteristic of response and/or non-response to a therapeutic regimen as described herein. In one embodiment, the microarray comprises one or more probes corresponding to one or more of markers selected from the group consisting of markers whose mutation status indicates response, markers whose mutation status indicates long time-to-progression, and markers whose mutation status indicates long term survivors among patients. A number of different microarray configurations and methods for their production are known to those of skill in the art and are disclosed, for example, in U.S. Pat. Nos: 5,242,974; 5,384,261; 5,405,783; 5,412,087; 5,424,186; 5,429,807; 5,436,327; 5,445,934; 5,556,752; 5,405,783; 5,412,087; 5,424,186; 5,429,807; 5,436,327; 5,472,672; 5,527,681; 5,529,756; 5,545,531; 5,554,501; 5,561,071; 5,571,639; 5,593,839; 5,624,711; 5,700,637; 5,744,305; 5,770,456; 5,770,722; 5,837,832; 5,856,101; 5,874,219; 5,885,837; 5,919,523; 5981185; 6,022,963; 6,077,674; 6,156,501; 6261776; 6346413; 6440677; 6451536; 6576424; 6610482; 5,143,854; 5,288,644; 5,324,633; 5,432,049; 5,470,710; 5,492,806; 5,503,980; 5,510,270; 5,525,464; 5,547,839; 5,580,732; 5,661,028; 5,848,659; and 5,874,219; Shena, et al. (1998), Tibtech 16:301; Duggan et al. (1999) Nat. Genet. 21:10; Bowtell et al. (1999) Nat. Genet. 21:25; Lipshutz et al. (1999) Nature Genet. 21:20-24, 1999; Blanchard, et al. (1996) Biosensors and Bioelectronics, 11:687-90; Maskos, et al., (1993) Nucleic Acids Res. 21:4663-69; Hughes, et al. (2001) Nat. Biotechol. 19:342, 2001. A tissue microarray can be used for protein identification (see Hans et al. (2004)Blood 103:275-282). A phage-epitope microarray can be used to identify one or more proteins in a sample based on whether the protein or proteins induce auto-antibodies in the patient (Bradford et al. (2006) Urol. Oncol. 24:237-242).

A microarray thus comprises one or more probes corresponding to one or more markers identified herein, *e.g.*, those indicative of treatment outcome, e.g., to identify wild type marker genes, normal allelic variants and mutations of marker genes. The microarray can comprise probes corresponding to, for example, at least 2, at least 3, at least 4, at least 5, at least 10, at least 15, at least 20, at least 25, at least 30, at least 35, at least 40, at least 45, at least 50, at least 75, or at least 100, biomarkers and/or mutations thereof indicative of treatment outcome. The microarray can comprise probes corresponding to one or more biomarkers as set forth herein. Still further, the microarray may comprise complete marker sets as set forth herein and which may be selected and compiled according to the methods set forth herein. The microarray can be used to assay expression of one or more predictive markers or predictive marker sets in the array. In one example, the array can be used to assay more than one predictive marker or marker set expression in a sample to ascertain an expression profile of markers in the array. In this manner, up to about 44,000 markers can

be simultaneously assayed for expression. This allows an expression profile to be developed showing a battery of markers specifically expressed in one or more samples. Still further, this allows an expression profile to be developed to assess treatment outcome.

The array is also useful for ascertaining differential expression patterns of one or more markers in normal and abnormal (*e.g.*, sample, e.g., tumor) cells. This provides a battery of markers that could serve as a tool for ease of identification of treatment outcome of patients. Further, the array is useful for ascertaining expression of reference markers for reference expression levels. In another example, the array can be used to monitor the time course of expression of one or more markers in the array.

In addition to such qualitative determination, the invention allows the quantification of marker expression. Thus, predictive markers can be grouped on the basis of marker sets or outcome indications by the amount of the marker in the sample. This is useful, for example, in ascertaining the outcome of the sample by virtue of scoring the amounts according to the methods provided herein.

The array is also useful for ascertaining the effect of the expression of a marker on the expression of other predictive markers in the same cell or in different cells. This provides, for example, a selection of alternate molecular targets for therapeutic intervention if patient is predicted to have an unfavorable outcome.

Reagents and Kits

The invention also encompasses kits for detecting the presence of a polypeptide or nucleic acid corresponding to a marker of the invention in a sample (*e.g.* a bone marrow sample, tumor biopsy or a reference sample). Such kits can be used to assess treatment outcome, *e.g.*, determine if a subject can have a favorable outcome, *e.g.*, after proteasome inhibitor treatment. For example, the kit can comprise a labeled compound or agent capable of detecting a genomic DNA segment, a polypeptide or a transcribed RNA corresponding to a marker of the invention or a mutation of a marker gene in a biological sample and means for determining the amount of the genomic DNA segment, the polypeptide or RNA in the sample. Suitable reagents for binding with a marker protein include antibodies, antibody derivatives, antibody fragments, and the like. Suitable reagents for binding with a marker nucleic acid (*e.g.*, a genomic DNA, an mRNA, a spliced mRNA, a cDNA, or the like) include complementary nucleic acids. The kit can also contain a control or reference sample or a series of control or reference samples which can be assayed and compared to the test sample. For example, the kit may have a positive control sample, e.g., including one or more markers or mutations described herein, or reference markers, *e.g*. housekeeping markers to standardize the assay among samples or timepoints or reference genomes, *e.g.,* form subjects without tumor *e.g.,* to establish diploid copy number baseline or reference expression level of a marker. By way of example, the kit may comprise fluids (*e.g.*, buffer) suitable for annealing complementary nucleic acids or for binding an antibody with a protein with which it specifically binds and one or more sample compartments. The kit of the invention may optionally comprise additional components useful for performing the methods of the invention, *e.g.*, a sample collection vessel, *e.g.,* a tube, and optionally, means for optimizing the amount of marker detected, for example if there may be time or adverse storage and handling conditions between the time of sampling and the time of analysis. For example, the kit can contain means for increasing the number of tumor cells in the sample, as described above, a buffering agent, a preservative, a stabilizing agent or additional reagents for preparation of cellular material or probes for use in the methods provided; and detectable label, alone or conjugated to or incorporated within the provided probe(s). In one exemplary embodiment, a kit comprising a sample collection vessel can comprise *e.g.*, a tube comprising anti-coagulant and/or stabilizer, as described above, or known to those skilled in the art. The kit can further comprise components necessary for detecting the detectable label (*e.g.*, an enzyme or a substrate). For marker sets, the kit can comprise a marker set array or chip for use in detecting the biomarkers. Kits also can include instructions for interpreting the results obtained using the kit. The kit can contain reagents for detecting one or more biomarkers, *e.g.*, 2, 3, 4, 5, or more biomarkers described herein.

In one embodiment, the kit comprises a probe to detect at least one biomarker, e.g., a marker indicative of treatment outcome (*e.g*., upon proteasome inhibitor treatment). In an exemplary embodiment, the kit comprises a nucleic acid probe to detect a marker gene selected from the group consisting of SEQ ID NO: 1, 2, 4, 5, or a sequence on chromosome 1p from base pair 115247085 to 115259515, chromosome 12p from base pair 25358180 to 25403854, or a complement of any of the foregoing or SEQ ID NO: 3 and/or 6. In some embodiments, the kit comprises a probe to detect a marker selected from the group consisting of NRAS and KRAS. In some embodiments, a kit comprises probes to detect a marker set comprising two or more markers from the group consisting of NRAS and KRAS. In another embodiment, a kit comprises a probe to detect NRAS in hematological cancer samples. In related embodiments, the kit comprises a nucleic acid probe comprising or derived from (*e.g.*, a fragment, mutant or variant (*e.g.*, homologous or complementary) thereof) a nucleic acid sequence selected from the group consisting of SEQ ID NOs: 1, 2, 4 and 5. A kit can comprise reagents for identifying the presence of a mutation in codon 12, codon 13 and/or codon 61 of SEQ ID NO:2 or the analogous sequence in SEQ ID NO:1. In some embodiments a kit comprises reagents, e.g., probes, e.g., a nucleic acid probe or a protein probe, or a combination thereof, to detect at least two markers. In some embodiments,

the at least two reagents are nucleic acid reagents. For kits comprising nucleic acid probes, *e.g.*, oligonucleotide-based kits, the kit can comprise, for example: one or more nucleic acid reagents such as an oligonucleotide (labeled or non-labeled) which hybridizes to a nucleic acid sequence corresponding to a marker of the invention, optionally fixed to a substrate; labeled oligonucleotides not bound with a substrate, a pair of PCR primers, useful for amplifying a nucleic acid molecule corresponding to a marker of the invention, molecular beacon probes, a marker set comprising oligonucleotides which hybridize to at least two nucleic acid sequences corresponding to markers of the invention, and the like. The kit can contain an RNA-stabilizing agent.

Alternatively, a kit can comprise reagents for determining whether the glycine at residue 12, the glycine at residue 13 and/or the glutamine at residue 61 of SEQ ID NO:3 is present or is a different amino acid. For kits comprising protein probes, e.g., antibody-based kits, the kit can comprise, for example: (1) a first antibody (*e.g.*, attached to a solid support) which binds to a polypeptide corresponding to a marker of the invention; and, optionally, (2) a second, different antibody which binds to either the polypeptide or the first antibody and is conjugated to a detectable label. The kit can contain a protein stabilizing agent. The kit can contain reagents to reduce the amount of non-specific binding of non-biomarker material from the sample to the probe. Examples of reagents include nonioinic detergents, non-specific protein containing solutions, such as those containing albumin or casein, or other substances known to those skilled in the art.

In some embodiments, the kit can comprise at least one reagent to test tumor subtype. In some embodiments, the kit can comprise at least one reagent to determine whether a hematological tumor has a t(4;14) translocation.

Also described herein is a kit containing an antibody described herein conjugated to a detectable substance, and instructions for use. Also described herein is a diagnostic composition comprising a probe described herein and a pharmaceutically acceptable carrier. In one embodiment, the diagnostic composition contains an antibody described herein, a detectable moiety, and a pharmaceutically acceptable carrier.

Antibodies

An isolated polypeptide corresponding to a predictive marker of the invention, or a fragment or mutant thereof, can be used as an immunogen to generate antibodies using standard techniques for polyclonal and monoclonal antibody preparation. For example, an immunogen typically is used to prepare antibodies by immunizing a suitable (*i.e.*, immunocompetent) subject such as a rabbit, goat, mouse, or other mammal or vertebrate. Also described herein are monoclonal antibodies or antigen binding fragments thereof, which antibodies or fragments specifically bind to a polypeptide comprising an amino acid sequence selected from the group consisting of the amino acid sequences described herein, an amino acid sequence encoded by the cDNA described herein, a fragment of at least 8, 10, 12, 15, 20 or 25 amino acid residues of an amino acid sequence described herein, an amino acid sequence which is at least 95%, 96%, 97%, 98% or 99% identical to an amino acid sequence described herein (wherein the percent identity is determined using the ALIGN program of the GCG software package with a PAM120 weight residue table, a gap length penalty of 12, and a gap penalty of 4) and an amino acid sequence which is encoded by a nucleic acid molecule which hybridizes to a nucleic acid molecule consisting of the nucleic acid molecules described herein, or a complement thereof, under conditions of hybridization of 6X SSC at 45°C and washing in 0.2 X SSC, 0.1% SDS at 65°C. An NRAS fragment for use as an immunogen can comprise amino acid 12, amino acid 13 or amino acid 61 of SEQ ID NO:3. The monoclonal antibodies can be human, humanized, chimeric and/or non-human antibodies. An appropriate immunogenic preparation can contain, for example, recombinantly-expressed or chemically-synthesized polypeptide. The preparation can further include an adjuvant, such as Freund's complete or incomplete adjuvant, or a similar immunostimulatory agent.

Methods for making human antibodies are known in the art. One method for making human antibodies employs the use of transgenic animals, such as a transgenic mouse. These transgenic animals contain a substantial portion of the human antibody producing genome inserted into their own genome and the animal's own endogenous antibody production is rendered deficient in the production of antibodies. Methods for making such transgenic animals are known in the art. Such transgenic animals can be made using XENOMOUSE™ technology or by using a "minilocus" approach. Methods for making XENOMICE™ are described in U.S. Pat. Nos. 6,162,963, 6,150,584, 6,114,598 and 6,075,181. Methods for making transgenic animals using the "minilocus" approach are described in U.S. Pat. Nos. 5,545,807, 5,545,806 and 5,625,825; also see International Publication No. WO93/12227.

Antibodies include immunoglobulin molecules and immunologically active portions of immunoglobulin molecules, *i.e.*, molecules that contain an antigen binding site which specifically binds an antigen, such as a polypeptide described herein, *e.g.*, an epitope of a polypeptide described herein. A molecule which specifically binds to a given polypeptide described herein is a molecule which binds the polypeptide, but does not substantially bind other molecules in a sample, *e.g.*, a biological sample, which naturally contains the polypeptide. For example, antigen-binding fragments, as well as full-length monomeric, dimeric or trimeric polypeptides derived from the above-described antibodies are themselves useful. Useful antibody homologs of this type include (i) a Fab fragment, a monovalent fragment consisting of the VL, VH, CL and CH1 domains; (ii) a $F(ab')_2$ fragment, a bivalent fragment comprising two Fab fragments linked by a disulfide bridge at the hinge region; (iii) a Fd fragment consisting of the VH and CH1 domains; (iv) a Fv fragment

consisting of the VL and VH domains of a single arm of an antibody, (v) a dAb fragment (Ward et al., Nature 341:544-546 (1989)), which consists of a VH domain; (vii) a single domain functional heavy chain antibody, which consists of a VHH domain (known as a nanobody) see *e.g.*, Cortez-Retamozo, et al., Cancer Res. 64: 2853-2857(2004), and references cited therein; and (vii) an isolated complementarity determining region (CDR), e.g., one or more isolated CDRs together with sufficient framework to provide an antigen binding fragment. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see *e.g.*, Bird et al. Science 242:423-426 (1988); and Huston et al. Proc. Natl. Acad. Sci. USA 85:5879-5883 (1988). Such single chain antibodies are also intended to be encompassed within the term "antigen-binding fragment" of an antibody. These antibody fragments are obtained using conventional techniques known to those with skill in the art, and the fragments are screened for utility in the same manner as are intact antibodies. Antibody fragments, such as Fv, $F(ab')_2$ and Fab may be prepared by cleavage of the intact protein, e.g. by protease or chemical cleavage. Described herein are polyclonal and monoclonal antibodies. Synthetic and genetically engineered variants (See U.S. Pat. No. 6,331,415) of any of the foregoing are also contemplated herein. Polyclonal and monoclonal antibodies can be produced by a variety of techniques, including conventional murine monoclonal antibody methodology e.g., the standard somatic cell hybridization technique of Kohler and Milstein, Nature 256: 495 (1975) the human B cell hybridoma technique (see Kozbor et al., 1983, Immunol. Today 4:72), the EBV-hybridoma technique (see Cole et al., pp. 77-96 In Monoclonal Antibodies and Cancer Therapy, Alan R. Liss, Inc., 1985) or trioma techniques. See generally, Harlow, E. and Lane, D. (1988) Antibodies: A Laboratory Manual, Cold Spring Harbor Laboratory Press, Cold Spring Harbor, NY; and Current Protocols in Immunology, Coligan et al. ed., John Wiley & Sons, New York, 1994. For diagnostic applications, the antibodies can be monoclonal antibodies, e.g., generated in mouse, rat, or rabbit. Additionally, for use in *in vivo* applications the antibodies described herein can be human or humanized antibodies. Hybridoma cells producing a monoclonal antibody described herein are detected by screening the hybridoma culture supernatants for antibodies that bind the polypeptide of interest, *e.g.*, using a standard ELISA assay.

If desired, the antibody molecules can be harvested or isolated from the subject (*e.g.*, from the blood or serum of the subject) and further purified by well-known techniques, such as protein A chromatography to obtain the IgG fraction. Alternatively, antibodies specific for a protein or polypeptide described herein can be selected or (*e.g.*, partially purified) or purified by, *e.g.*, affinity chromatography to obtain substantially purified and purified antibody. By a substantially purified antibody composition is meant, in this context, that the antibody sample contains at most only 30% (by dry weight) of contaminating antibodies directed against epitopes other than those of the desired protein or polypeptide described herein, and at most 20%, at most 10%, or at most 5% (by dry weight) of the sample is contaminating antibodies. A purified antibody composition means that at least 99% of the antibodies in the composition are directed against the desired protein or polypeptide described herein.

An antibody directed against a polypeptide corresponding to a marker of the invention (*e.g.*, a monoclonal antibody) can be used to detect the marker (*e.g.*, in a cellular sample) in order to evaluate the level and pattern of expression of the marker. The antibodies can also be used diagnostically to monitor protein levels in tissues or body fluids (*e.g.* in a blood sample) as part of a clinical testing procedure, *e.g.*, to, for example, determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include $^{125}I$, $^{131}I$, $^{35}S$ or $^{3}H$.

Accordingly, described herein are substantially purified antibodies or fragments thereof, and non-human antibodies or fragments thereof, which antibodies or fragments specifically bind to a polypeptide comprising an amino acid sequence encoded by a marker identified herein. The substantially purified antibodies described herein, or fragments thereof, can be human, non-human, chimeric and/or humanized antibodies.

Also described herein are non-human antibodies or fragments thereof, which antibodies or fragments specifically bind to a polypeptide comprising an amino acid sequence which is encoded by a nucleic acid molecule of a predictive marker of the invention. Such non-human antibodies can be goat, mouse, sheep, horse, chicken, rabbit, or rat antibodies. Alternatively, the non-human antibodies described herein can be chimeric and/or humanized antibodies. In addition, the non-human antibodies described herein can be polyclonal antibodies or monoclonal antibodies.

The substantially purified antibodies or fragments thereof may specifically bind to a signal peptide, a secreted sequence, an extracellular domain, a transmembrane or a cytoplasmic domain or cytoplasmic loop of a polypeptide described herein. The substantially purified antibodies or fragments thereof, the non-human antibodies or fragments thereof, and/or the monoclonal antibodies or fragments thereof, described herein specifically bind to a secreted sequence

or an extracellular domain of the amino acid sequences described herein.

Sensitivity Assays

A sample of cancerous cells is obtained from a patient. An expression level is measured in the sample for a marker corresponding to at least one of the markers described herein. A marker set can be utilized comprising markers identified described herein, and put together in a marker set using the methods described herein. Such analysis is used to obtain an expression profile of the tumor in the patient. Evaluation of the expression profile is then used to determine whether the patient is expected to have a favorable outcome and would benefit from treatment, e.g., proteasome inhibition therapy (e.g., treatment with a proteasome inhibitor (e.g., bortezomib or ixazomib citrate) alone, or in combination with additional agents)), or an alternative agent expected to have a similar effect on survival. Evaluation of the expression profile can also be used to determine whether a patient is expected to have an unfavorable outcome and would benefit from a cancer therapy other than proteasome inhibition therapy or would benefit from an altered proteasome inhibition therapy regimen. Evaluation can include use of one marker set prepared using any of the methods provided or other similar scoring methods known in the art (e.g., weighted voting, combination of threshold features (CTF), Cox proportional hazards analysis, principal components scoring, linear predictive score, K-nearest neighbor, etc), e.g., using expression values deposited with the Gene Expression Omnibus (GEO) program at the National Center for Biotechnology Information (NCBI, Bethesda, MD). Still further, evaluation can comprise use of more than one prepared marker set. A proteasome inhibition therapy will be identified as appropriate to treat the cancer when the outcome of the evaluation demonstrates a favorable outcome or a more aggressive therapy regimen will be identified for a patient with an expected unfavorable outcome.

In one aspect, the invention features a method of evaluating a patient, e.g., a patient with cancer, e.g. a hematological cancer (e.g myeloma, *e.g.*, multiple myeloma, lymphoma, *e.g.*, mantle cell lymphoma or follicular lymphoma, or leukemia, e.g., acute myeloid leukemia or acute lymphoid leukemia) for treatment outcome. The method includes providing an evaluation of the expression of the markers in a marker set of markers in the patient, wherein the marker set has the following properties: it includes a plurality of genes, each of which is differentially expressed as between patients with identified outcome and non-afflicted subjects and it contains a sufficient number of differentially expressed markers such that differential amount (e.g., as compared to a level in a non-afflicted reference sample) of each of the markers in the marker set in a subject is predictive of treatment outcome with no more than about 15%, about 10%, about 5%, about 2.5%, or about 1% false positives (wherein false positive means predicting that a patient as responsive or non-responsive when the subject is not); and providing a comparison of the amount of each of the markers in the set from the patient with a reference value, thereby evaluating the patient.

Examining the amount of one or more of the identified markers or marker sets in a tumor sample taken from a patient during the course of proteasome inhibition therapy, it is also possible to determine whether the therapeutic agent is continuing to work or whether the cancer has become non-responsive (refractory) to the treatment protocol. For example, a patient receiving a treatment of bortezomib or ixazomib citrate would have tumor cells removed and monitored for the expression of a marker or marker set. If the profile of the amount of one or more markers identified herein more typifies favorable outcome in the presence of the agent, e.g., the proteasome inhibitor, the treatment would continue. However, if the profile of the amount of one or more markers identified herein more typifies unfavorable outcome in the presence of the agent, then the cancer may have become resistant to therapy, e.g., proteasome inhibition therapy, and another treatment protocol should be initiated to treat the patient.

Importantly, these determinations can be made on a patient-by-patient basis or on an agent-by-agent (or combinations of agents). Thus, one can determine whether or not a particular proteasome inhibition therapy is likely to benefit a particular patient or group/class of patients, or whether a particular treatment should be continued.

Use of Information

In one method, information, e.g., about the mutational status of a patient's tumor, e.g., the patient's marker(s) characteristic, e.g., size, sequence, composition, activity or amount (e.g., the result of evaluating a marker or marker set described herein), or about whether a patient is expected to have a favorable outcome, is provided (e.g., communicated, e.g., electronically communicated) to a third party, e.g., a hospital, clinic, a government entity, reimbursing party or insurance company (e.g., a life insurance company). For example, choice of medical procedure, payment for a medical procedure, payment by a reimbursing party, or cost for a service or insurance can be function of the information. E.g., the third party receives the information, makes a determination based at least in part on the information, and optionally communicates the information or makes a choice of procedure, payment, level of payment, coverage, etc. based on the information. In the method, informative expression level of a marker or a marker set selected from or derived from Table 1 and/or described herein is determined.

In one embodiment, a premium for insurance (e.g., life or medical) is evaluated as a function of information

about one or more marker expression levels, e.g., a marker or marker set, e.g., a level of expression associated with treatment outcome (e.g., the informative amount). For example, premiums can be increased (e.g., by a certain percentage) if the marker genes of a patient or a patient's marker set described herein have different characteristic, e.g., size, sequence, composition, activity or amount between an insured candidate (or a candidate seeking insurance coverage) and a reference value (e.g., a non-afflicted person) or a reference sample, e.g., matched control. Premiums can also be scaled depending on the result of evaluating a marker or marker set described herein. For example, premiums can be assessed to distribute risk, e.g., as a function of marker, e.g., the result of evaluating a marker or marker set described herein. In another example, premiums are assessed as a function of actuarial data that is obtained from patients that have known treatment outcomes.

Information about marker characteristic, e.g., size, sequence, composition, activity or amount, e.g., the result of evaluating a marker or marker set described herein (e.g., the informative characteristic, e.g., amount), can be used, e.g., in an underwriting process for life insurance. The information can be incorporated into a profile about a subject. Other information in the profile can include, for example, date of birth, gender, marital status, banking information, credit information, children, and so forth. An insurance policy can be recommended as a function of the information on marker characteristic, e.g., size, sequence, composition, activity or amount, e.g., the result of evaluating a marker or marker set described herein, along with one or more other items of information in the profile. An insurance premium or risk assessment can also be evaluated as function of the marker or marker set information. In one implementation, points are assigned on the basis of expected treatment outcome.

In one embodiment, information about marker characteristic, e.g., size, sequence, composition, activity or amount, e.g., the result of evaluating a marker or marker set described herein, is analyzed by a function that determines whether to authorize the transfer of funds to pay for a service or treatment provided to a subject (or make another decision referred to herein). For example, the results of analyzing a characteristic, e.g., size, sequence, composition, activity or amount of a marker or marker set described herein may indicate that a subject is expected to have a favorable outcome, suggesting that a treatment course is needed, thereby triggering an result that indicates or causes authorization to pay for a service or treatment provided to a subject. In one example, informative characteristic, e.g., size, sequence, composition, activity or amount of a marker or a marker set selected from or derived from Table 1 and/or described herein is determined and payment is authorized if the informative amount identifies a favorable outcome. For example, an entity, e.g., a hospital, care giver, government entity, or an insurance company or other entity which pays for, or reimburses medical expenses, can use the result of a method described herein to determine whether a party, e.g., a party other than the subject patient, will pay for services (e.g., a particular therapy) or treatment provided to the patient. For example, a first entity, e.g., an insurance company, can use the outcome of a method described herein to determine whether to provide financial payment to, or on behalf of, a patient, e.g., whether to reimburse a third party, e.g., a vendor of goods or services, a hospital, physician, or other care-giver, for a service or treatment provided to a patient. For example, a first entity, e.g., an insurance company, can use the outcome of a method described herein to determine whether to continue, discontinue, enroll an individual in an insurance plan or program, e.g., a health insurance or life insurance plan or program.

In one aspect, the disclosure features a method of providing data. The method includes providing data described herein, e.g., generated by a method described herein, to provide a record, e.g., a record described herein, for determining if a payment will be provided. In some embodiments, the data is provided by computer, compact disc, telephone, facsimile, email, or letter. In some embodiments, the data is provided by a first party to a second party. In some embodiments, the first party is selected from the subject, a healthcare provider, a treating physician, a health maintenance organization (HMO), a hospital, a governmental entity, or an entity which sells or supplies the drug. In some embodiments, the second party is a third party payor, an insurance company, employer, employer sponsored health plan, HMO, or governmental entity. In some embodiments, the first party is selected from the subject, a healthcare provider, a treating physician, an HMO, a hospital, an insurance company, or an entity which sells or supplies the drug and the second party is a governmental entity. In some embodiments, the first party is selected from the subject, a healthcare provider, a treating physician, an HMO, a hospital, an insurance company, or an entity which sells or supplies the drug and the second party is an insurance company.

In another aspect, the disclosure features a record (e.g., computer readable record) which includes a list and value of characteristic, e.g., size, sequence, composition, activity or amount for the marker or marker set for a patient. In some embodiments, the record includes more than one value for each marker.

Screening Assays

Also described herein are methods (also referred to herein as "screening assays") for identifying proteasome inhibitors, *i.e.*, candidate or test compounds or agents (*e.g.*, proteins, peptides, peptidomimetics, peptoids, small molecules or other drugs) which have a inhibitory effect on, for example, NRAS mutant expression or RAS pathway activity, or have a stimulatory or inhibitory effect on, for example, the expression or activity of a NRAS substrate or proteins in

the RAS pathway. Compounds thus identified can be used to modulate the activity of target gene products (e.g., NRAS mutant genes) in a therapeutic protocol, to elaborate the biological function of the target gene product, or to identify compounds that disrupt NRAS mutant interactions. Compounds, e.g., proteasome inhibitors, can be identified that cause the death, apoptosis or senescence of cells, e.g., cells from a hematological tumor, e.g., myeloma, *e.g.*, multiple myeloma, lymphoma, *e.g.*, mantle cell lymphoma or follicular lymphoma, or leukemia, e.g., acute myeloid leukemia or acute lymphoid leukemia, or a cell line, e.g., cells grown from an explant of a tumor in a nonresponsive patient, which have a mutant NRAS gene, or an active RAS pathway.

In other embodiments, the assay can identify compounds which modulate one or more activity of a NRAS mutant, e.g., the ability to bind a nucleotide, e.g., GTP or GDP; the ability to hydrolyze a nucleotide; the ability to bind RASGAP, the ability to bind a phospholipid bilayer, e.g, a cell membrane; the ability to control the cell cycle, the ability of the cell to regulate protein homeostasis; and/or the ability to support tumor cell survival. In some embodiments, there can be a comparison of the activity of the NRAS mutant in the presence of the test agent with the activity in the presence of a proteasome inhibitor, e.g., a peptidyl boronic acid, e.g., bortezomib or ixazomib citrate, to which the NRAS mutant or the cell comprising the NRAS mutant has resistance.

The test compounds described herein can be obtained using any of the numerous approaches in combinatorial library methods known in the art, including: biological libraries; peptoid libraries (libraries of molecules having the functionalities of peptides, but with a novel, non-peptide backbone which are resistant to enzymatic degradation but which nevertheless remain bioactive; see, *e.g.*, Zuckermann et al. (1994) J. Med. Chem. 37:2678-85); spatially addressable parallel solid phase or solution phase libraries; synthetic library methods requiring deconvolution; the 'one-bead one-compound' library method; and synthetic library methods using affinity chromatography selection. The biological library and peptoid library approaches are limited to peptide libraries, while the other four approaches are applicable to peptide, non-peptide oligomer or small molecule libraries of compounds (Lam (1997) Anticancer Drug Des. 12:145). Additional compounds can be synthesized from the guidance provided in the publications disclosing proteasome inhibitors described in an earlier section.

Libraries of compounds can be presented in solution (*e.g.*, Houghten (1992) Biotechniques 13:412-421), or on beads (Lam (1991) Nature 354:82-84), chips (Fodor (1993) Nature 364:555-556), bacteria (Ladner, USP 5,223,409), spores (Ladner USP '409), plasmids (Cull et al. (1992) Proc Natl Acad Sci USA 89:1865-1869) or on phage (Scott and Smith (1990) Science 249:386-390; Devlin (1990) Science 249:404-406; Cwirla et al. (1990) Proc. Natl. Acad. Sci. 87:6378-6382; Felici (1991) J. Mol. Biol. 222:301-310; Ladner *supra.*).

In one embodiment, an assay is a cell-based assay in which a cell which expresses a NRAS mutant protein or biologically active portion thereof is contacted with a test compound, and the ability of the test compound to modulate NRAS mutant activity is determined. Determining the ability of the test compound to modulate NRAS mutant activity can be accomplished by monitoring, for example, the ability to bind a nucleotide, e.g., GTP or GDP; the ability to hydrolyze a nucleotide; the ability to bind RASGAP, the ability to bind a phospholipid bilayer, e.g, a cell membrane, the ability to control the cell cycle, the ability of the cell to regulate protein homeostasis, and/or the ability to support tumor cell survival. The effect of the test compound can be compared to a control cell not exposed to the test compound. In some embodiments, there can be a comparison of the activity of the NRAS mutant in the presence of the test agent with the activity in the presence of a proteasome inhibitor, e.g., a peptidyl boronic acid, e.g., bortezomib or ixazomib citrate, to which the NRAS mutant or the cell comprising the NRAS mutant has resistance. The cell, for example, can be of mammalian origin, *e.g.*, human. In other embodiments, the assay can determine the ability of the test compound to modulate a variant of an enzyme structurally or mechanistically similar to NRAS in a drug resistant cell line *in vitro* or *in vivo*, e.g, in a xenograft tumor model. The compound is identified as modulator of drug resistance or a proteasome inhibitor agent when the cell viability or cell growth is decreased.

The present invention will now be illustrated by the following Examples, which are not intended to be limiting in any way.

EXAMPLES

Example 1

Based on positive findings in multiple myeloma in Phase 1 clinical trials (Orlowski, J Clin Oncol. 2002 Nov 15;20(22):4420-7., Aghajanian, Clin Cancer Res. 2002 Aug;8(8):2505-11,) Phase 2 myeloma studies were conducted in order to allow a more precise estimate of anti-tumor activity of bortezomib in a more homogeneous population of patients. Patient samples and response criteria from patients participating in these studies, as well as the following additional studies described below were sought for use in pharmacogenomic analyses to identify markers associated with patient survival. Bone marrow aspirate samples were collected from 133 patients who participated in phase 2 and phase 3 trials of bortezomib or dexamethasone treatment. The bone marrow aspirates were collected prior to the patient's treatment in the studies. Drug information: Bortezomib is a boronic acid derivative of a leucine phenylalanine dipeptide,

CAS Registry No. 179324-69-7, administered by injection at 1 mg/ml after reconstitution from a lyophilized powder. Dexamethasone is a synthetic adrenocorticosteroid, CAS Registry No. 312-93-6, administered as tablets (DECADRON® Merck & Co., Inc.). The trials are described in the following paragraphs.

**024**: The CREST phase 2 trial (024) of either relapsed or refractory disease (subjects with first relapse, Jagannath et al. (2004) Br. J. Haematol. 127:165-172). In Study -024, complete response (CR) + partial response (PR) rates of 30% and 38% were seen among patients with relapsed multiple myeloma treated with bortezomib 1.0 mg/$m^2$ and 1.3 mg/$m^2$, respectively.
**025**: The SUMMIT phase 2 trial of patients with relapsed and refractory myeloma (subjects with second or greater relapse and refractory to their last prior therapy, Richardson PG, et al. (2003) N. Engl. J. Med. 348:2609-2617). In Study -025, the CR+PR rate to bortezomib alone was 27% (53 of 193 patients), and the overall response rate (CR+PR+minimal response (MR)) to bortezomib alone was 35% (67 of 193 patients).
**039**: The APEX phase 3 trial was a multicenter, open-label, randomized study, comprising 627 enrolled patients with relapsed or refractory multiple myeloma with 1-3 prior therapies, randomly assigned to treatment with bortezomib (315 patients) or high-dose dexamethasone (312 patients) (Richardson et al. (2005) N. Engl. J. Med. 352:2487-2498). Patients who received bortezomib were treated for a maximum of 273 days by the following method: up to eight 3-week treatment cycles followed by up to three 5-week treatment cycles of bortezomib. Within each 3-week treatment cycle, the patient received bortezomib 1.3 mg/$m^2$/dose alone as a bolus intravenous (IV) injection twice weekly for two weeks (on Days 1, 4, 8, and 11) of a 21-day cycle. Within each 5-week treatment cycle, the patient received bortezomib 1.3 mg/$m^2$/dose alone as a bolus IV injection once weekly (on Days 1, 8, 15, and 22) of a 35-day cycle. Patients who received dexamethasone were treated for a maximum of 280 days by the following method: received up to four 5-week treatment cycles, followed by up to five 4-week treatment cycles. Within each 5-week treatment cycle, the patient received dexamethasone 40 mg/day PO, once daily on Days 1 to 4, 9 to 12, and 17 to 20 of a 35-day cycle. Within each 4-week treatment cycle, the patient received dexamethasone 40 mg/day PO once daily on Days 1 to 4 of a 28 day cycle.
**040**: Companion trial to 039 for patients who had more than 3 prior therapies. This bortezomib treatment trial included patients in the dexamethasone group of the -039 trial who experienced confirmed progressive disease (PD). An additional 240 patients not from the -039 study, but who received at least 4 prior therapies also enrolled in this study.

Review boards at all participating institutions approved the studies; all patients provided written informed consent. Additional consent was provided for pharmacogenomics analysis. The studies were conducted in accordance with the Declaration of Helsinki and International Conference on Harmonisation Good Clinical Practice guidelines.

-039 Trial Summary

The following section presents more detailed information on the -039 trial. During the study, disease response was assessed according to the European Group for Blood and Marrow Transplant (EBMT) criteria as presented in Table 3.

**Table 3 Disease Response Criteria[1]**

| Response | Criteria for response |
|---|---|
| Complete response (CR)[2] | Requires all of the following:<br>Disappearance of the original monoclonal protein from the blood and urine on at least two determinations for a minimum of six weeks by immunofixation studies.<br>< 5% plasma cells in the bone marrow[3].<br>No increase in the size or number of lytic bone lesions (development of a compression fracture does not exclude response).<br>Disappearance of soft tissue plasmacytomas for at least six weeks. |
| Partial response (PR) | PR includes patients in whom some, but not all, criteria for CR are fulfilled providing the remaining criteria satisfy the requirements for PR.<br>Requires all of the following:<br>≥50% reduction in the level of serum monoclonal protein for at least two determinations six weeks apart.<br>If present, reduction in 24-hour urinary light chain excretion by either<br>≥90% or to < 200 mg for at least two determinations six weeks apart. |

(continued)

| Response | Criteria for response |
| --- | --- |
| | ≥ 50% reduction in the size of soft tissue plasmacytomas (by clinical or radiographic examination) for at least six weeks. |
| | No increase in size or number of lytic bone lesions (development of compression fracture does not exclude response). |
| Minimal response (MR) | MR includes patients in whom some, but not all, criteria for PR are fulfilled providing the remaining criteria satisfy the requirements for MR. Requires all of the following:<br>≥25% to ≤ 50% reduction in the level of serum monoclonal protein for at least two determinations six weeks apart.<br>If present, a 50 to 89% reduction in 24-hour light chain excretion, which still exceeds 200 mg/24 h, for at least two determinations six weeks apart.<br>25-49% reduction in the size of plasmacytomas (by clinical or radiographic examination (e.g., 2D MRI, CT scan).<br>No increase in size or number of lytic bone lesions (development of compression fracture does not exclude response). |
| No change (NC) | Not meeting the criteria for MR or PD. |
| Progressive disease (PD) (for patients not in CR) | Requires one or more of the following:<br>>25% increase in the level of serum monoclonal paraprotein, which must also be an absolute increase of at least 5 g/L and confirmed on a repeat investigation one to three weeks later[4,5].<br>>25% increase in 24-hour urinary light chain excretion, which must also be an absolute increase of at least 200 mg/24 h and confirmed on a repeat investigation one to three weeks later[4,5].<br>>25% increase in plasma cells in a bone marrow aspirate or on trephine biopsy, which must also be an absolute increase of at least 10%.<br>Definite increase in the size of existing lytic bone lesions or soft tissue plasmacytomas.<br>Development of new bone lesions or soft tissue plasmacytomas (not including compression fracture).<br>Development of hypercalcemia (corrected serum calcium<br>>111.5 mg/dL or 2.8 mmol/L not attributable to any other cause)[4]. |
| Relapse from CR | Requires at least one of the following:<br>Reappearance of serum or urine monoclonal paraprotein on immunofixation or routine electrophoresis to an absolute value of >5g/L for serum and >200 mg/24 hours for urine, and excluding oligoclonal immune reconstitution. Reappearance of monoclonal paraprotein must be confirmed by at least one follow-up.<br>≥5% plasma cells in the bone marrow aspirate or biopsy. Development of new lytic bone lesions or soft tissue plasmacytomas or definite increase in the size of residual bone lesions (not including compression fracture).<br>Development of hypercalcemia (corrected serum calcium >11.5 mg/dL or 2.8 mmol/L not attributable to any other cause). |

1 Based on the EBMT criteria. See, Blade et al. (1998) Br. J. Haematol. 102:1115-23.
2 For proper evaluation of CR, bone marrow should be ≥20% cellular and serum calcium should be within normal limits.
3 A bone marrow collection and evaluation is required to document CR. Repeat collection and evaluation of bone marrow is not required to confirm CR for patients with secretory myeloma who have a sustained absence of monoclonal protein on immunofixation for a minimum of 6 weeks; however, repeat collection and evaluation of bone marrow is required at the Response Confirmation visit for patients with non-secretory myeloma.
4 The need for urgent therapy may require repeating these tests earlier or eliminating a repeat examination.
5 For determination of PD, increase in paraprotein is relative to the nadir.

Patients were evaluable for response if they had received at least one dose of study drug and had measurable disease at baseline (627 total patients: 315 in the bortezomib group and 312 in the dexamethasone group). The evaluation

of confirmed response to treatment with bortezomib or dexamethasone according to the European Group for Blood and Marrow Transplant (EBMT) criteria is provided in Table 4. Response and date of disease progression was determined by computer algorithm that integrated data from a central laboratory and case report forms from each clinical site, according to the Blade criteria (Table 3). The response rate (complete plus partial response(CR + PR)) in the bortezomib group was 38 percent; and in the dexamethasone group was 18 percent (P<0.0001). Complete response was achieved in 20 patients (6 percent) who received bortezomib, and in 2 patients (< 1 percent) who received dexamethasone (P<0.001), with complete response plus near-complete response in 13 and 2 percent (P<0.0001) in patients receiving bortezomib and dexamethasone, respectively. See Richardson *et al., supra.*

**Table 4: Summary of Best Confirmed Response to Treatment[1,2] (Population, N = 627)**

| Best Confirmed Response | bortezomib n (%) (n = 315) | dexamethasone n (%) (n = 312) | Difference (95% CI)[a] | p-value[b] |
|---|---|---|---|---|
| Overall Response Rate (CR+PR) | 121 (38) | 56(18) | 0.20 (0.14, 0.27) | <0.0001 |
| Complete Response | 20 (6) | 2 (<1) | 0.06 (0.03, 0.09) | 0.0001 |
| Partial Response | 101 (32) | 54 (17) | 0.15 (0.08, 0.21) | <0.0001 |
| Near CR: IF+ | 21 (7) | 3 (<1) | 0.06 (0.03, 0.09) | |
| SWOG Remission | 46 (15) | 17 (5) | 0.09 (0.05, 0.14) | |
| Minor Response | 25 (8) | 52 (17) | -0.09 (-0.14, -0.04) | |
| CR+ PR + MR | 146 (46) | 108 (35) | 0.12 (0.04, 0.19) | |
| No Change | 137 (43) | 149 (48) | -0.04 (-0.12, 0.04) | |
| Progressive Disease | 22 (7) | 41 (13) | -0.06 (-0.11,-0.01) | |
| Not Evaluable | 10 (3) | 14 (4) | -0.01 (-0.04, 0.02) | |

1: Response based on computer algorithm using the protocol-specified EBMT criteria.
2: Percents calculated for the statistical output in section 14 are 'rounded' to the nearest integer including percents ≥0.5% but <1% rounding to 1%; these are reported in the in-text tables as <1%.
a Asymptotic confidence interval for the difference in response rates.
b P-value from the Cochran-Mantel-Haenszel chi-square test adjusted for the actual randomization stratification factors.

Disease progression was determined by Blade criteria as described in Table 3 and above. The median time to disease progression in the bortezomib group was 6.2 month (189 days); and the in the dexamethasone group was 3.5 months (106 days) (hazard ratio 0.55, P<0.0001). The date of progression was determined by computer algorithm. P-value from log-rank test adjusted by actual randomization factors. See Richardson *et al., supra.*

Median time to response was 43 days for patients in both groups. Median duration of response was 8 months in the bortezomib group and 5.6 months in the dexamethasone group.

Patients given bortezomib had a superior overall survival. One-year survival was 80% on bortezomib and 66% on dexamethasone (P<0.0030). This represents a 41% decrease in risk of death in the bortezomib group during the first year after enrollment. The hazard ratio for overall survival was 0.57 (P<0.0013), favoring bortezomib. The analysis of overall survival includes data from 147 patients (44 percent) in the dexamethasone group who had disease progression and subsequently crossed over to receive bortezomib in a companion study.

Quality of Life assessment can be analyzed to determine if response to therapy was accompanied by measurable improvement in quality of life. Analysis is performed on summary scores as well as individual items, with specific analytical methods outlined in a formal statistical analysis plan developed prior to database lock.

The overall response rate to bortezomib in this set of patients was 42.3% (CR+PR rate of 32%). The overall response rate to dexamethasone was 39.7% (CR+PR rate of 22.2%). For the survival studies, some patients were followed for at least 30 months. For example, the patients in the -039 study were followed for a median of 22 months.

A. Pharmacogenomic Sample Handling

Upon collection of patient bone marrow aspirate, the myeloma cells were enriched via rapid negative selection. The enrichment procedure employs a cocktail of cell-type specific antibodies coupled with an antibody that binds red blood cells RosetteSep (Stem Cell Technologies). The antibody cocktail has antibodies with the following specificity: CD14 (monocytes), CD2 (T and NK cells), CD33 (myeloid progenitors and monocytes), CD41 (platelets and megakaryocytes), CD45RA (naïve B and T cells) and CD66b (granulocytes). The antibodies cross-linked the non-myeloma cell

types to the red blood cells in the samples. The bound cell types were removed using a modified ficoll density gradient. Myeloma cells were then collected and frozen. In the international studies, the first two samples from each site were collected and subjected to RNA isolation so that feedback on quantity and quality could be provided; ultimately Phase 2 and 3 trials provided a similar percentage of informative samples. Control bone marrow plasma cell samples were obtained from normal donors (AllCells, Berkeley CA).

Total RNA was isolated using a QIAGEN® Group RNEASY® isolation kit (Valencia, CA) and quantified by spectrophotometry.

DNA was isolated from the flow through fraction of the column used in the RNA isolation method.

B. Analysis of Genomic Alterations

Flow through from the RNEASY® column was clarified by centrifugation, then concentrated about 10-fold with centrifugal ultrafilters (MICROCON® centrifugal filter device, YM-30 membrane (30 kDa limit), Millipore Corp. Billerica, MA). Impurities were removed using the Qiagen QIAMP® DNA Micro Kit. DNA from the sample was amplified using the Qiagen REPLI-G® WGA kit. Mutation status of DNA from 133 bone marrow tumor biopsies was determined by testing a panel of cancer genes and known tumor suppressors using the Sequenom (San Diego, CA) mass spectrometry genotype analysis system.

Because the studies did not collect paired tumor and normal tissues, the data were not derived by sequencing paired tumor and normal tissues to identify somatic mutations that may be cancer-related; these trials did not collect germline DNA samples from patients. Rather, the platform interrogated tumor DNA only for specific sequences that were previously implicated in cancer (i.e. 'hotspot' mutations), relying largely on the prior knowledge that these are highly relevant in human cancer. The panel of mutations evaluated consisted of the ONCOCARTA™ version 1.0 and custom assays designed in collaboration with Sequenom and Millennium to expand the list of mutations surveyed to 514 known mutations in 41 oncogenes and tumor suppressor genes. Table 5 lists genes with mutations included in the panel and the number in parentheses () indicates the approximate number of mutations targeted in assays of the genes.

Table 5. Genes with mutant regions included in ONCOCARTA™ panel.

| | | | | |
|---|---|---|---|---|
| ABL1 (16) | EGFR (74) | GNAQ (1) | MLH1 (1) | RB1 (11) |
| AKT1 (9) | ERBB2 (8) | HRAS (6) | MYC (6) | RET (20) |
| AKT2 (2) | ERBB3 (1) | JAK2 (1) | NRAS (10) | SOS1 (3) |
| APC (12) | FBX4 (6) | JAK3 (3) | PDGFRA (27) | SRC (1) |
| BRAF (44) | FBXW7 (4) | KIT (69) | PIK3CA (39) | STK11 (11) |
| CDK (2) | FGFR1 (2) | KRAS (16) | PTEN (12) | TP53 (15) |
| CDKN2A (7) | FGFR2 (2) | MAP2K1 (5) | PTPN11 (1) | VHL (7) |
| CSF1R (4) | FGFR3 (6) | MAP2K2 (5) | | |
| CTNNB1 (27) | FLT3 (7) | MET (11) | | |

The custom assays were designed using TYPEPLEX® chemistry with single-base extension which determines the expected mass weight of the extend products to ensure separation between all potential peaks found within a multiplexed reaction. 15 nl of of amplified and extended product is spotted on a 384 SpectroCHIP II using a Nanodispenser. A 3-point calibrant is added to every chip to ensure proper performance of the Sequenom Maldi-tof compact mass spectrometer. The SpectroCHIP II is placed in the Sequenom MALDI-TOF compact mass spectrometer. The mass spectrometer is set to fire a maximum of 9 acquisitions for each spot on the 384 well spectroCHIP. TypePLEX Gold kit SpectroCHIP II is used following manufacturers recommended protocols.

Analysis is performed using Sequenom analysis software, MassARRAY® Typer Analyzer v4 with a mutation call filter of ≥ 8%, Signal:Noise > 5 (background), followed by a manual inspections for false positives due to Impurities (ie Salts). Default threshold for positive mutant call is a minimal 10% mutant allele frequency, though signal as low as 3% may be detected. A 10% threshold works well for cell line or xenograft samples, where nearly 100% of the cells are tumor cells. However, clinical biopsy samples often have a significant percent of non-tumor cells; in some cases, only 10-12% of the cells are tumor cells. Therefore, in order to analyze mutations in the clinical samples, the threshold was lowered to 8%. In house evaluation of sensitivity suggests 8% threshold may be employed with low false positive rates. The false positives led to additional manual quality control (QC) of the data to identify mutations, such as distinguishing the signal from the noise and discarding peaks which were not centered at the molecular weight location on the chromatogram expected for a particular base. After automated and manual rounds of QC, we tested for mutational associations with response to bortezomib or dexamethasone using Fisher's Exact test. For each of the previously mentioned 41 oncogenes (Table 5) we tested for over and/or under-representation of response groups within subsets of patients who were positive for mutations of a given gene. Mutant groups with significant enrichment for either responders or non-responders were further analyzed to determine if the presence of a mutated oncogene was associated with time to tumor progression (TTP) or overall survival (OS) using both a log ratio test and a Cox proportional hazard test. RAS mutations were further investigated for effect on TTP and OS. Kaplan-Meier curves describing the results of one of these analyses are shown in Figure 1. Results for each subgroup were compared using log rank and Cox-proportional hazards method. Table 6 summarizes the genes with mutations which were detected in the screen.

Table 6 : Protein coding mutations detected from Sequenom screen of 133 MM tumor samples

| Gene | Observed Mutants | Percentage of Mutants (95% CI) |
|---|---|---|
| RAS/RAF | 61 | 45.9(37 -54.3) |
| RAS | 58 | 43.6(35 -52.0) |
| KRAS | 32 | 24.1(17 -31.3) |
| NRAS | 26 | 19.5(13 -26.3) |
| PIK3CA*^ | 8 | 6.0(2 -10.1) |
| TP53 | 3 | 2.3(0 - 4.8) |
| BRAF | 3 | 2.3(0 - 4.8) |
| MET* | 3 | 2.3(0 - 4.8) |
| PDGFRA* | 3 | 2.3(0 - 4.8) |
| JAK3* | 2 | 1.5(0 - 3.6) |
| EGFR | 1 | 0.8(0 - 2.2) |
| APC* | 1 | 0.8(0 - 2.2) |
| CDKN2A* | 1 | 0.8(0 - 2.2) |
| CTNNB1* | 1 | 0.8(0 - 2.2) |
| FGFR3* | 1 | 0.8(0 - 2.2) |
| KIT* | 1 | 0.8(0 - 2.2) |
| PTEN* | 1 | 0.8(0 - 2.2) |
| STK11* | 1 | 0.8(0 - 2.2) |
| ^ The probe which identifies this mutant recognizes a pseudogene | | |

These results were compared to Chapman et al. supra, who applied either whole genome or whole exome sequencing to 38 myeloma cases. They reported significantly higher than expected frequencies of protein-altering mutations in KRAS, NRAS, BRAF and TP53 relative to other locations of the genome. Slight differences in the frequencies (compare Tables 2 and 3) of other mutations could be due to the limited number of cases in these datasets and/or general heterogeneity of MM cancer genomes. An Asterisk (*) denotes 11 mutations were detected in this screen that were not reported in Chapman et al. (Table 6) and are absent and/or rarely seen in literature describing genetic profiles of MM tumors. One mutation of the FGFR3 gene was detected in this screen. This is consistent with the role of FGFR3 in multiple myeloma which is often translocated (t 4;14;p16) and exhibits rare protein coding mutations (Soverini S et al. (2002) Haematologica. 87:1036-1040.) The additional 22 genes assayed in this screen (ABL1, AKT1, AKT2, CDK, CSF1R, ERBB2, FBX4, FBXW7, FGFR1, FLT3, FLT4, FLT5, GNAQ, HRAS, MAP2K1, MAP2K2, MLH1, MYC, RET, SOS1, SRC, VHL), consistent with the Chapman et al. screen, no mutations were observed in this series of myeloma cases. At least 48 samples did not have mutations tested in the panel.

C. Analysis of RAS mutations

The most common mutations observed were in KRAS (n=32 [24.1%], 95% CI:17.0-31.3) and NRAS (n=26

[19.5%], 95% CI:12.8-26.3). Mutations in BRAF were detected in three patient samples (2.3%, 95% CI: 0-4.8). For all three genes, the mutation rate in this patient population was similar to that reported by Chapman et al. *supra* (26.3%, 23.7% and 4% for KRAS, NRAS, and BRAF, respectively). Fisher exact test results correlating response with NRAS and KRAS status in samples from patients treated with bortezomib (N=64) and dexamethasone (N=31) are depicted in 2-by-2 frequency Tables 7-10.

Table 7. Fisher 2-by-2 table comparing NRAS status with response in samples from patients treated with bortezomib (p-value = $1.16 \times 10^{-3}$)

| | Response | Non-response | |
|---|---|---|---|
| NRAS mutant | 1 | 14 | 15 |
| NRAS wild type | 31 | 28 | 59 |
| | 32 | 42 | totals |

Table 8. Fisher 2-by-2 table comparing KRAS status with response in samples from patients treated with bortezomib (p-value = 0.41)

| | Response | Non-response | |
|---|---|---|---|
| KRAS mutant | 9 | 8 | 17 |
| KRAS wild type | 23 | 34 | 57 |
| | 32 | 42 | totals |

Table 9. Fisher 2-by-2 table comparing NRAS status with response in samples from patients treated with dexamethasone (p-value = 0.64)

| | Response | Non-response | |
|---|---|---|---|
| NRAS mutant | 3 | 3 | 6 |
| NRAS wild type | 8 | 17 | 25 |
| | 11 | 20 | totals |

Table 10. Fisher 2-by-2 table comparing KRAS status with response in samples from patients treated with dexamethasone (p-value = 1)

| | Response | Non-response | |
|---|---|---|---|
| KRAS mutant | 3 | 6 | 9 |
| KRAS wild type | 8 | 14 | 22 |
| | 11 | 20 | totals |

As can be seen in Tables 7-10, wild type NRAS in pre-treatment samples is significantly associated with response to bortezomib, while mutated NRAS in pre-treatment samples is significantly associated with non-response to bortezomib. KRAS status in pre-treatment samples did not correlate with response to bortezomib in myeloma patients. Neither NRAS nor KRAS status in pre-treatment samples correlated with response to dexamethasone in myeloma patients. Table 11 lists NRAS mutations and response categories identified for some samples from bortezomib- and dexamethasone (Dex)-treated patients. (PD= progressive disease, IE= in-evaluable, NC=no change, PR=partial response, MR=minimal response.)

Table 11. NRAS mutations and response

| treatment | sex | race | AgeAtRandomize | pgxResponse | Mutation | Codon |
|---|---|---|---|---|---|---|
| bortezomib | Male | White | 74 | PD | Q61R | Q61 |

(continued)

| treatment | sex | race | AgeAtRandomize | pgxResponse | Mutation | Codon |
|---|---|---|---|---|---|---|
| bortezomib | Male | White | 61 | PD | Q61R | Q61 |
| bortezomib | MALE | WHITE | 64 | IE | Q61R | Q61 |
| bortezomib | Male | White | 48 | NC | Q61R | Q61 |
| bortezomib | MALE | WHITE | 58 | NC | Q61R | Q61 |
| bortezomib | Male | White | 54 | NC | Q61R | Q61 |
| bortezomib | Male | White | 72 | NC | Q61R | Q61 |
| bortezomib | MALE | WHITE | 67 | N/A | Q61R | Q61 |
| bortezomib | MALE | WHITE | 54 | NC | Q61R | Q61 |
| bortezomib | MALE | WHITE | 48 | PD | Q61L | Q61 |
| bortezomib | Female | White | 56 | NC | Q61L | Q61 |
| bortezomib | Male | White | 56 | NC | Q61K | Q61 |
| bortezomib | MALE | WHITE | 63 | N/A | Q61K | Q61 |
| bortezomib | Male | White | 52 | IE | Q61K | Q61 |
| bortezomib | Female | Asia n/Pacific Islander | 48 | PD | Q61K | Q61 |
| bortezomib | Male | White | 59 | IE | Q61H | Q61 |
| bortezomib | Female | Black | 57 | PR | Q61H | Q61 |
| bortezomib | Female | White | 64 | NC | G13R | G13 |
| bortezomib | Male | White | 55 | NC | G13R | G13 |
| bortezomib | FEMALE | WHITE | 58 | NC | G13D | G13 |
| Dex | Female | White | 54 | PD | Q61R | Q61 |
| Dex | Male | White | 65 | PR | Q61R | Q61 |
| Dex | Male | White | 66 | PD | Q61R | Q61 |
| Dex | Female | White | 69 | PR | Q61K/R | Q61 |
| Dex | Male | White | 72 | MR | Q61K | Q61 |
| Dex | | | | NR | G12C | G12 |

Figure 1 depicts a Kaplan-Meier plot comparing time to disease progression (progression-free survival) to pre-treatment RAS status in 95 bortezomib-treated patients. In a pairwise log rank tests, the comparisons of NRAS mutant (N=20) to RAS wild type (N=56) and to KRAS mutant (N=19) were significant (p-values of $1.9 \times 10^{-4}$ and $1.6 \times 10^{-3}$, respectively), but the comparison of KRAS mutant to RAS wild type was not significant (p-value 0.695). Cox proportional hazards modeling of time-to-progression with RAS status gave NRAS mutation a 3.9 hazard ratio (p-value $3.5 \times 10^{-4}$), but KRAS mutation a 0.828 hazard ratio (p-value 0.69). Patients whose pre-treatment bone marrow tumor samples had NRAS mutations had significantly lower time-to-progression after bortezomib treatment.

In contrast with the shortened TTP of patients whose myeloma tumors were NRAS mutant, analysis of these 95 patients for overall survival did not identify any significant differences when comparing RAS mutant to RAS wild type (p-values of pairwise log rank tests from 0.204 for NRAS mutant and 0.497 for KRAS mutant, Cox proportional hazard ratios of 0.57 for NRAS mutant (p-value 0.18) and 1.25 for KRAS mutant (p-value 0.51)). There was a significant trend in the pairwise log rank comparison of NRAS mutant to KRAS mutant (p-value 0.074). Patients having pre-treatment NRAS mutant had significantly longer survival upon treatment with other therapies after the bortezomib treatment. One possibility is that subsequent therapies were notably more effective in *NRAS*-mutant patients, such that their OS did not lag relative to the *NRAS* wild-type patients.

A potential confounding variable for the observed association between NRAS mutation and reduced response to bortezomib is over-representation of KRAS in multiple myeloma (MM) subtypes known to be associated with bortezomib

response. It has been shown that bortezomib alone or in combination at least partially overcomes the poor prognosis associated with the t(4;14) translocation in MM (Avet-Loiseau et al (2010) J. Clin. Oncol. 28:4630-4634; Chang et al. (2011) 35:95-98). One study (Walker et al. (2012) Blood 120:1077-1086) showed that KRAS mutants were more frequent than NRAS mutants in t(4; 14) MM samples. The responses in KRAS-mutant patients were tested for a relationship to t(4;14) sensitivity to bortezomib. Table 12 details the t(4;14) and NRAS status of the current dataset for patients both with tumor subtype data (Mulligan et al. (2007) Blood 109:3177-3188) and who were response-evaluable (n = 40).

Table 12. t(4; 14) and NRAS status of patients who had both tumor subtype data and were response-evaluable (n = 40)

| **A.** | Response | Non-Response |
|---|---|---|
| t(4;14) | 6 | 2 |
| Other | 11 | 21 |
| p-value (Fisher's exact test) | 0.05322 | |
| **B.** | Response | Non-Response |
| NRAS mutant | 1 | 10 |
| NRAS wild type | 16 | 13 |
| p-value (Fisher's exact test) | 0.01186 | |
| **C.** | Response | Non-Response |
| NRAS mutant and not t(4;14) | 0 | 10 |
| NRAS wild type and not t(4;14) | 11 | 11 |
| p-value (Fisher's exact test) | 0.006023 | |

Table 12A. shows that the subset of patients with t(4;14) translocations exhibited a higher response rate than other subsets in aggregate, consistent with prior reports. However, this trend was not significant. In contrast, Table 12B showed that the NRAS mutant patients within this same cohort were significantly less likely to respond to bortezomib (*P* = .01186). Because the response trends associated with the genetic alterations are in opposite directions and given that the number of patients who are both NRAS mutant and have a t(4;14) translocation in the sample is small, the t(4;14) positive patient samples were removed to test the persistence of the association between lack of response and NRAS mutant samples (N=32). As further evidence that the NRAS mutation association with bortezomib is not just an artifact of minimal co-occurrence of t(4;14) translocations and NRAS mutations, Table 12C shows that this lack of response endures after removal of all the t(4;14) samples from this group, and actually appears to be more significant in this subset.

<u>Example 3. Alternative nucleic acid sequencing methods</u>

<u>SANGER Sequencing methodology.</u> PCR amplifications are conducted using optimized cycling conditions per gene-exon. Primer extension sequencing is performed using Applied Biosystems BigDye version 3.1. The reactions are then run on Applied Biosystem's 3730x1 DNA Analyzer. Sequencing base calls are done using KBTM Basecaller (Applied Biosystems). Somatic Mutation calls are determined by Mutation Surveyor (SoftGenetics) and confirmed manually by aligning sequencing data with the corresponding reference sequence using Seqman (DNASTAR).

<u>NEXT GENERATION SEQUENCING (NGS) methodology.</u> Targeted NGS using the Illumina platform (Illumina, Inc. San Diego, CA) is used to confirm and identify low frequency mutations in a marker. Primer pairs are designed to amplify coding exons. PCR products are quantified using a PicoGreen assay and combined in equal molar ratios for each sample. The purified products are end-repaired and concatenated by ligation. The concatenated products are used for Hi-Seq 2000 library preparation. The concatenated PCR products are sheared and used to make barcoded Hi-Seq 2000 libraries consisting of 12 bar-coded samples per multiplexed pool. The pooled Hi-Seq 2000 libraries undergo clonal amplification by cluster generation on eight lanes of a Hi-Seq 2000 flow cell and are sequenced using 1x100 single-end sequencing on a Hi-Seq 2000. Matching of primary sequencing reads to the human genome build Hg18, as well as SNP analysis are performed using Illumina's CASAVA software version 1.7.1.

General Procedures

Quantitative RT-PCR

cDNA synthesis and quantitative RT-PCR is performed using ABI Gene Expression Assays, reagents, and ABI PRISM® 7900HT Sequence Detection Systems (Applied Biosystems, Foster City, CA) using the following cycle conditions: hold at 50°C for 2 minutes for AmpErase UNG activation, then 95.0°C for 10 minutes to activate DNA polymerase then run 40 two-part cycles of 95.0°C for 15 seconds and 60.0°C for 1 minute. The dCt is calculated by using the average Ct of control genes B2M (Hs99999907_m1) and RPLPO (Hs99999902_m1). Relative mRNA expression quantification is derived using the Comparative Ct Method (Applied Biosystems). mRNA expression fold change values are generated from a normal sample and corresponding tumor sample.

Analysis of Myeloma Gene Expression on an Array

RNA is converted to biotinylated cRNA by a standard T7 based amplification protocol (AFFYMETRIX® Inc., Santa Clara, CA). A small number of samples with ≥0.5 - 2.0 μg are also labeled and subsequently hybridized if 6 μg of cRNA is produced. For the automated T7 amplification procedure, the cDNA and the biotin labeled cRNA are purified using AMPURE® PCR Purification System, following the manufacturer's protocol (AGENCOURT® Bioscience Corporation, Beverly, MA). The cRNA yield is assessed by spectrophotometry and 10 μg of cRNA is fragmented and further processed for triplicate hybridization on the AFFYMETRIX® Human Genome HG-U133A and HG-U133B GENECHIP® arrays. In cases where cRNA yield ranged between 6 μg to 10 μg, the entire cRNA sample is fragmented.

cRNA for each sample is hybridized to the U133A/B arrays in triplicate; operators, chip lots, clinical sites and scanners (GENECHIP® Scanner 3000) are controlled throughout. Background subtraction, smoothing adjustment, noise corrections, and signal calculations are performed with ASYMETRIX® MAS5.0. Quality control metrics include: percent present call (>25) scale factor (< 11), β-actin 3':5' ratio (<15) and background (<120). Samples that fall outside these metrics are excluded from subsequent analysis.

The myeloma purity score examines expression of genes known in the literature to be expressed highly in myeloma cells (and their normal plasma precursor cells), to expression of genes known to be expressed highly in erythroid cells, neutrophils and T cells - see list of 14 markers below). The myeloma score= expression of myeloma markers (#1-4 below) / erythroid (#5-7) + neutrophil (#8-11) + T cell (#12-14):

1. 205692_s_at CD38 CD38 antigen (p45) myeloma/plasma cell
2. 201286_at SDC1 syndecan-1 myeloma/plasma cell
3. 201891_s_at B2M beta-2 microglobulin myeloma/plasma cell
4. 211528_x_at B2M beta-2 microglobulin myeloma/plasma cell
5. 37986_at EpoR erythropoetin receptor erythroid cell
6. 209962_at EpoR erythropoetin receptor erythroid cell
7. 205838_at GYPA glycophorinA erythroid cell
8. 203948_s_at MPO myeloperoxidase neutrophil
9. 203591_s_at CSFR3colony stimulating factor 3receptor (granulocyte) neutrophil
10. 204039_at CEBPACCAAT/enhancer bindingprotein (C/EBP), alpha neutrophil
11. 214523_at CEBPECCAAT/enhancer bindingprotein (C/EBP), epsilon neutrophil
12. 209603_at GATA3 GATA binding protein 3 T lymphocyte
13. 209604_s_at GATA4 GATA binding protein 4 T lymphocyte
14.205456_at CD3ECD3E antigen, epsilon polypeptide T lymphocyte

Samples with a myeloma purity score less than 10 are excluded from further analysis.

## Claims

1. A method for identifying a cancer patient as a candidate for treatment with bortezomib, wherein the patient has a hematological tumor, comprising:

   a) identifying the sequence of at least one marker associated with at least one marker gene in a patient sample comprising hematological tumor cells, wherein one marker gene is neuroblastoma RAS viral (v-ras) oncogene homolog (NRAS); and

b) identifying the patient as a candidate for treatment with bortezomib if the sequence indicates that the tumor cells comprise wild type NRAS.

**2.** The method of claim 1, wherein the at least one marker is selected from the group consisting of nucleic acid and protein corresponding to the at least one marker gene.

**3.** The method of claim 1 or 2, wherein the hematological tumor is selected from the group consisting of myeloma, lymphoma and leukemia.

**4.** The method of any one of the preceding claims, wherein the sample comprises cells selected from the group consisting of myeloma tumor cells and lymphoma tumor cells, and optionally, further comprising enriching the sample for tumor cells.

**5.** The method of any one of the preceding claims, wherein the at least one marker is at least two markers.

**6.** The method of claim 5, wherein the at least two markers comprises at least a marker associated with a NRAS marker gene and a tumor subtype marker.

**7.** The method of claim 6, wherein the tumor subtype marker is a t(4;14) translocation.

**8.** The method of claim 4, wherein the sample is blood.

**9.** The method of any one of the preceding claims, further comprising determining whether to continue bortezomib treatment of the hematological tumor in the patient by a method comprising:

a) identifying on a second biological sample comprising tumor cells from the patient identified for treatment with bortezomib the sequence of the at least one marker associated with the at least one marker gene in the sample, wherein at least one marker gene is NRAS, wherein the patient is treated with bortezomib prior to the second sample being obtained;
b) comparing the results in a) to the results in the first sample; and
c) determining to continue treatment with bortezomib if the comparison indicates that the tumor cells in the second sample comprise wild type NRAS.

**10.** Use of a kit comprising a stabilizer to add to a sample comprising tumor cells and a reagent to identify the sequence of at least one marker in a sample, wherein the result indicates whether there is a mutation in at least one marker gene, wherein the at least one marker gene is NRAS, in a method of any one of the preceding claims.

**11.** The use of claim 10, wherein the at least one marker is nucleic acid.

**12.** The use of claim 11, wherein the reagent is a primer.

**13.** The use of claim 12, wherein the primer hybridizes to a nucleic acid sequence selected from the group consisting of SEQ ID NO: 1, 2, 4, 5, a sequence on chromosome 1 from base pair 115247085 to 115259515, a sequence on chromosome 12p from base pair 25358180 to 25403854, and a complement of any of the foregoing.

**14.** The use of claim 12 or 13, further comprising a second primer or a probe.

**15.** A proteasome inhibitor for use in treating a patient having a hematological tumor comprising wild type NRAS, comprising the step of administering to the patient a therapeutically effective amount of a proteasome inhibitor, if a sample of hematological tumor cells from the patient has been determined to have wild type NRAS, wherein the proteasome inhibitor is bortezomib.

**16.** The proteasome inhibitor for use according to claim 15, wherein the hematological tumor is selected from the group consisting of myeloma, lymphoma and leukemia.

**17.** The proteasome inhibitor for use according to claim 15 or 16, wherein the hematological tumor further comprises a t(4;14) translocation.

18. A method of identifying a hematological cancer patient who will be nonresponsive to treatment with bortezomib, comprising determining the presence or absence of at least one NRAS mutation by sequencing a marker or a portion of a marker suspected of comprising the mutation in a sample comprising tumor cells from the patient, wherein the at least one NRAS mutation is an activating mutation, whereby the presence of at least one NRAS mutation indicates that the hematological cancer patient will not respond to bortezomib.

19. The method of claim 18, wherein the marker or a portion of a marker comprises SEQ ID NO:2 or a portion thereof comprising codon 12, codon 13 or codon 61.

20. The method of any one of claims 18 to 19, wherein the tumor cells are not of the t(4; 14) translocation subtype.

21. The method of any one of claims 18 to 20, wherein the hematological cancer is selected from the group consisting of myeloma, lymphoma and leukemia.

**Patentansprüche**

1. Verfahren zum Identifizieren eines Krebspatienten als Kandidaten für eine Behandlung mit Bortezomib, wobei der Patient einen hämatologischen Tumor hat, das umfasst:

   a. Identifizieren der Sequenz von mindestens einem Marker, der mit mindestens einem Markergen assoziiert ist, in einer Patientenprobe, die hämatologische Tumorzellen umfasst, wobei es sich bei einem Markergen um Neuroblastoma RAS viral (v-ras) Oncogene Homolog (NRAS) handelt; und
   b. Identifizieren des Patienten als Kandidaten für eine Behandlung mit Bortezomib, wenn die Sequenz anzeigt, dass die Tumorzellen Wildtyp-NRAS umfassen.

2. Verfahren nach Anspruch 1, wobei der mindestens eine Marker aus der Gruppe ausgewählt ist, die aus Nukleinsäure und Protein entsprechend dem mindestens einen Markergen besteht.

3. Verfahren nach Anspruch 1 oder 2, wobei der hämatologische Tumor aus der Gruppe ausgewählt ist, die aus Myelom, Lymphom und Leukämie besteht.

4. Verfahren nach einem der vorhergehenden Ansprüche, wobei die Probe Zellen umfasst, die aus der Gruppe ausgewählt sind, die aus Myelomtumorzellen und Lymphomtumorzellen besteht, und, wahlweise, weiter umfassend das Anreichern der Probe bezüglich Tumorzellen.

5. Verfahren nach einem der vorhergehenden Ansprüche, wobei es sich bei dem mindestens einen Marker um mindestens zwei Marker handelt.

6. Verfahren nach Anspruch 5, wobei die mindestens zwei Marker mindestens einen Marker umfassen, der mit einem NRAS-Markergen assoziiert ist, und einen Tumorsubtypmarker.

7. Verfahren nach Anspruch 6, wobei es sich bei dem Tumorsubtypmarker um eine t(4;14)-Translokation handelt.

8. Verfahren nach Anspruch 4, wobei es sich bei der Probe um Blut handelt.

9. Verfahren nach einem der vorhergehenden Ansprüche, weiter umfassend die Entscheidung, ob die Bortezomib-Behandlung des hämatologischen Tumors in dem Patienten fortgesetzt wird, durch ein Verfahren, das umfasst:

   a. Identifizieren auf einer zweiten biologischen Probe, die Tumorzellen von dem Patienten umfasst, der für die Behandlung mit Bortezomib identifiziert wurde, der Sequenz des mindestens einen Markers, der mit dem mindestens einen Markergen in der Probe assoziiert ist, wobei es sich bei mindestens einem Markergen um NRAS handelt, wobei der Patient mit Bortezomib behandelt wird, bevor die zweite Probe gewonnen wird;
   b. Vergleichen der Ergebnisse in a. mit den Ergebnissen in der ersten Probe; und
   c. Entscheiden, die Behandlung mit Bortezomib fortzusetzen, wenn der Vergleich anzeigt, dass die Tumorzellen in der zweiten Probe Wildtyp-NRAS umfassen.

10. Verwendung eines Testsatzes, umfassend einen Stabilisator, zum Hinzufügen zu einer Probe, die Tumorzellen

umfasst, und ein Reagenz, zum Identifizieren der Sequenz von mindestens einem Marker in einer Probe, wobei das Ergebnis anzeigt, ob eine Mutation in mindestens einem Markergen vorliegt, wobei es sich bei dem mindestens einen Markergen um NRAS handelt, in einem Verfahren nach einem der vorhergehenden Ansprüche.

**11.** Verwendung nach Anspruch 10, wobei es sich bei dem mindestens einen Marker um Nukleinsäure handelt.

**12.** Verwendung nach Anspruch 11, wobei es sich bei dem Reagenz um einen Primer handelt.

**13.** Verwendung nach Anspruch 12, wobei der Primer mit einer Nukleinsäuresequenz hybridisiert, die aus der Gruppe ausgewählt ist, die aus SEQ ID NO: 1, 2, 4, 5, einer Sequenz auf Chromosom 1p von Basenpaar 115247085 bis 115259515, einer Sequenz auf Chromosom 12p von Basenpaar 25358180 bis 25403854, und einem Komplement von einem der vorstehenden besteht.

**14.** Verwendung nach Anspruch 12 oder 13, weiter umfassend einen zweiten Primer oder eine Sonde.

**15.** Proteasomenhemmer zur Verwendung bei der Behandlung eines Patienten mit einem hämatologischem Tumor, der Wildtyp-NRAS umfasst, die den Schritt des Verabreichens einer therapeutisch wirksamen Menge eines Proteasomenhemmers an den Patienten umfasst, wenn von einer Probe hämatologischer Tumorzellen aus dem Patienten bestimmt wurde, dass sie Wildtyp-NRAS umfasst, wobei es sich bei dem Proteasomenhemmer um Bortezomib handelt.

**16.** Proteasomenhemmer zur Verwendung nach Anspruch 15, wobei der hämatologische Tumor aus der Gruppe ausgewählt ist, die aus Myelom, Lymphom und Leukämie besteht.

**17.** Proteasomenhemmer zur Verwendung nach Anspruch 15 oder 16, wobei der hämatologische Tumor weiter eine t(4;14)-Translokation umfasst.

**18.** Verfahren zum Identifizieren eines Hämatologischer-Krebs-Patienten, der auf eine Behandlung mit Bortezomib nicht ansprechen wird, umfassend das Bestimmen der Anwesenheit oder Abwesenheit von mindestens einer NRAS-Mutation durch Sequenzieren eines Markers oder eines Teils eines Markers, von dem man annimmt, dass er die Mutation umfasst, in einer Probe, die Tumorzellen aus dem Patienten umfasst, wobei es sich bei der mindestens einen NRAS-Mutation um eine aktivierende Mutation handelt, wobei die Anwesenheit von mindestens einer NRAS-Mutation anzeigt, dass der Hämatologischer-Krebs-Patient nicht auf Bortezomib ansprechen wird.

**19.** Verfahren nach Anspruch 18, wobei der Marker oder ein Teil eines Markers SEQ ID NO:2 oder einen Teil davon umfassend Codon 12, Codon 13 oder Codon 61 umfasst.

**20.** Verfahren nach einem der Ansprüche 18 bis 19, wobei die Tumorzellen nicht vom t(4;14)-Translokation-Subtyp sind.

**21.** Verfahren nach einem der Ansprüche 18 bis 20, wobei der hämatologische Krebs aus der Gruppe ausgewählt ist, die aus Myelom, Lymphom und Leukämie besteht.

## Revendications

**1.** Procédé d'identification d'un patient cancéreux en tant que candidat pour traitement avec le bortézomib, dans lequel le patient a une tumeur hématologique, comprenant :

a) l'identification de la séquence d'au moins un marqueur associé à au moins un gène marqueur dans un échantillon de patient comprenant des cellules de tumeur hématologique, dans lequel un gène marqueur est l'homologue oncogène de RAS viral (v-ras) de neuroblastome (NRAS) ; et
b) l'identification du patient en tant que candidat pour traitement avec le bortézomib si la séquence indique que les cellules tumorales comprennent NRAS de type sauvage.

**2.** Procédé de la revendication 1, dans lequel l'au moins un marqueur est choisi dans le groupe constitué d'un acide nucléique et une protéine correspondant à l'au moins un gène marqueur.

**3.** Procédé de la revendication 1 ou 2, dans lequel la tumeur hématologique est choisie dans le groupe constitué d'un

myélome, un lymphome et une leucémie.

4. Procédé de l'une quelconque des revendications précédentes, dans lequel l'échantillon comprend des cellules choisies dans le groupe constitué de cellules tumorales de myélome et de cellules tumorales de lymphome, et facultativement, comprenant en outre l'enrichissement de l'échantillon en cellules tumorales.

5. Procédé de l'une quelconque des revendications précédentes, dans lequel l'au moins un marqueur est au moins deux marqueurs.

6. Procédé de la revendication 5, dans lequel les au moins deux marqueurs comprennent au moins un marqueur associé à un gène marqueur NRAS et un marqueur de sous-type de tumeur.

7. Procédé de la revendication 6, dans lequel le marqueur de sous-type de tumeur est une translocation t(4;14).

8. Procédé de la revendication 4, dans lequel l'échantillon est du sang.

9. Procédé de l'une quelconque des revendications précédentes, comprenant en outre la détermination du fait que le traitement avec le bortézomib de la tumeur hématologique dans le patient doit continuer ou non par un procédé comprenant :

a) l'identification, sur un deuxième échantillon biologique comprenant des cellules tumorales du patient identifié pour traitement avec le bortézomib, de la séquence de l'au moins un marqueur associé à l'au moins un gène marqueur dans l'échantillon, dans lequel au moins un gène marqueur est NRAS, dans lequel le patient est traité avec le bortézomib avant que le deuxième échantillon soit obtenu ;
b) la comparaison des résultats dans a) aux résultats dans le premier échantillon ; et
c) la détermination du fait que le traitement avec le bortézomib doit continuer si la comparaison indique que les cellules tumorales dans le deuxième échantillon comprennent NRAS de type sauvage.

10. Utilisation d'un kit comprenant un stabilisant à ajouter à un échantillon comprenant des cellules tumorales et un réactif pour identifier la séquence d'au moins un marqueur dans un échantillon, dans laquelle le résultat indique si une mutation est présente dans au moins un gène marqueur, dans laquelle l'au moins un gène marqueur est NRAS, dans un procédé de l'une quelconque des revendications précédentes.

11. Utilisation de la revendication 10, dans laquelle l'au moins un marqueur est un acide nucléique.

12. Utilisation de la revendication 11, dans laquelle le réactif est une amorce.

13. Utilisation de la revendication 12, dans laquelle l'amorce s'hybride à une séquence d'acide nucléique choisie dans le groupe constitué de SEQ ID NO: 1, 2, 4, 5, une séquence sur le chromosome 1p des paires de bases 115247085 à 115259515, une séquence sur le chromosome 12p des paires de bases 25358180 à 25403854, et un complément de l'une quelconque de celles-ci.

14. Utilisation de la revendication 12 ou 13, comprenant en outre une deuxième amorce ou une sonde.

15. Inhibiteur de protéasome pour utilisation dans le traitement d'un patient ayant une tumeur hématologique comprenant NRAS de type sauvage, comprenant l'étape d'administration au patient d'une quantité thérapeutiquement efficace d'un inhibiteur de protéasome, s'il a été déterminé qu'un échantillon de cellules de tumeur hématologique du patient contient NRAS de type sauvage, l'inhibiteur de protéasome étant le bortézomib.

16. Inhibiteur de protéasome pour utilisation selon la revendication 15, la tumeur hématologique étant choisie dans le groupe constitué d'un myélome, un lymphome et une leucémie.

17. Inhibiteur de protéasome pour utilisation selon la revendication 15 ou 16, la tumeur hématologique comprenant en outre une translocation t(4;14).

18. Procédé d'identification d'un patient atteint d'un cancer hématologique qui sera non-répondeur au traitement avec le bortézomib, comprenant la détermination de la présence ou l'absence d'au moins une mutation de NRAS par séquençage d'un marqueur ou une partie d'un marqueur suspecté de comprendre la mutation dans un échantillon

comprenant des cellules tumorales du patient, dans lequel l'au moins une mutation NRAS est une mutation activatrice, dans laquelle la présence d'au moins une mutation de NRAS indique que le patient atteint d'un cancer hématologique ne répondra pas au bortézomib.

**19.** Procédé de la revendication 18, dans lequel le marqueur ou une partie d'un marqueur comprend SEQ ID NO: 2 ou une partie de de celui-ci comprenant le codon 12, le codon 13 ou le codon 61.

**20.** Procédé de l'une quelconque des revendications 18 à 19, dans lequel les cellules tumorales ne sont pas du soustype de translocation t(4;14).

**21.** Procédé de l'une quelconque des revendications 18 à 20, dans lequel le cancer hématologique est choisi dans le groupe constitué d'un myélome, un lymphome et une leucémie.

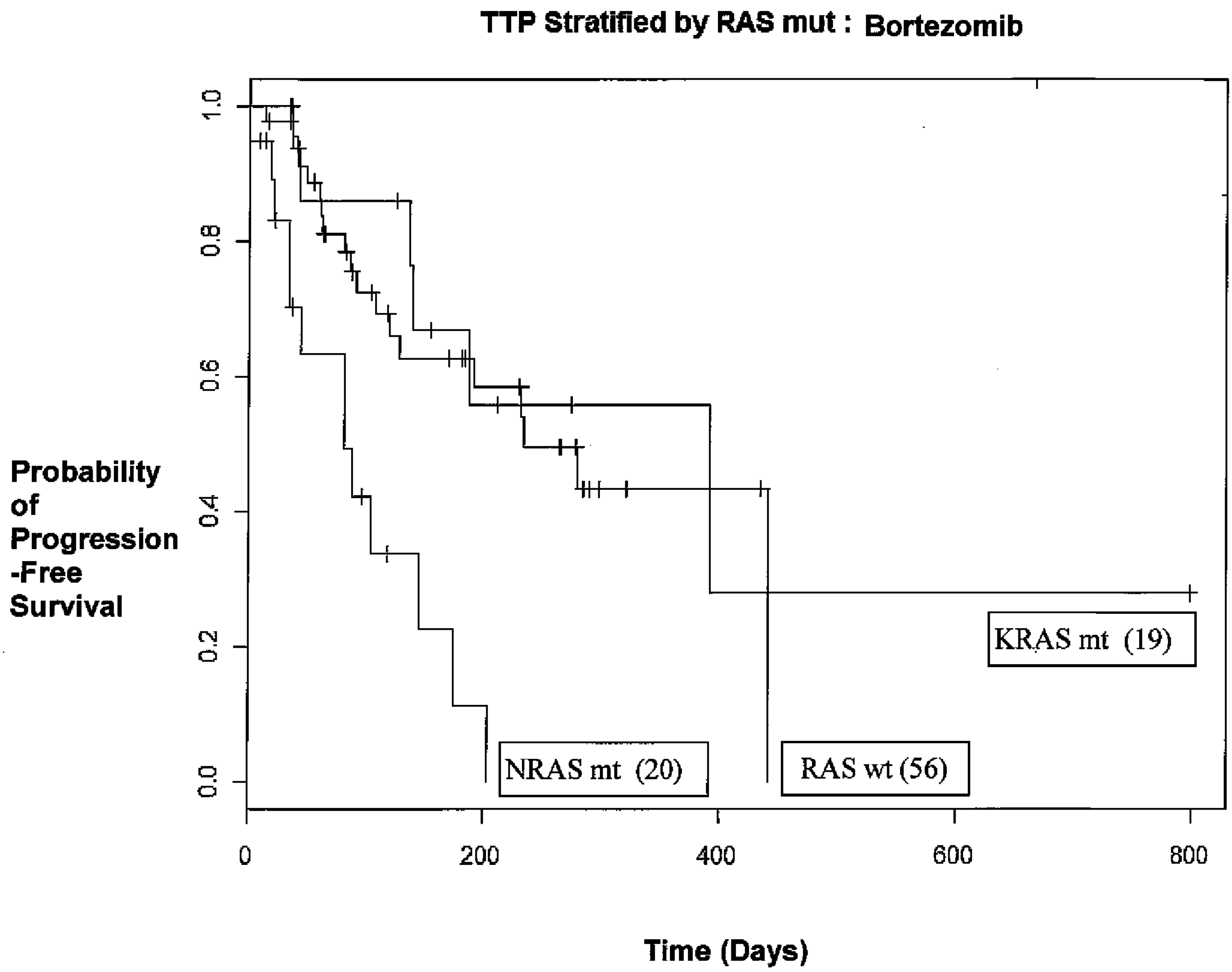
TTP Stratified by RAS mut : Bortezomib
Probability of Progression -Free Survival
0.0
0.2
0.4
0.6
0.8
1.0
0
200
400
600
800
Time (Days)
KRAS mt (19)
NRAS mt (20)
RAS wt (56)

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004053066 A
- WO 9525533 A
- US 5780454 A, Adams
- US 6066730 A
- US 6083903 A
- US 6297217 B
- US 6465433 B
- US 6548668 B
- US 6617317 B
- US 6747150 B
- US 7442830 B
- WO 2009154737 A
- US 7915236 B
- WO 2010036357 A
- WO 2011123502 A
- US 7867662 B
- US 8003819 B
- WO 02059131 A, Plamondon
- WO 02059130 A, Gupta
- WO 09154737 A, Elliott
- US 5693617 A, Stein
- WO 9113904 A
- WO 05105826 A
- US 6831099 B, Crews
- WO 05111008 A
- WO 06045066 A
- US 20050245435 A
- US 6310057 B, Chatterjee and Mallamo
- US 6096778 A
- US 6075150 A, Wang
- US 6781000 B
- WO 05030707 A
- WO 03018557 A
- WO 05115431 A
- US 5756764 A, Fenteany
- US 6147223 A
- US 6335358 B
- US 6645999 B
- WO 05003137 A
- WO 05002572 A
- WO 04071382 A
- US 2005023162 A, Xiao and Patel
- WO 05099687 A
- US 4843155 A, Chomczynski
- US 5346994 A
- US 5539083 A
- WO 0105935 A
- US 5631169 A, Lakowicz
- US 4868103 A, Stavrianopoulos
- US 7045610 B
- US 4683202 A, Mullis
- US 5854033 A, Lizardi
- US 5242974 A
- US 5384261 A
- US 5405783 A
- US 5412087 A
- US 5424186 A
- US 5429807 A
- US 5436327 A
- US 5445934 A
- US 5556752 A
- US 5472672 A
- US 5527681 A
- US 5529756 A
- US 5545531 A
- US 5554501 A
- US 5561071 A
- US 5571639 A
- US 5593839 A
- US 5624711 A
- US 5700637 A
- US 5744305 A
- US 5770456 A
- US 5770722 A
- US 5837832 A
- US 5856101 A
- US 5874219 A
- US 5885837 A
- US 5919523 A
- US 5981185 A
- US 6022963 A
- US 6077674 A
- US 6156501 A
- US 6261776 B
- US 6346413 B
- US 6440677 B
- US 6451536 B
- US 6576424 B
- US 6610482 B
- US 5143854 A
- US 5288644 A
- US 5324633 A
- US 5432049 A
- US 5470710 A
- US 5492806 A
- US 5503980 A
- US 5510270 A
- US 5525464 A

- US 5547839 A
- US 5580732 A
- US 5661028 A
- US 5848659 A
- US 6162963 A
- US 6150584 A
- US 6114598 A
- US 6075181 A
- US 5545807 A
- US 5545806 A
- US 5625825 A
- WO 9312227 A
- US 6331415 B
- US 5223409 A
- US 61558470 B

**Non-patent literature cited in the description**


- **KING et al.** *Science,* 1996, vol. 274, 1652-1659
- **READ et al.** *Immunity,* 1995, vol. 2, 493-506
- **ZETTER.** *Seminars in Cancer Biology,* 1993, vol. 4, 219-229
- **MOREOVER, BEG ; BALTIMORE.** *Science,* 1996, vol. 274, 782
- **RUGGERI et al.** *Adv. Pharmacol.,* 2009, vol. 57, 91-135
- **KUPPERMAN et al.** *Cancer Res.,* 2010, vol. 70, 1970-1980
- **SNYDER et al.** *J. Am. Chem. Soc.,* 1958, vol. 80, 3611
- **IQBAL et al.** *J. Med. Chem.,* 1995, vol. 38, 2276-2277
- **SPALTENSTEIN et al.** *Tetrahedron Lett.,* 1996, vol. 37, 1343
- **MENG.** *Proc. Natl. Acad. Sci.,* 1999, vol. 96, 10403
- **MENG.** *Cancer Res.,* 1999, vol. 59, 2798
- *J. Med. Chem.,* 2005, vol. 48, 5038
- **RYDZEWSKI et al.** *J. Med. Chem.,* 2006, vol. 49, 2953
- **BOGYO et al.** *Proc. Natl. Acad. Sci.,* 1997, vol. 94, 6629
- **BOUGET et al.** *Bioorg. Med. Chem.,* 2003, vol. 11, 4881
- **FENTEANY et al.** *Proc. Natl. Acad. Sci. USA,* 1994, vol. 91, 3358
- **CHAPMAN et al.** *Nature,* 2011, vol. 471, 467-472
- **PRIOR ; HANCOCK.** *Semin.Clin. Dev. Biol.,* 2011
- **CUIFFO ; REN.** *Blood,* 2010, vol. 114, 3598-3605
- **LIM et al.** *Eur. J. Biochem.,* 1996, vol. 242, 171-185
- **SAMBROOK et al.** Molecular Cloning: A Laboratory Manual. Cold Spring Harbor Laboratory Press, 2000
- **HARLOW, E. ; LANE, D.** Antibodies: A Laboratory Manual. Cold Spring Harbor Laboratory Press, 1988
- **FOTIADOU et al.** *Mol. Cel. Biol.,* 2007, vol. 27, 6742-6755
- **BLADE et al.** *Br J Haematol.,* 1998, vol. 102, 1115-23
- **EISENHAUER et al.** *E. J. Canc.,* 2009, vol. 45, 228-247
- **CHESON et al.** *J. Clin. Oncol.,* 2003, vol. 21, 4642-4649
- **CHESON et al.** *J. Clin. Oncol.,* 2007, vol. 25, 579-596
- **PILARSKI et al.** *Blood,* 2000, vol. 95, 1056-65
- **RIGOLIN et al.** *Blood,* 2006, vol. 107, 2531-5
- **ALIZADEH et al.** *Nature,* 2000, vol. 403, 503-511
- **MCDERMOTT et al.** *PNAS,* 2007, vol. 104, 19936-19941
- **ZHANG et al.** *J. Genet. Genomics,* 2011, vol. 38, 95-109
- **SHENDURE ; HANLEE.** *Nature Biotech.,* 2008, vol. 26, 1135-1145
- **AUSUBEL et al.** Current Protocols in Molecular Biology. John Wiley & Sons, 1987
- **CHOMCZYNSKI ; SACCHI.** *Anal. Biochern.,* 1987, vol. 162, 156-159
- **CHIRGWIN et al.** *Biochemistry,* 1979, vol. 18, 5294-99
- **SAMBROOK et al.** Molecular Cloning--A Laboratory Manual. Cold Spring Harbor Laboratory, 1989
- **AUSUBEL et al.** Current Protocols In Molecular Biology. Current Protocols Publishing, 1994, vol. 2
- **MYERS et al.** *Science,* 1985, vol. 230, 1242
- **SAIKI et al.** *Nature,* 1986, vol. 324, 163
- **SAIKI et al.** *Proc. Natl Acad. Sci USA,* 1989, vol. 86, 6230
- **WALLACE et al.** *Nucl. Acids Res.,* 1979, vol. 6, 3543
- **EGHOLM et al.** *Nature,* 1993, vol. 363, 566-568
- **HUGHES et al.** *Nat. Biotech.,* 2001, vol. 19, 342-7
- **LITTELL ; MILIKEN ; STROUP ; WOLFINGER ; SCHABENBERGER.** SAS for Mixed Models. SAS Institute, Inc, 2006
- **MULLIGAN et al.** *Blood,* 2006, vol. 109, 3177-88
- **CHNG et al.** *Cancer Res.,* 2007, vol. 67, 292-9

- **GILMAN A.G. et al.** The Pharmacological Basis of Therapeutics. 1990, vol. 12, 1202-1263
- **SJOLANDER, S. ; URBANICZKY, C.** *Anal. Chem.,* 1991, vol. 63, 2338-2345
- **SZABO et al.** *Curr. Opin. Struct. Biol.,* 1995, vol. 5, 699-705
- **RIVAS, G. ; MINTON, A.P.** *Trends Biochem Sci.,* 1993, vol. 18, 284-7
- **HEEGAARD, N.H.** *J. Mol. Recognit.,* 1998, vol. 11, 141-8
- **HAGE, D.S. ; TWEED, S.A.** *J. Chromatogr. B. Biomed. Sci. Appl.,* 1997, vol. 699, 499-525
- Current Protocols in Molecular Biology,. John Wiley & Sons, 1987
- Current Protocols in Molecular Biology. John Wiley & Sons, Inc, 1995
- **SAMBROOK et al.** Molecular Cloning: A Laboratory Manual. Cold Spring Harbor Press, 1989
- **CHURCH ; GILBERT.** *Proc. Natl. Acad. Sci. USA,* 1984, vol. 81, 1991-1995
- **BARANY.** *Proc. Natl. Acad. Sci. USA,* 1991, vol. 88, 189-193
- **GUATELLI et al.** *Proc. Natl. Acad. Sci. USA,* 1990, vol. 87, 1874-1878
- **KWOH et al.** *Proc. Natl. Acad. Sci. USA,* 1989, vol. 86, 1173-1177
- **LIZARDI et al.** *Bio/Technology,* 1988, vol. 6, 1197
- **LIAO et al.** *Arthritis Rheum.,* 2004, vol. 50, 3792-3803
- **SHENA et al.** *Tibtech,* 1998, vol. 16, 301
- **DUGGAN et al.** *Nat. Genet.,* 1999, vol. 21, 10
- **BOWTELL et al.** *Nat. Genet.,* 1999, vol. 21, 25
- **LIPSHUTZ et al.** *Nature Genet.,* 1999, vol. 21, 20-24
- **BLANCHARD et al.** *Biosensors and Bioelectronics,* 1996, vol. 11, 687-90
- **MASKOS et al.** *Nucleic Acids Res.,* 1993, vol. 21, 4663-69
- **HUGHES et al.** *Nat. Biotechol.,* 2001, vol. 19, 342
- **HANS et al.** *Blood,* 2004, vol. 103, 275-282
- **BRADFORD et al.** 24. *Urol. Oncol.,* 2006, 237-242
- **WARD et al.** *Nature,* 1989, vol. 341, 544-546
- **CORTEZ-RETAMOZO et al.** *Cancer Res.,* 2004, vol. 64, 2853-2857
- **BIRD et al.** *Science,* 1988, vol. 242, 423-426
- **HUSTON et al.** *Proc. Natl. Acad. Sci. USA,* 1988, vol. 85, 5879-5883
- **KOHLER ; MILSTEIN.** *Nature,* 1975, vol. 256, 495
- **KOZBOR et al.** *Immunol.,* 1983, vol. 4, 72
- **COLE et al.** In Monoclonal Antibodies and Cancer Therapy. Alan R. Liss, Inc, 1985, 77-96
- Current Protocols in Immunology. John Wiley & Sons, 1994
- **ZUCKERMANN et al.** *J. Med. Chem.,* 1994, vol. 37, 2678-85
- **LAM.** *Anticancer Drug Des.,* 1997, vol. 12, 145
- **HOUGHTEN.** *Biotechniques,* 1992, vol. 13, 412-421
- **LAM.** *Nature,* 1991, vol. 354, 82-84
- **FODOR.** *Nature,* 1993, vol. 364, 555-556
- **CULL et al.** *Proc Natl Acad Sci USA,* 1992, vol. 89, 1865-1869
- **SCOTT ; SMITH.** *Science,* 1990, vol. 249, 386-390
- **DEVLIN.** *Science,* 1990, vol. 249, 404-406
- **CWIRLA et al.** *Proc. Natl. Acad. Sci.,* 1990, vol. 87, 6378-6382
- **FELICI.** *J. Mol. Biol.,* 1991, vol. 222, 301-310
- **ORLOWSKI.** *J Clin Oncol.,* 15 November 2002, vol. 20 (22), 4420-7
- **AGHAJANIAN.** *Clin Cancer Res.,* August 2002, vol. 8 (8), 2505-11
- *CHEMICAL ABSTRACTS,* 179324-69-7
- *CHEMICAL ABSTRACTS,* 312-93-6
- **JAGANNATH et al.** *Br. J. Haematol.,* 2004, vol. 127, 165-172
- **RICHARDSON PG et al.** *N. Engl. J. Med.,* 2003, vol. 348, 2609-2617
- **RICHARDSON et al.** *N. Engl. J. Med.,* 2005, vol. 352, 2487-2498
- **BLADE et al.** *Br. J. Haematol.,* 1998, vol. 102, 1115-23
- **SOVERINI S et al.** *Haematologica,* 2002, vol. 87, 1036-1040
- **AVET-LOISEAU et al.** *J. Clin. Oncol.,* 2010, vol. 28, 4630-4634
- **WALKER et al.** *Blood,* 2012, vol. 120, 1077-1086
- **MULLIGAN et al.** *Blood,* 2007, vol. 109, 3177-3188

SEQUENCE LISTING

<110> Berger, Allison
Bernard, Hugues
Chattopadhyay, Nibedita
Koenig, Erik M.
Mulligan, George J.
Schu, Matthew C.

Millennium Pharmaceuticals, Inc.

<120> BIOMARKERS OF RESPONSE TO PROTEASOME INHIBITORS

<130> MPI11-014P1RNWOM

<150> US 61/558470
<151> 2011-11-11

<160> 6

<170> FastSEQ for Windows Version 4.0

<210> 1
<211> 4454
<212> DNA
<213> Homo sapiens

<400> 1

```
gaaacgtccc gtgtgggagg ggcgggtctg ggtgcggcct gccgcatgac tcgtggttcg 60
gaggcccacg tggccggggc ggggactcag gcgcctgggg cgccgactga ttacgtagcg 120
ggcggggccg gaagtgccgc tccttggtgg gggctgttca tggcggttcc ggggtctcca 180
acatttttcc cggctgtggt cctaaatctg tccaaagcag aggcagtgga gcttgaggtt 240
cttgctggtg tgaaatgact gagtacaaac tggtggtggt tggagcaggt ggtgttggga 300
aaagcgcact gacaatccag ctaatccaga accactttgt agatgaatat gatcccacca 360
tagaggattc ttacagaaaa caagtggtta tagatggtga aacctgtttg ttggacatac 420
tggatacagc tggacaagaa gagtacagtg ccatgagaga ccaatacatg aggacaggcg 480
aaggcttcct ctgtgtattt gccatcaata atagcaagtc atttgcggat attaacctct 540
acagggagca gattaagcga gtaaaagact cggatgatgt acctatggtg ctagtgggaa 600
acaagtgtga tttgccaaca aggacagttg atacaaaaca agcccacgaa ctggccaaga 660
gttacgggat tccattcatt gaaacctcag ccaagaccag acagggtgtt gaagatgctt 720
tttacacact ggtaagagaa atacgccagt accgaatgaa aaaactcaac agcagtgatg 780
atgggactca gggttgtatg ggattgccat gtgtggtgat gtaacaagat acttttaaag 840
ttttgtcaga aaagagccac tttcaagctg cactgacacc ctggtcctga cttccctgga 900
ggagaagtat tcctgttgct gtcttcagtc tcacagagaa gctcctgcta cttccccagc 960
tctcagtagt ttagtacaat aatctctatt tgagaagttc tcagaataac tacctcctca 1020
cttggctgtc tgaccagaga atgcacctct tgttactccc tgttattttt ctgccctggg 1080
ttcttccaca gcacaaacac acctctgcca ccccaggttt ttcatctgaa aagcagttca 1140
tgtctgaaac agagaaccaa accgcaaacg tgaaattcta ttgaaaacag tgtcttgagc 1200
tctaaagtag caactgctgg tgattttttt tttcttttta ctgttgaact tagaactatg 1260
ctaatttttg gagaaatgtc ataaattact gttttgccaa gaatatagtt attattgctg 1320
tttggtttgt ttataatgtt atcggctcta ttctctaaac tggcatctgc tctagattca 1380
taaatacaaa aatgaatact gaattttgag tctatcctag tcttcacaac tttgacgtaa 1440
ttaaatccaa ctttcacagt gaagtgcctt tttcctagaa gtggtttgta gacttccttt 1500
ataatatttc agtggaatag atgtctcaaa aatccttatg catgaaatga atgtctgaga 1560
tacgtctgtg acttatctac cattgaagga aagctatatc tatttgagag cagatgccat 1620
tttgtacatg tatgaaattg gttttccaga ggcctgtttt ggggctttcc caggagaaag 1680
atgaaactga aagcacatga ataatttcac ttaataattt ttacctaatc tccacttttt 1740
tcataggtta ctacctatac aatgtatgta atttgtttcc cctagcttac tgataaacct 1800
aatattcaat gaacttccat ttgtattcaa atttgtgtca taccagaaag ctctacattt 1860
gcagatgttc aaatattgta aaactttggt gcattgttat ttaatagctg tgatcagtga 1920
ttttcaaacc tcaaatatag tatattaaca aattacattt tcactgtata tcatggtatc 1980
ttaatgatgt atataattgc cttcaatccc cttctcaccc caccctctac agcttccccc 2040
```

acagcaatag gggcttgatt atttcagttg agtaaagcat ggtgctaatg gaccagggtc 2100
acagtttcaa aacttgaaca atccagttag catcacagag aaagaaattc ttctgcattt 2160
gctcattgca ccagtaactc cagctagtaa ttttgctagg tagctgcagt tagccctgca 2220
aggaaagaag aggtcagtta gcacaaaccc tttaccatga ctggaaaact cagtatcacg 2280
tatttaaaca tttttttttc ttttagccat gtagaaactc taaattaagc caatattctc 2340
atttgagaat gaggatgtct cagctgagaa acgttttaaa ttctctttat tcataatgtt 2400
ctttgaaggg tttaaaacaa gatgttgata aatctaagct gatgagtttg ctcaaaacag 2460
gaagttgaaa ttgttgagac aggaatggaa aatataatta attgatacct atgaggattt 2520
ggaggcttgg cattttaatt tgcagataat accctggtaa ttctcatgaa aaatagactt 2580
ggataacttt tgataaaaga ctaattccaa aatggccact ttgttcctgt ctttaatatc 2640
taaatactta ctgaggtcct ccatcttcta tattatgaat tttcatttat taagcaaatg 2700
tcatattacc ttgaaattca gaagagaaga aacatatact gtgtccagag tataatgaac 2760
ctgcagagtt gtgcttctta ctgctaattc tgggagcttt cacagtactg tcatcatttg 2820
taaatggaaa ttctgctttt ctgtttctgc tccttctgga gcagtgctac tctgtaattt 2880
tcctgaggct tatcacctca gtcatttctt ttttaaatgt ctgtgactgg cagtgattct 2940
ttttcttaaa aatctattaa atttgatgtc aaattaggga gaaagatagt tactcatctt 3000
gggctcttgt gccaatagcc cttgtatgta tgtacttaga gttttccaag tatgttctaa 3060
gcacagaagt ttctaaatgg ggccaaaatt cagacttgag tatgttcttt gaatacctta 3120
agaagttaca attagccggg catggtggcc cgtgcctgta gtcccagcta cttgagaggc 3180
tgaggcagga gaatcacttc aacccaggag gtggaggtta cagtgagcag agatcgtgcc 3240
actgcactcc agcctgggtg acaagagaga cttgtctcca aaaaaaaagt tacacctagg 3300
tgtgaatttt ggcacaaagg agtgacaaac ttatagttaa aagctgaata acttcagtgt 3360
ggtataaaac gtggttttta ggctatgttt gtgattgctg aaaagaattc tagtttacct 3420
caaaatcctt ctctttcccc aaattaagtg cctggccagc tgtcataaat tacatattcc 3480
ttttggtttt tttaaaggtt acatgttcaa gagtgaaaat aagatgttct gtctgaaggc 3540
taccatgccg gatctgtaaa tgaacctgtt aaatgctgta tttgctccaa cggcttacta 3600
tagaatgtta cttaatacaa tatcatactt attacaattt ttactatagg agtgtaatag 3660
gtaaaattaa tctctatttt agtgggccca tgtttagtct ttcaccatcc tttaaactgc 3720
tgtgaatttt tttgtcatga cttgaaagca aggatagaga aacactttag agatatgtgg 3780
ggttttttta ccattccaga gcttgtgagc ataatcatat ttgctttata tttatagtca 3840
tgaactccta agttggcagc tacaaccaag aaccaaaaaa tggtgcgttc tgcttcttgt 3900
aattcatctc tgctaataaa ttataagaag caaggaaaat tagggaaaat attttatttg 3960
gatggtttct ataaacaagg gactataatt cttgtacatt atttttcatc tttgctgttt 4020
ctttgagcag tctaatgtgc cacacaatta tctaaggtat ttgttttcta taagaattgt 4080
tttaaaagta ttcttgttac cagagtagtt gtattatatt tcaaaacgta agatgatttt 4140
taaaagcctg agtactgacc taagatggaa ttgtatgaac tctgctctgg agggagggga 4200
ggatgtccgt ggaagttgta agacttttat ttttttgtgc catcaaatat aggtaaaaat 4260
aattgtgcaa ttctgctgtt taaacaggaa ctattggcct ccttggccct aaatggaagg 4320
gccgatattt taagttgatt attttattgt aaattaatcc aacctagttc tttttaattt 4380
ggttgaatgt tttttcttgt taaatgatgt ttaaaaaata aaaactggaa gttcttggct 4440
tagtcataat tctt 4454

<210> 2
<211> 570
<212> DNA
<213> Homo sapiens

<400> 2

atgactgagt acaaactggt ggtggttgga gcaggtggtg ttgggaaaag cgcactgaca 60
atccagctaa tccagaacca ctttgtagat gaatatgatc ccaccataga ggattcttac 120
agaaaacaag tggttataga tggtgaaacc tgtttgttgg acatactgga tacagctgga 180
caagaagagt acagtgccat gagagaccaa tacatgagga caggcgaagg cttcctctgt 240
gtatttgcca tcaataatag caagtcattt gcggatatta acctctacag ggagcagatt 300
aagcgagtaa aagactcgga tgatgtacct atggtgctag tgggaaacaa gtgtgatttg 360
ccaacaagga cagttgatac aaaacaagcc cacgaactgg ccaagagtta cgggattcca 420
ttcattgaaa cctcagccaa gaccagacag ggtgttgaag atgcttttta cacactggta 480
agagaaatac gccagtaccg aatgaaaaaa ctcaacagca gtgatgatgg gactcagggt 540
tgtatgggat tgccatgtgt ggtgatgtaa 570

<210> 3
<211> 189
<212> PRT
<213> Homo sapiens

<400> 3

```
Met Thr Glu Tyr Lys Leu Val Val Val Gly Ala Gly Gly Val Gly Lys
 1               5                  10                  15
Ser Ala Leu Thr Ile Gln Leu Ile Gln Asn His Phe Val Asp Glu Tyr
            20                  25                  30
Asp Pro Thr Ile Glu Asp Ser Tyr Arg Lys Gln Val Val Ile Asp Gly
        35                  40                  45
Glu Thr Cys Leu Leu Asp Ile Leu Asp Thr Ala Gly Gln Glu Glu Tyr
    50                  55                  60
Ser Ala Met Arg Asp Gln Tyr Met Arg Thr Gly Glu Gly Phe Leu Cys
65                  70                  75                  80
Val Phe Ala Ile Asn Asn Ser Lys Ser Phe Ala Asp Ile Asn Leu Tyr
                85                  90                  95
Arg Glu Gln Ile Lys Arg Val Lys Asp Ser Asp Asp Val Pro Met Val
            100                 105                 110
Leu Val Gly Asn Lys Cys Asp Leu Pro Thr Arg Thr Val Asp Thr Lys
        115                 120                 125
Gln Ala His Glu Leu Ala Lys Ser Tyr Gly Ile Pro Phe Ile Glu Thr
    130                 135                 140
Ser Ala Lys Thr Arg Gln Gly Val Glu Asp Ala Phe Tyr Thr Leu Val
145                 150                 155                 160
Arg Glu Ile Arg Gln Tyr Arg Met Lys Lys Leu Asn Ser Ser Asp Asp
                165                 170                 175
Gly Thr Gln Gly Cys Met Gly Leu Pro Cys Val Val Met
            180                 185
```

<210> 4
<211> 5312
<212> DNA
<213> Homo sapiens

<400> 4

ggccgcggcg gcggaggcag cagcggcggc ggcagtggcg gcggcgaagg tggcggcggc 60
tcggccagta ctcccggccc ccgccatttc ggactgggag cgagcgcggc gcaggcactg 120
aaggcggcgg cggggccaga ggctcagcgg ctcccaggtg cgggagagag gcctgctgaa 180
aatgactgaa tataaacttg tggtagttgg agctggtggc gtaggcaaga gtgccttgac 240
gatacagcta attcagaatc attttgtgga cgaatatgat ccaacaatag aggattccta 300
caggaagcaa gtagtaattg atggagaaac ctgtctcttg gatattctcg acacagcagg 360
tcaagaggag tacagtgcaa tgagggacca gtacatgagg actggggagg gctttctttg 420
tgtatttgcc ataaataata ctaaatcatt tgaagatatt caccattata gagaacaaat 480
taaaagagtt aaggactctg aagatgtacc tatggtccta gtaggaaata aatgtgattt 540
gccttctaga acagtagaca caaaacaggc tcaggactta gcaagaagtt atggaattcc 600
ttttattgaa acatcagcaa agacaagaca gggtgttgat gatgccttct atacattagt 660
tcgagaaatt cgaaaacata aagaaaagat gagcaaagat ggtaaaaaga agaaaaagaa 720
gtcaaagaca aagtgtgtaa ttatgtaaat acaatttgta ctttttctt aaggcatact 780
agtacaagtg gtaattttg tacattacac taaattatta gcatttgttt tagcattacc 840
taattttttt cctgctccat gcagactgtt agcttttacc ttaaatgctt attttaaaat 900
gacagtggaa gttttttttt cctctaagtg ccagtattcc cagagttttg gtttttgaac 960
tagcaatgcc tgtgaaaaag aaactgaata cctaagattt ctgtcttggg gtttttggtg 1020
catgcagttg attacttctt atttttctta ccaattgtga atgttggtgt gaaacaaatt 1080
aatgaagctt ttgaatcatc cctattctgt gttttatcta gtcacataaa tggattaatt 1140
actaatttca gttgagacct tctaattggt ttttactgaa acattgaggg aacacaaatt 1200
tatgggcttc ctgatgatga ttcttctagg catcatgtcc tatagtttgt catccctgat 1260
gaatgtaaag ttacactgtt cacaaaggtt ttgtctcctt tccactgcta ttagtcatgg 1320
tcactctccc caaaatatta tattttttct ataaaaagaa aaaaatggaa aaaaattaca 1380
aggcaatgga aactattata aggccatttc cttttcacat tagataaatt actataaaga 1440
ctcctaatag cttttcctgt taaggcagac ccagtatgaa atggggatta ttatagcaac 1500
cattttgggg ctatatttac atgctactaa atttttataa taattgaaaa gattttaaca 1560
agtataaaaa attctcatag gaattaaatg tagtctccct gtgtcagact gctctttcat 1620
agtataactt taaatctttt cttcaacttg agtctttgaa gatagtttta attctgcttg 1680
tgacattaaa agattatttg ggccagttat agcttattag gtgttgaaga gaccaaggtt 1740

```
gcaaggccag gccctgtgtg aacctttgag ctttcataga gagtttcaca gcatggactg 1800
tgtccccacg gtcatccagt gttgtcatgc attggttagt caaaatgggg agggactagg 1860
gcagtttgga tagctcaaca agatacaatc tcactctgtg gtggtcctgc tgacaaatca 1920
agagcattgc ttttgtttct taagaaaaca aactcttttt taaaaattac ttttaaatat 1980
taactcaaaa gttgagattt tggggtggtg gtgtgccaag acattaattt tttttttaaa 2040
caatgaagtg aaaaagtttt acaatctcta ggtttggcta gttctcttaa cactggttaa 2100
attaacattg cataaacact tttcaagtct gatccatatt taataatgct ttaaaataaa 2160
aataaaaaca atccttttga taaatttaaa atgttactta ttttaaaata aatgaagtga 2220
gatggcatgg tgaggtgaaa gtatcactgg actaggaaga aggtgactta ggttctagat 2280
aggtgtcttt taggactctg attttgagga catcacttac tatccatttc ttcatgttaa 2340
aagaagtcat ctcaaactct tagttttttt tttttacaac tatgtaattt atattccatt 2400
tacataagga tacacttatt tgtcaagctc agcacaatct gtaaattttt aacctatgtt 2460
acaccatctt cagtgccagt cttgggcaaa attgtgcaag aggtgaagtt tatatttgaa 2520
tatccattct cgttttagga ctcttcttcc atattagtgt catcttgcct ccctaccttc 2580
cacatgcccc atgacttgat gcagttttaa tacttgtaat tcccctaacc ataagattta 2640
ctgctgctgt ggatatctcc atgaagtttt cccactgagt cacatcagaa atgccctaca 2700
tcttatttcc tcagggctca agagaatctg acagatacca taaagggatt tgacctaatc 2760
actaatttc aggtggtggc tgatgctttg aacatctctt tgctgcccaa tccattagcg 2820
acagtaggat ttttcaaacc tggtatgaat agacagaacc ctatccagtg gaaggagaat 2880
ttaataaaga tagtgctgaa agaattcctt aggtaatcta taactaggac tactcctggt 2940
aacagtaata cattccattg ttttagtaac cagaaatctt catgcaatga aaaatacttt 3000
aattcatgaa gcttactttt tttttttggt gtcagagtct cgctcttgtc acccaggctg 3060
gaatgcagtg gcgccatctc agctcactgc aacctccatc tcccaggttc aagcgattct 3120
cgtgcctcgg cctcctgagt agctgggatt acaggcgtgt gccactacac tcaactaatt 3180
tttgtatttt taggagagac ggggtttcac cctgttggcc aggctggtct cgaactcctg 3240
acctcaagtg attcacccac cttggcctca taaacctgtt ttgcagaact catttattca 3300
gcaaatattt attgagtgcc taccagatgc cagtcaccgc acaaggcact gggtatatgg 3360
tatccccaaa caagagacat aatcccggtc cttaggtagt gctagtgtgg tctgtaatat 3420
cttactaagg cctttggtat acgacccaga gataacacga tgcgtatttt agttttgcaa 3480
agaaggggtt tggtctctgt gccagctcta taattgtttt gctacgattc cactgaaact 3540
cttcgatcaa gctactttat gtaaatcact tcattgtttt aaaggaataa acttgattat 3600
attgtttttt tatttggcat aactgtgatt cttttaggac aattactgta cacattaagg 3660
tgtatgtcag atattcatat tgacccaaat gtgtaatatt ccagttttct ctgcataagt 3720
aattaaaata tacttaaaaa ttaatagttt tatctgggta caaataaaca ggtgcctgaa 3780
ctagttcaca gacaaggaaa cttctatgta aaaatcacta tgatttctga attgctatgt 3840
gaaactacag atctttggaa cactgtttag gtagggtgtt aagacttaca cagtacctcg 3900
tttctacaca gagaaagaaa tggccatact tcaggaactg cagtgcttat gaggggatat 3960
ttaggcctct tgaatttttg atgtagatgg gcattttttt aaggtagtgg ttaattacct 4020
ttatgtgaac tttgaatggt ttaacaaaag atttgttttt gtagagattt taaaggggga 4080
gaattctaga aataaatgtt acctaattat tacagcctta aagacaaaaa tccttgttga 4140
agttttttta aaaaaagcta aattacatag acttaggcat taacatgttt gtggaagaat 4200
atagcagacg tatattgtat catttgagtg aatgttccca agtaggcatt ctaggctcta 4260
tttaactgag tcacactgca taggaattta gaacctaact tttataggtt atcaaaactg 4320
ttgtcaccat tgcacaattt tgtcctaata tatacataga aactttgtgg ggcatgttaa 4380
gttacagttt gcacaagttc atctcatttg tattccattg attttttttt tcttctaaac 4440
atttttcttc caaacagtat ataacttttt ttaggggatt ttttttaga cagcaaaaac 4500
tatctgaaga tttccatttg tcaaaaagta atgatttctt gataattgtg tagtaatgtt 4560
ttttagaacc cagcagttac cttaaagctg aatttatatt tagtaacttc tgtgttaata 4620
ctggatagca tgaattctgc attgagaaac tgaatagctg tcataaaatg aaactttctt 4680
tctaaagaaa gatactcaca tgagttcttg aagaatagtc ataactagat taagatctgt 4740
gttttagttt aatagtttga agtgcctgtt tgggataatg ataggtaatt tagatgaatt 4800
taggggaaaa aaaagttatc tgcagatatg ttgagggccc atctctcccc ccacaccccc 4860
acagagctaa ctgggttaca gtgttttatc cgaaagtttc caattccact gtcttgtgtt 4920
ttcatgttga aaatactttt gcatttttcc tttgagtgcc aatttcttac tagtactatt 4980
tcttaatgta acatgtttac ctggaatgta ttttaactat ttttgtatag tgtaaactga 5040
aacatgcaca ttttgtacat tgtgctttct tttgtgggac atatgcagtg tgatccagtt 5100
gttttccatc atttggttgc gctgacctag gaatgttggt catatcaaac attaaaaatg 5160
accactcttt taattgaaat taacttttaa atgtttatag gagtatgtgc tgtgaagtga 5220
tctaaaattt gtaatatttt tgtcatgaac tgtactactc ctaattattg taatgtaata 5280
aaaatagtta cagtgacaaa aaaaaaaaaa aa                               5312
```

<210> 5
<211> 567
<212> DNA
<213> Homo sapiens

<400> 5

```
atgactgaat ataaacttgt ggtagttgga gctggtggcg taggcaagag tgccttgacg 60
atacagctaa ttcagaatca ttttgtggac gaatatgatc caacaataga ggattcctac 120
aggaagcaag tagtaattga tggagaaacc tgtctcttgg atattctcga cacagcaggt 180
caagaggagt acagtgcaat gagggaccag tacatgagga ctggggaggg ctttctttgt 240
gtatttgcca taaataatac taaatcattt gaagatattc accattatag agaacaaatt 300
aaaagagtta aggactctga agatgtacct atggtcctag taggaaataa atgtgatttg 360
ccttctagaa cagtagacac aaaacaggct caggacttag caagaagtta tggaattcct 420
tttattgaaa catcagcaaa gacaagacag ggtgttgatg atgccttcta tacattagtt 480
cgagaaattc gaaaacataa agaaaagatg agcaaagatg gtaaaaagaa gaaaaagaag 540
tcaaagacaa agtgtgtaat tatgtaa                                     567
```

<210> 6
<211> 188
<212> PRT
<213> Homo sapiens

<400> 6

```
Met Thr Glu Tyr Lys Leu Val Val Val Gly Ala Gly Gly Val Gly Lys
 1               5                  10                  15
Ser Ala Leu Thr Ile Gln Leu Ile Gln Asn His Phe Val Asp Glu Tyr
            20                  25                  30
Asp Pro Thr Ile Glu Asp Ser Tyr Arg Lys Gln Val Val Ile Asp Gly
        35                  40                  45
Glu Thr Cys Leu Leu Asp Ile Leu Asp Thr Ala Gly Gln Glu Glu Tyr
    50                  55                  60
Ser Ala Met Arg Asp Gln Tyr Met Arg Thr Gly Glu Gly Phe Leu Cys
65                  70                  75                  80
Val Phe Ala Ile Asn Asn Thr Lys Ser Phe Glu Asp Ile His His Tyr
                85                  90                  95
Arg Glu Gln Ile Lys Arg Val Lys Asp Ser Glu Asp Val Pro Met Val
            100                 105                 110
Leu Val Gly Asn Lys Cys Asp Leu Pro Ser Arg Thr Val Asp Thr Lys
        115                 120                 125
Gln Ala Gln Asp Leu Ala Arg Ser Tyr Gly Ile Pro Phe Ile Glu Thr
    130                 135                 140
Ser Ala Lys Thr Arg Gln Gly Val Asp Asp Ala Phe Tyr Thr Leu Val
145                 150                 155                 160
Arg Glu Ile Arg Lys His Lys Glu Lys Met Ser Lys Asp Gly Lys Lys
                165                 170                 175
Lys Lys Lys Lys Ser Lys Thr Lys Cys Val Ile Met
            180                 185
```